# EUROPEAN PATENT APPLICATION

(11) **EP 4 487 838 A2**
(43) Date of publication of application: **08.01.2025**
(21) Application number: 24214233.9
(22) Date of filing: 06.03.2020
(51) Int. Cl.: A61K 9/00

(54) **AVENANTHRAMIDE COMPOSITIONS WITH IMPROVED SOLUBILITY COMPRISING 4-HYDROXYPHENONE**

(62) Divisional of application: 20710137.9
(71) Applicant: Symrise AG, 37603 Holzminden (DE)
(72) Inventor: LANGE, Sabine, 37603 Holzminden (DE); HERRMANN, Martina, 31789 Hameln (DE); MEYER, Imke, 37619 Bodenwerder (DE); BRUNCKE, Sebastian, 37671 Höxter (DE)
(74) Representative: Global IP Europe Patentanwaltskanzlei

(57) **Abstract**

The present invention relates generally to: a composition comprising or consisting of at least one avenanthramide or an analogue thereof and 4-hydroxyacetophenone with an improved solubility; its cosmetic or medical use; the use of such composition for the preparation of foods, food supplements, cosmetic, pharmaceutical or veterinary preparations; and foods, food supplements, cosmetic, pharmaceutical or veterinary perparations comprising such a composition. Finally, the present invention relates to 4-hydroxyacetophenone for improving solutility of an avenantramide or an analogue thereof.

## Description

### Technical field

The present invention relates generally to: a composition comprising or consisting of at least one avenanthramide or an analogue thereof and 4-hydroxyacetophenone with an improved solubility; its cosmetic or medical use; the use of such composition for the preparation of foods, food supplements, cosmetic, pharmaceutical or veterinary preparations; and foods, food supplements, cosmetic, pharmaceutical or veterinary perparations comprising such a composition. Finally, the present invention relates to 4-hydroxyacetophenone for improving solutility of an avenanthramide or an analogue thereof.

### Background Art

Avenanthramides (in the following abbreviated as Avns or Avn for a single avenanthramide compound), which are low-molecular-weight phenolic amides containing anthranilic acid and hydroxycinnamic acid moieties with an amide bond, are a group of naturally occurring phenolic amides in oats, both *A*. *sativa* and *A. nuda.* They were originally identified as phytoalexins produced by the plant in response to exposure to pathogens, such as fungi. Oats contain a unique group of approximately 40 different types of Avns, which are present in both oat grains and leaves. The most abundant are Avn A (N-(4'-hydroxycinnamoyl)-5-hydroxyanthranilic acid), Avn B (N-(4'-hydroxy-3'-methoxycinnamoyl)-5-hydroxyanthranilic acid) and Avn C (N-(3'-4'-dihydroxycinnamoyl)-5-hydroxyanthranilic acid), which are amides of 5-hydroxyanthranilic acid with p-coumaric, ferulic and caffeic hydroxycinnamic acids, respectively. These Avns are constitutively expressed in the kernels, appearing in almost all milling fractions, but occur at their highest concentrations in the bran and outer layers of the kernel [Boz H, Czech Journal of Food Sciences 2015, 33(5): 399-404]. The total content of avenanthramides (Avns) in oat grain has been found to be about 2 to 700 mg/kg (0.0002 to 0.07 %), depending on the cultivar and agronomic treatment [Maliarova M et al., Journal of the Brazilian Chemical Society 2015, 26(11), 2369-2378].

The extraction of Avns from oats was carried out using various solvent compositions such as pure or diluted ethanol and methanol. Extraction procedures were achieved over different times at room temperature or under controlled heating, such as naked oats, 50% aqueous ethanol [Tong L et al., Journal of Integrative Agriculture 2014, 13, 1809].

Maliarova, M. et al., Journal of the Brazilian Chemical Society 2015, 26(11), 2369 - 2378 compared the efficiency of methanol, ethanol and isopropanol on the extraction of Avns from naked oat bran. The optimum conditions for the highest yield of Avns were a methanol concentration of 70 %, an extraction temperature of 55 °C and an extraction time of 165 minutes.

The application of avenanthramides is a growing field in therapeutics since a number of studies have demonstrated that avenanthramides have excellent antioxidant activity both *in vitro* and *in vivo,* as well as anti-inflammatory, anti-irritant, anti-atherogenic and anti-proliferative activities which may prevent or limit cellular oxidative dysfunctions and the development of oxidative stress-related diseases, such as neurodegenerative and cardiovascular diseases, and provide additional protection against skin irritation, aging, CHD and cancer [Perrelli A et al., Oxidative Medicine and Cellular Longevity 2018, DOI: 10.1155/2018/6015351].

The antioxidant activity of Avns has been found to be 10 to 30 times higher than those of the typical cereal components ferulic acid, gentisic acid, phydroxybenzoic acid, protocagtechuic acid, syringic acid, vanillic acid and vanillin. The Avns differ in the antioxidant activity, Avn C having the highest activity, followed by Avn B and Avn A. Avns enriched oat extracts inhibit LDL oxidation in vitro. Both, animal studies and human clinical trials confirmed that oats antioxidants have the potential of reducing cardiovascular risks by lowering serum cholesterol, inhibiting LDL cholesterol oxidation and peroxidation. Another study has indicated that the consumption of oats and oats bran may reduce the risk of colon cancer not only because of their high fiber contents but also due to Avns. Furthermore, Avns enriched oat extracts have been shown to inhibit atherosclerosis and activation of the NF-kB transcription factor, which is the regulator of infection and inflammation [Hüseyin Boz, Phenolic Amides (Avenanthramides) in Oats - A Review, Czech J. Food Sci., 33, 2015 (5), 399 - 404].

WO 2004/047833 A1 describes the inhibition of substance P-induced liberation of histamine from mast cells and the treatment and prevention of itching by avenanthramides and avenanthramide analogue substances.

WO 2017/159964 A1 describes compositions comprising, as an active ingredient, avenanthramide or a derivative thereof, for preventing or treating hearing loss.

EP 0 157 420 A2 describes avenanthramides, including compounds structurally related to avenanthramide L, as 5-lipoxygenase inhibitors.

Lotts T et al., Experimental Dermatology 2017, 26(8): 739 - 742, describes how dihydroavenanthramide D (CAS 697235-49-7, INCI name: hydroxyphenyl propamidobenzoic acid; the active ingredient in SymCalmin^{®} provided by Symrise) inhibits mast cell degranulation and exhibits anti-inflammatory effects through the interaction with the neurokinin-1 receptor.

Avenanthramides, have potent beneficial biological activities, making them valuable and highly interesting naturally active ingredients for nutritional, cosmetic and health use for oral and/or topical applications for humans and animals.

There is thus an ongoing need and consumer demand in the food, cosmetic, pharmaceutical and veterinary industry for the development of new preparations or customary formulations based on naturally occurring, well tolerated and biodegradable substances, especially for use in skin protection and in the prevention and/or treatment of dermatoses, which are stable and do not degrade during processing steps, storage or transport as well as by the influence of light and UV irradiation, metal cations, air or certain enzymes, respectively.

Oxidative degradation processes and autoxidative processes play an important role for the stability of preparations, since they can destroy desired ingredients that then are not present, can produce unpleasant or undesired degradation products or adversely influence physical performance characteristics of the product, for example color, and, hence, often decreases the value of the product.

Avenanthramides, that find application in the nutrition, cosmetic, pharmaceutical or veterinary industry, however, have a poor solubility in organic, aqueous organic or aqueous solvents. This limits either their concentration in solutions with clear appearance or make them flocculate or precipitate during storage which is disadvantageous for the composition itself or for the final product in which they are incorporated. The solvent is generally a liquid, which can be a pure substance or a mixture of two or more solvent substances. Due to flocculation or precipitation, the avenanthramides as active substances are no longer present or only present in a reduced concentration in the dissolved form which lowers their bioavailabilty. Among the avenanthramide compounds, especially avenanthramide A and L have a low solubility.

Solubility is one of the important parameters to achieve a desired concentration of an active substance or drug in the respective tissue or systemic circulation for achieving required biological or pharmacological response. Poorly soluble substances often require high doses in order to reach biologically relevant or therapeutic concentrations after administration. Low solubility is the major problem encountered with formulation development of new chemical entities as well as generic development, most importantly low water solubility. Any active substance or drug to be absorbed must be present in the dissolved form at the site of absorption. Poorly soluble substances lead to inadequate and variable bioavailability. For topically and orally administered drugs or active substances solubility is the most important parameter to achieve their desired concentration or bioavailability for biological or pharmacological response. Any drug or active substance to be absorbed must be present in the form of solution at the site of absorption. Problem of solubility is a major challenge for formulation scientist.

Hence, in order to maintain a good solubility of avenanthramides, and, thus, to achieve a biologically relevant or therapeutic concentration or bioavailability of avenanthramides in a composition, the solubility of avenanthramide compounds needs to be improved.

Accordingly, it is the object of the present invention to provide a composition comprising an avenanthramide or an analogue thereof which exhibits improved solubility of avenanthramide(s), in particular avenanthramide A or avenanthramide L or Dihydroavenanthramide D.

In particular, the aim of the present invention is to suggest naturally, biodegradable, cosmetically or pharmaceutically well tolerated, safe, easy-to-use and stable substances which are able to improve the solubility of an avenanthramide in a composition and which do not interfere with the beneficial biological activity of avenanthramides or the preparation or customary formulation properties as such. Especially, the aim of the present invention is to suggest naturally, biodegradable, cosmetically or pharmaceutically well tolerated, safe, easy-to-use and stable substances which are able to improve the solubility of avenanthramide A or avenanthramide L or Dihydroavenanthramide D in a composition or customary formulation.

Surprisingly, it turns out that the solubility of avenanthramides or their analogues, in particular avenanthramide A or avenanthramide L or Dihydroavenanthramide D, which are the one with the poorest solubility among the avenanthramides, can be significantly increased with 4-hydroxyacetophenone, particularly at a low concentration. This is particularly the case, wherein the at least one avenanthramide analogue is Dihydroavenanthramide D.

### Summary of the invention

The aforementioned object is achieved in accordance with a first aspect of the present invention by providing a composition comprising or consisting of:
- at least one avenanthramide or an analogue thereof; and
- 4- hydroxyacetophenone.

In a second aspect, the present invention relates to the use of said composition as a cosmetic, in particular for use as a dermatological cosmetic in skin care, scalp care, hair care, nail care or in the prevention and/or treatment of skin conditions, intolerant and sensitive skin, skin irritation, skin reddening, wheals, pruritus (itching), skin aging, wrinkle formation, loss of skin volume, loss of skin elasticity, pigment spots, pigment abnormalities, or dry skin, i.e. for moisturising the skin.

In a third aspect, the present invention relates to the use of said composition as a medicament, in particular for use in the prevention and/or treatment of dermatological or keratological diseases, in particular dermatological or keratological diseases having a barrier related, inflammatory, immunoallergic, atherogenic, xerotic or hyperproliferative component or in the prevention and/or treatment of dermatological diseases associated with increased ROS production or in the prevention and/or treatment of cardiovascular diseases, allergic reactions, coronary heart disease, for decreasing the level of LDL cholesterol and lipids in blood serum, for reducing blood pressure, for improving sensitivity to insulin and for enabling the control of blood glucose levels.

In a fourth aspect, the present invention relates to the use of said composition for preparing foods, food supplements, cosmetic, pharmaceutical or veterinary preparations.

In a fifth aspect, the present invention relates to foods, food supplements, cosmetic, pharmaceutical or veterinary preparations comprising the composition according to the present invention.

Finally, the present invention relates to the use of 4- hydroxyacetophenone for improving the solubility of avenanthramide(s) or aventhramide analogue compound(s), in particular avenanthramide A or avenanthramide L or Dihydroavenanthramide D (2-[(3-(4-hydroxyphenyl)propanoyl]amido)benzoic acid; INCI: Hydroxyphenyl Propamidobenzoic Acid; CAS 697235-49-7).

The invention is specified in the appended claims. The invention itself, and its preferred variants, other objects and advantages, are however also apparent from the following detailed description in conjunction with the accompanying examples and figures.

### Figures

Figure 1 are images of solutions with avenanthramide A
Figures 2 a and 2 b are images of solutions with avenanthramide L
Figure 3 are images of solutions with a blend of avenanthramides A and B
Figure 4 are images of solutions with a blend of avenanthramides B, C and D
Figures 5 a and 5 b are images of solutions with Dihydroavenanthramide D

### Detailed description of the invention

In a first aspect, the present invention relates to a composition comprising or consisting of:
- at least one avenanthramide or an analogue thereof; and
- 4-hydroxyacetophenone.

The first main ingredient of the composition according to the first aspect of the present invention is at least one avenanthramide or an analogue thereof.

As used in this document, the phrase "at least one" means that the composition can comprise for example either one avenanthramide or more than one avenanthramide. Additionally, the phrase "at least one of", when applied to a list, means anyone combination of the items specified in the list.

The composition according to the first aspect of the present invention is prepared by combining the ingredients specified, as described in further detail below.

Within the context of the present invention, the term "avenanthramide(s)" (anthranilic acid amides) is understood to mean inter alia a member of a group of phenolic alkaloids, i.e. naturally occurring aventhramide(s), found mainly in oats (*Avena sativa*) but also present in white cabbage butterfly eggs (*Pieris brassicae and P. rapae*) and in fungus-infected carnations (*Dianthus caryophyllus*)*,* as described in detail hereinafter, or non-naturally occurring artificial produced avenanthramide analogue(s), as described in detail hereinafter.

The avenanthramides of the composition of the present invention are naturally found in and can be enriched, isolated and purified from oats. The two main species of oats are *Avena sativa* L. and *Avena nuda* L. (synonyms include *Avena sativa subsp. nuda* (L.) after Gillet & Magne, and *Avena sativa var. nuda* (L.) after Körn), wherein they appear to be most concentrated in the peripheral regions, husks, trichomes or straw. More than 50 distinct avenanthramides have been isolated from oat grains [Collins, Journal of Agricultural and Food Chemistry, 37 (1989), 60-66].

Avns can be represented by the following general Formula 1:

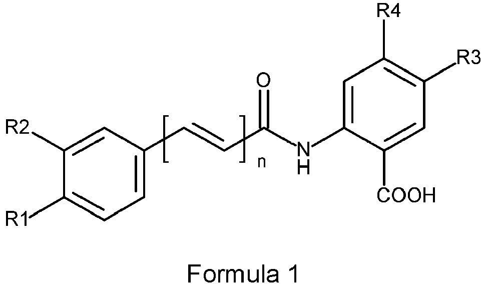

The following Table 1 shows examples of naturally occurring Avns based on general Formula 1.

**Table 1:**

| **Avenanthramide *)** | **CAS number** | **N** | **R1** | **R2** | **R3** | **R4** |
|---|---|---|---|---|---|---|
| A | 108605-70-5 | 1 | OH | H | OH | H |
| B | 108605-69-2 | 1 | OH | OMe | OH | H |
| C | 116764-15-9 | 1 | OH | OH | OH | H |
| D | 115610-36-1 | 1 | OH | H | H | H |
| E | 93755-77-2 | 1 | OH | OMe | H | H |
| F | 116764-16-0 | 1 | OH | OH | H | H |
| G | 116764-17-1 | 1 | OH | H | H | OH |
| H | 116764-18-2 | 1 | OH | OMe | H | OH |
| K | 116764-19-3 | 1 | OH | OH | H | OH |
| X | 1158480-77-3 | 1 | OH | H | OH | OMe |
| Y (2 **) | 154992-25-3 | 1 | OH | OMe | OH | OMe |
| Z | 1158480-80-8 | 1 | OH | OH | OH | OMe |
| AA | 157799-28-5 | 1 | OH | H | OH | OH |
| BB | 2304718-64-5 | 1 | OH | OMe | OH | OH |
| CC | 1819995-77-1 | 1 | OH | OH | OH | OH |
| O (L **) | 172549-38-1 | 2 | OH | H | OH | H |
| P | 1358438-37-5 | 2 | OH | OMe | OH | H |
| Q | 2227208-43-5 | 2 | OH | OH | OH | H |
| L | 2301866-39-5 | 2 | OH | H | H | H |
| M | 101618-11-5 | 2 | OH | OMe | H | H |
| N | 101618-21-7 | 2 | OH | OH | H | H |
| R | 1191042-39-3 | 2 | OH | H | H | OH |
| S | 2301866-43-1 | 2 | OH | OMe | H | OH |
| T | 2301864-63-9 | 2 | OH | OH | H | OH |
| U | 2301864-86-6 | 2 | OH | H | OH | OMe |
| V | 2304718-63-4 | 2 | OH | OMe | OH | OMe |
| W | 2304718-62-3 | 2 | OH | OH | OH | OMe |
| OO | 2301866-28-2 | 2 | OH | H | OH | OH |
| PP | 2301864-57-1 | 2 | OH | OMe | OH | OH |
| QQ | 2301864-89-9 | 2 | OH | OH | OH | OH |

| | | | | | | |
|---|---|---|---|---|---|---|
| *) Abbreviations Collins [de Bruijn et al., Food Chemistry (2018), doi: https://doi.org/10.1016/j.foodchem.2018.11.013, supplementary information Table S1] **) More commonly used, non-Collins abbreviations | | | | | | |

A number of studies have demonstrated that said avenanthramides have anti-inflammatory, anti-oxidant, anti-itch, anti-irritant and anti-atherogenic activities.

The naturally occurring avenanthramides or mixtures of avenanthramides as described above, can be obtained, enriched and isolated from the plant of the genus *Avena* by extraction, in particular from any oat species, fresh or dried, or parts thereof, such as milled grains, non-milled grains, husks, trichomes or oat straw of the oat species *Avena sativa* or *Avena nuda.*

The extracting solvent (extractant) for favourably extracting the avenanthramide L is selected from the group consisting of mixtures of water and an organic solvent, wherein the organic solvent is preferably a solvent suitable for foodstuffs or cosmetic or pharmaceutical preparations. It goes without saying that such solvents need be suitable for and compatible with the preparation of foods, cosmetics or pharmaceutical preparations.

In a more preferred variant, the extracting solvent comprises a mixture of water and an alcohol or acetone. The alcohol is preferably selected from the group consisting of methanol, ethanol, n-propanol, isopropanol, n-butanol, isobutanol, t-butanol and mixtures, i.e. combinations, thereof. The most preferred extracting solvents (extractant) for the extraction step of the present invention are methanol, ethanol, n-propanol, isopropanol or acetone or any mixtures respective combinations of said solvents, each in mixture with water. The use of pure organic solvents is not advantageous, due to the co-extraction of triglycerides.

The mixing ratio of water to the organic solvent, preferably water to the alcohol or water to acetone, in the extracting solvent is in a range of 10 : 90 to 90 : 10 (v/v), preferably in a range of 20 : 80 to 80 : 20 (v/v) and most preferably in a range of 30 : 70 to 70 : 30 (v/v), based in each case on the resulting extracting solvent.

Particularly preferred extracting solvents (extractants) are: methanol/water (3 : 7), methanol/water (1 : 1), methanol/water (7 : 3), ethanol/water (3 : 7), ethanol/water (1 : 1), ethanol/water (1 : 4), ethanol/water (7 : 3), isopropanol/water (3 : 7), isopropanol/water (1 : 1), isopropanol/water (7 : 3), aceton/water (3 : 7), aceton/water (1 : 1), aceton/water (7 : 3).

In order to improve the extraction yield, the oat source is extracted at a temperature ranging from 30 to 80 °C, preferably from 40 to 70 °C and more preferably from 50 to 60 °C. The extraction yield for milled oat grains increases with increasing temperatures between 40 and 70 °C.

Apart from avenanthramide compounds enriched, isolated and purified from natural sources, the naturally occurring avenanthramides can be produced by organic synthesis. Methods of synthesis known in the art are illustrated for example in US Patent Nos 6,096,770 and 6,127,392, Japanese Patent No. J60019 754 A and Hungarian Patent No. HU 200 996 B.

Said synthetic prepared avenanthramide substances are identical to the corresponding naturally occurring avenanthramide compounds as isolated or extracted from oats.

The non-naturally occurring avenanthramide analogue(s) in the composition according to the present invention, hereinafter also referred to as "analogue(s)" or "analogue avenanthramide compound(s)" which are in accordance with the following Formula 2 and endowed with important biological properties have been artificially produced by organic synthesis methodologies, such as for example those given in WO 2004/047833 A1 or WO 2007/062957 A1: 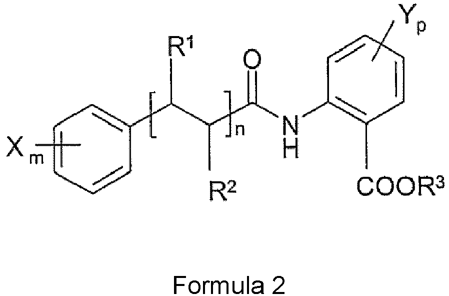
where m = 0, 1, 2 or 3, p = 0, 1 or 2, and n = 0, 1 or 2,
with the proviso that if n = 1 or 2, then p + m > 0,
and if n = 1 or 2, then R¹ and R², in respective pairs, respectively denote H or together denote another chemical bond (as for example in cinnamic acid derivatives), and if m = 1, 2 or 3, then each X independently denotes OH, Oalkyl or Oacyl,
and if p = 1 or 2, then each Y independently denotes OH, Oalkyl or Oacyl,
and if p + m > 0, then at least one of X and Y is selected from the group consisting of OH and Oacyl,
and where R³ is -H or an alkyl (in particular -CH₃, or other straight-chain or branched alkyl chains with 2 to 30 C atoms; in this context, R³ is also -H for the corresponding pharmaceutically acceptable salts).

Particularly preferred compounds of Formula 2 according to the invention are those in which:
n = 1 or 2 and p + m > 0; and/or
p + m > 0 and X or Y at least one of X and Y is selected from the group consisting of OH and Oalkyl.

Particularly preferably, a compound of Formula 2 is used in which n = 1 and p + m > 2, with the proviso that at least two of X and Y are together selected from the group comprising OH and Oalkyl.

It is also preferable to use a compound of Formula 2 in which n = 1 and m = 1, 2 or 3, with the proviso that at least one X is selected from the group comprising OH and Oalkyl, and/or P = 1 or 2, with the proviso that at least one Y is selected from the group comprising OH and Oalkyl.

If n has the value 1, then R¹ and R² are each preferably H, although it is also possible for R¹ and R² together to be another chemical bond.

With regard to the definition of Formula 2 and the specific avenanthramide compounds disclosed in WO 2004/047833 A1 or WO 2007/062957 A1, the corresponding disclosure in said documents is hereby incorporated by reference.

The avenanthramide analogue compound of Formula 2 is preferably selected from the group consisting of:

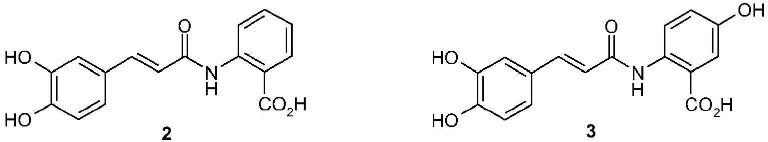

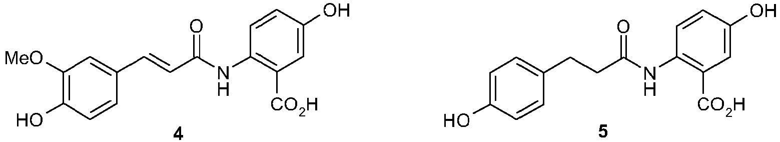

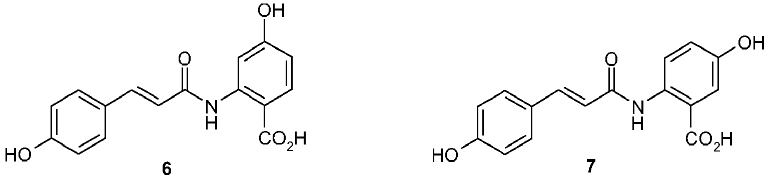

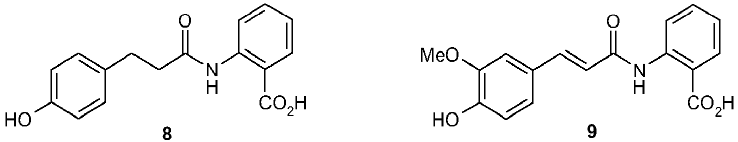

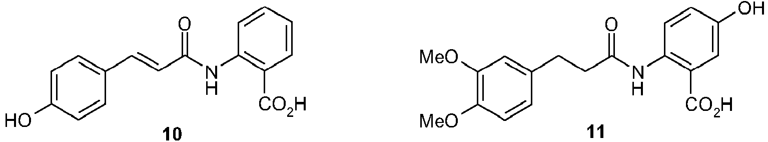

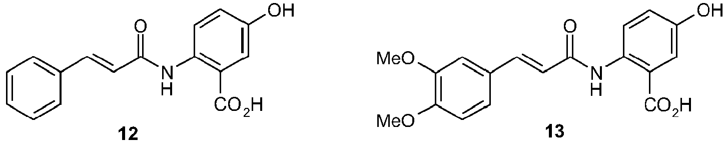

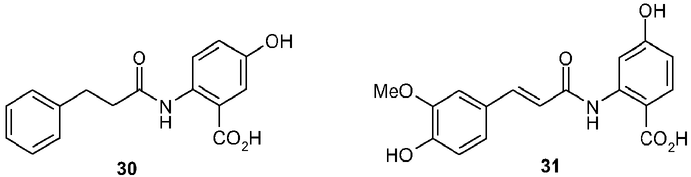

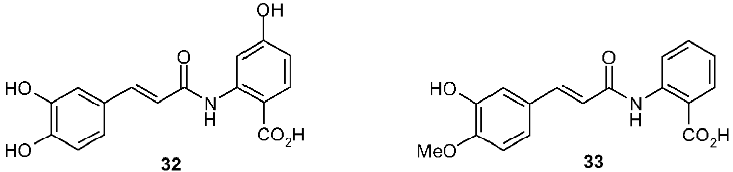

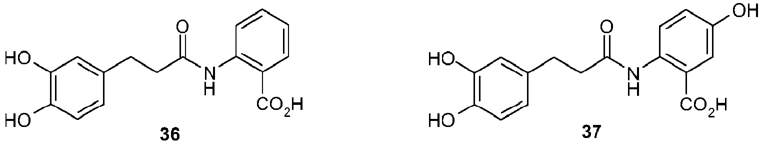

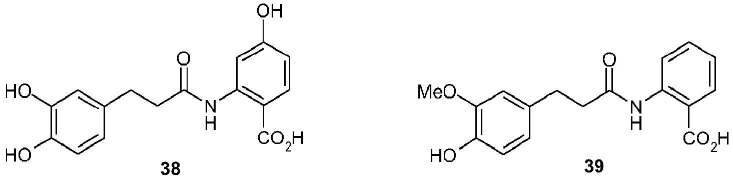

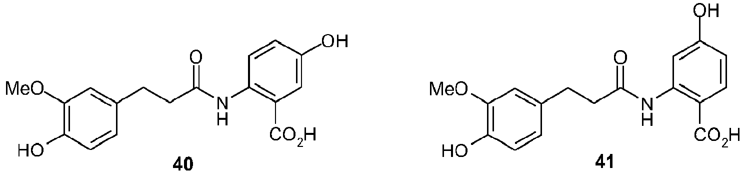

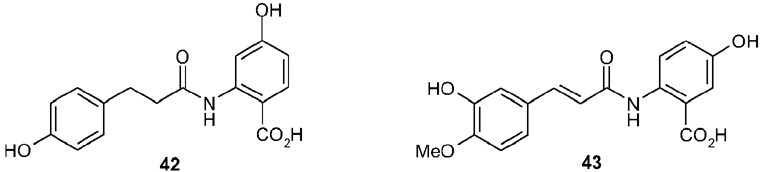

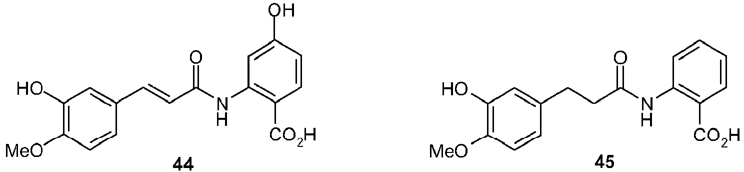

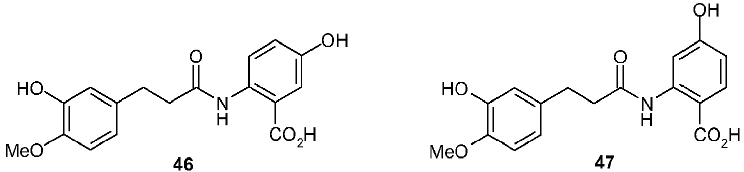

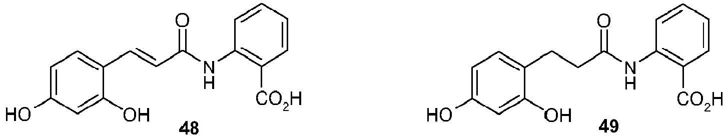

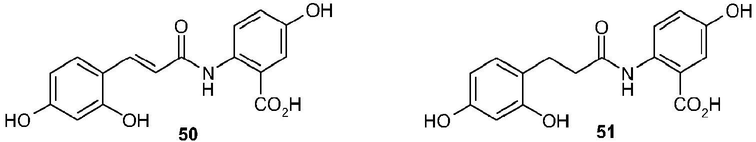

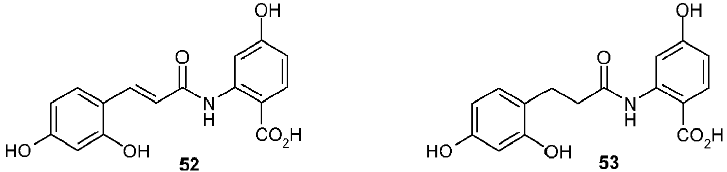

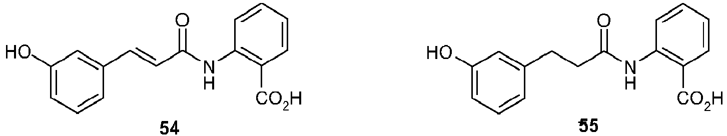

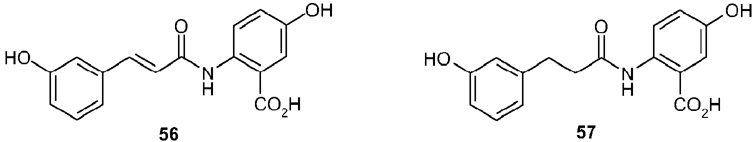

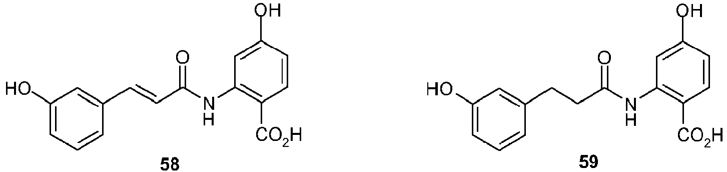

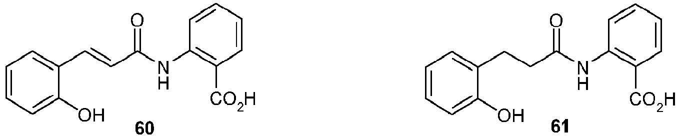

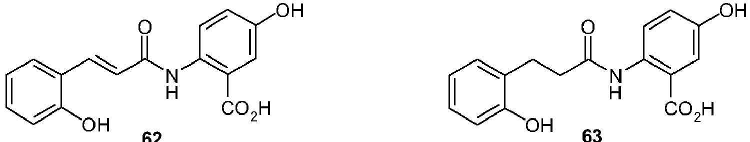

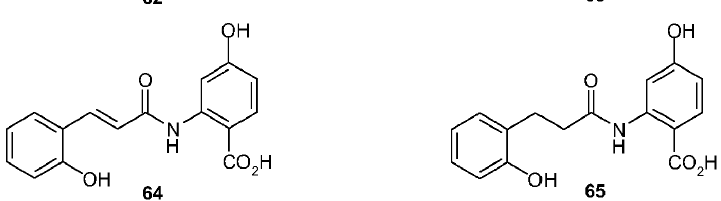

The above illustrations relate essentially to compounds of Formula 2 in which n = 1.

However, the use of compounds of Formula 2 in which n = 0 is also frequently preferred, in which case it preferably holds that m + p = 0, or m + p > 1 or 2, with the proviso that at least two of the substituents X and Y are selected from the group comprising OH and Oalkyl.

It is particularly preferable to use compounds of Formula 2 (where n = 0) selected from the group comprising:

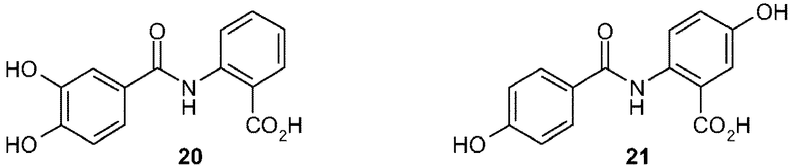

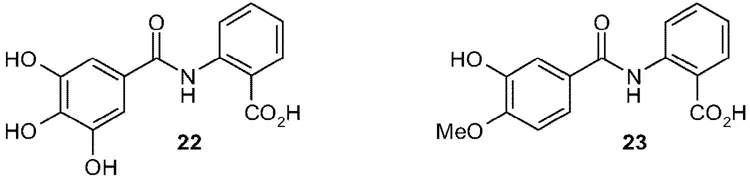

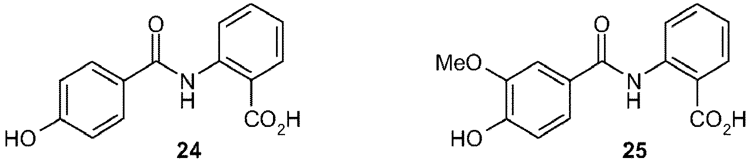

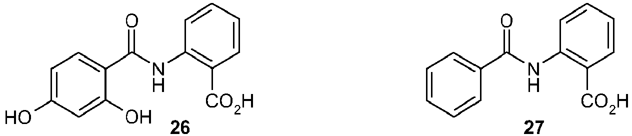

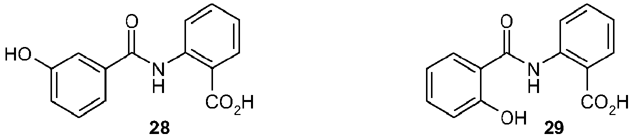

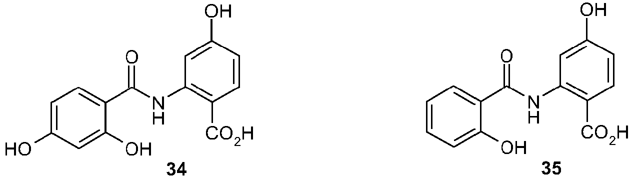

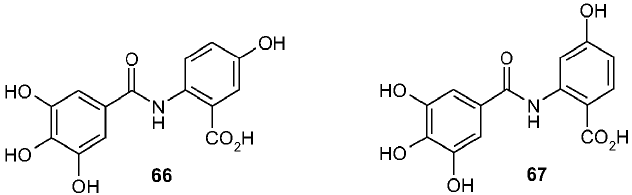

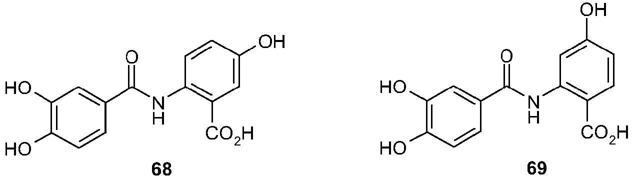

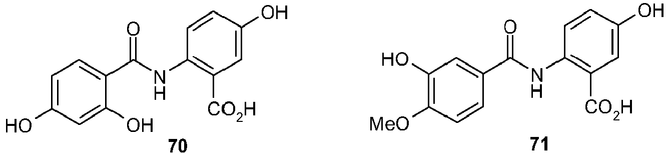

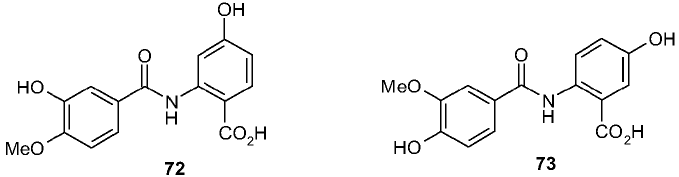

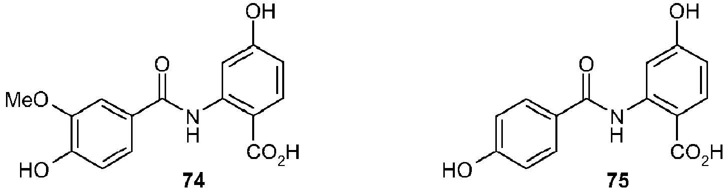

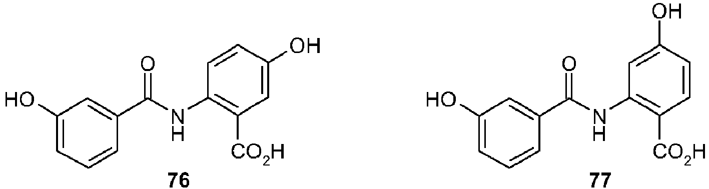

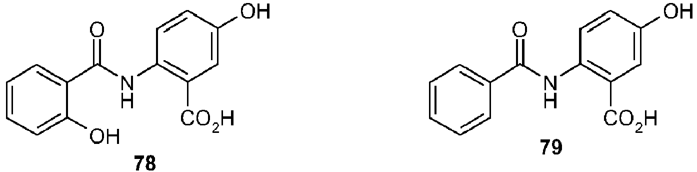

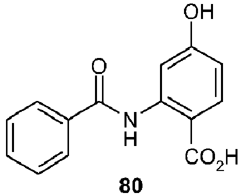

In the compounds described as particularly preferred and indicated by their structural formulae, R³ is always H.

Instead of these preferred compounds, it is also preferable in each case to use the corresponding compounds in which R³ is CH₃ or a linear or branched alkyl having 2 to 30 C atoms.

From the above avenanthramide analogues, compounds No. 8 (Dihydroavenanthramide D (2-[(3-(4-hydroxyphenyl)propanoyl]amido)benzoic acid; INCI: Hydroxyphenyl Propamidobenzoic Acid; CAS 697235-49-7)) and No. 27 are particularly preferred.

Besides the above natural occurring avenanthramides and non-natural occurring avenanthramides analogues, novel avenanthramide analogues have been produced in recombinant yeast, including N-(4'-hydroxycinnamoyl)-3-hydroxyanthranilic acid (YAvn I) and N-(3'-4'-dihydroxycinnamoyl)-3-hydroxyanthranilic acid (YAvn II), which were generated by engineering a *Saccharomyces cerevisiae* strain with two plant genes (4cl-2 from tobacco and *hct* from globe artichoke) encoding key proteins involved in the biosynthesis of phenolic esters. Remarkably, YAvn I and YAvn II share structural similarities with Avn A and Avn C, respectively.

In the context of the present invention naturally occurring avenanthramides obtained from naturally sources or naturally occurring avenanthramides produced synthetically are preferred and are used likewise.

The term "avenanthramide or an analogue thereof" is intended to also include their various isomers that exist, notably the (naturally occurring) trans-isomers as well as the cis-isomers, such as avenanthramides with cis-isomerized double bond (Formula 1 or 2 with n = 1) or 1 or 2 cis-isomerized double bonds (Formula 1 or 2 with n = 2) induced e.g. by photoisomerization due to light exposure.

In particular, within the context of the present invention, the avenanthramide is any one of the avenanthramide compounds represented by the general Formula 1 and defined in Table 1 or any isomer thereof or the avenanthramide analogue is any one of the avenanthramide analogue compounds represented by the general Formula 2 and its definition or any isomer thereof as described above.

In a preferred variant of the present invention according to the first aspect, the composition comprises at least one avenanthramide selected from the group consisting of avenanthramides A, B, C, G, H, K, L and R, still more preferred avenanthramide A or avenanthramide L.

In a preferred variant of the present invention according to the first aspect, the composition comprises at least the avenanthramide analogue compound Dihydroavenanthramide D.

In another variant, the composition of the present invention comprises a mixture of two, three, four or even more different avenanthramides selected from the group consisting of avenanthramides A, B, C, G, H, K, L (non-Collins abbreviations; CAS number 172549-38-1) (also called O or 2pd) and R. The combinations/mixtures of avenanthramides can thus include any one of the following combinations of avenanthramides: A/B; A/C; A/G; A/H; A/K; A/L; A/R; B/C; B/G; B/H; B/K; B/L; B/R; C/G; C/H; C/K; C/L; C/R; G/H; G/K; G/L; G/R; H/K; H/L; H/R; K/L; K/R; L/R; A/B/C; A/B/G; A/B/H; A/B/K; A/B/L; A/B/R; A/C/G; A/C/H; A/C/K; A/C/L; A/C/R; A/G/H; A/G/K; A/G/L; A/G/R; A/H/K; A/H/L; A/H/R; A/K/L; A/K/R; A/L/R; B/C/G; B/C/H; B/C/K, B/C/L; B/C/R; C/G/H; C/G/K, C/G/L; C/G/R, G/H/K; G/H/L; G/H/R; H/K/L, H/K/R; K/L/R; A/B/C/G; A/B/C/H; A/B/C/K; A/B/C/L; A/B/C/R; A/C/G/H; A/C/G/K; A/C/G/L; A/C/G/R; A/G/H/K; A/G/H/L; A/G/H/R; A/H/K/L; A/H/K/R; A/K/L/R; B/C/G/H; B/C/G/K; B/C/G/L; B/C/G/R; C/G/H/K; C/G/H/L; C/G/H/R; G/H/K/L; G/H/K/R and H/K/L/R .

The most preferred mixtures of avenanthramides according to the present invention are however A/B, A/C, A/L, B/C and A/B/C.

In addition to the above avenanthramide compounds or avenanthramide combinations, the composition can further comprise one or more avenanthramide(s) other than the avenanthramides A, B, C, G, H, K, L (non-Collins abbreviations; CAS number 172549-38-1) (also called O or 2pd) and R, such as avenanthramides D, E, F U, X, Y (also termed 2), AA, CC or OO or any of the remaining avenanthramide compounds specified in Table 1.

In another variant, the composition of the present invention comprises at least one, i.e. one, two or even more, avenanthramide(s) selected from the group consisting of avenanthramides A, B, C, G, H, K, L (non-Collins abbreviations; CAS number 172549-38-1) (also called O or 2pd) and R in combination with the avenanthramide analogue compound Dihydroavenanthramide D. The mixtures of avenanthramides can thus include any one of the following combinations: A/Dihydroavenanthramide D; B/Dihydroavenanthramide D; C/Dihydroavenanthramide D; G/Dihydroavenanthramide D; H/Dihydroavenanthramide D; K/Dihydroavenanthramide D; L/Dihydroavenanthramide D; or R/Dihydroavenanthramide D; A/B/Dihydroavenanthramide D; A/C/Dihydroavenanthramide D; A/D/Dihydroavenanthramide D; A/G/Dihydroavenanthramide D; A/H/Dihydroavenanthramide D; A/K/Dihydroavenanthramide D; A/L/Dihydroavenanthramide D; A/R/Dihydroavenanthramide D; B/C/Dihydroavenanthramide D; B/D/Dihydroavenanthramide D; B/G/Dihydroavenanthramide D; B/H/Dihydroavenanthramide D; B/K/Dihydroavenanthramide D; B/L/Dihydroavenanthramide D; B/R/Dihydroavenanthramide D; C/D/Dihydroavenanthramide D; C/G/Dihydroavenanthramide D; C/H/Dihydroavenanthramide D; C/K/Dihydroavenanthramide D; C/L/Dihydroavenanthramide D; C/R/Dihydroavenanthramide D; G/H/Dihydroavenanthramide D; G/K/Dihydroavenanthramide D; G/L/Dihydroavenanthramide D; G/R/Dihydroavenanthramide D; H/K/Dihydroavenanthramide D; H/L/Dihydroavenanthramide D; H/R/Dihydroavenanthramide D; K/L/Dihydroavenanthramide D; K/R/Dihydroavenanthramide D; or L/R/Dihydroavenanthramide D, still more preferred A/Dihydroavenanthramide D or avenanthramide L/Dihydroavenanthramide D.

In addition to the above avenanthramide combinations, the composition can further comprise combinations with one or more avenanthramides other than the avenanthramides A, B, C, G, H, K, L (non-Collins abbreviations; CAS number 172549-38-1) (also called O or 2pd) and R, such as avenanthramides D, E, F U, X, Y (also termed 2), AA, CC or OO or any of the remaining avenanthramide compounds specified in Table 1 with Dihydroavenanthramide D.

In another variant, the composition of the present invention comprises one avenanthramide analogue compound, preferably Dihydroavenenthramide D, or a combination of an avenanthramide analogue compound, preferably Dihydroavenanthramide D, and any one or more different avenanthramide analogue compound(s) represented by the general Formula 2 and specified above.

The at least one avenanthramide or avenanthramide analogue compound or mixture of avenanthramides or avenanthramide analogue compounds, as described above, may be present in the composition at a concentration or total amount of 0.0001 to 5.0 wt%, based on the total weight of the composition. In a preferred variant, the composition comprises the at least one avenanthramide or avenanthramide analogue compound or mixture of avenanthramides or avenanthramide analogue compounds at a concentration or total amount of 0.0005 to 2.0 wt%, still more preferred at a concentration or total amount of 0.001 to 1.0 wt%, based on the total weight of the composition.

The second main ingredient of the composition according to the first aspect of the present invention is 4-hydroxyacetophenone (INCI: Hydroxyacetophenone; CAS 99-93-4) or p-hydroxyacetophenone, represented by the following Formula 3:

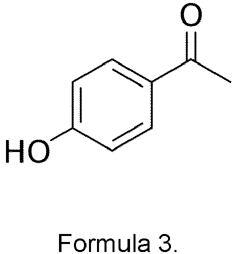

4-Hydroxyacetophenone is a nature identical ingredient. While it is synthetically manufactured, it is also found in nature in Norwegan spruce trees.

In cosmetic or pharmaceutical preparations, 4-hydroxyacetophenone is a synthetic antioxidant and skin-conditioning ingredient. The specific antioxidant compound is actually known as p-hydroxyacetophenone, a phenolic antioxidant capable of neutralizing several different types of free radicals. Its secondary benefit is boosting the preservation system in cosmetics. This is advantageous because it allows cosmetic chemists to use a lower amount of preservatives without losing efficacy but with the benefit of reducing the risk of an allergic reaction. 4-Hydroxyacetophenone also has soothing ability because it can inhibit an enzyme (known as COX-2) in skin's surface that can lead to signs of irritation. In addition, the product is a nature-identical ingredient that is FEMA/GRAS-listed. It shows excellent stability at high and low pH levels and temperatures. Furthermore, hydroxyacetophenone is easy to formulate, good for cold process formulations such as shampoos, wet wipes and lotions, is colorless with low odor, soluble in ethanol and glycols and has food grade, which makes the compound suitable for oral care. Additionally, 4-hydroxyacetophenone is known and used as compound for improving solubility, for example of flavours and fragrances, and especially for dissolving lipophilic compounds as described in EP 2 962 678 A1. However, because substances differ in their chemical properties, the effect of one compound in combination with other substances cannot simply be generalised and applied to other compounds.

Surprisingly, it turns out that combining with 4-hydroxyacetophenone is beneficial in improving the solubility of an avenanthramide or an avenanthramide analogue in a solvent or in a composition comprising an avenantrahmide or more avenanthramides or an avenanthramide analogue. Depending on its concentration, the 4-hydroxyacetophenone can even completely solve an avenanthramide or more avenanthramides or an avenanthramide analogue compound or more avenanthramide analogoue compounds.

More surprisingly, it turns out that the addition of 4-hydroxyacetophone is particular effective in increasing the solubility of avenanthramide A or avenanthramide L or Dihydroavenanthramide D which have the the poorest solubility among the avenanthramides or avenanthramide analogues respectively.

A compound for improving the solubility means a compound used to improve the solubility of another poorly soluble component or substance in a solvent or in a composition and to prevent flocculation or precipitation of said component or substance. By increasing the solubility of the poorly soluble component or substance, its desired concentration in the solvent or in the composition in which the component is incorporated or its bioavailability for biological or pharmacological response is increased. Additionally, by increasing the solubility of the poor soluble component or substance, liquid compositions or solutions comprising said poor soluble component or substance can be stabilized during storage.

Thus, 4-hydroxyacetophenones within the context of the present invention is a substance that is used to improve the solubility of an active component, i.e. an avenanthramide or avenanthramides in a solvent or in a composition, thus increasing the bioavailability of the active substance in the composition or a final product in which they are incorporated.

By adding 4-hydroxyaceophenone the solubility of the avenanthramides can be increased significantly, i.e. the avenanthramides do not flocculate or precipitate. In particular, liquid compositions solutions comprising one or more avenanthramide(s) thus can be stabilized during storage. This effect is demonstrated by the following examples: the addition of 4-hydroxyacetophenone leads to a more transpartent solution with less precipitation.

The total amount of 4-hydroxyacetophenone present in the composition according to the present invention can be between 0.005 and 2.0 wt%, based on the total weight of the composition. In a preferred variant, the concentration of the total stabilizer in the composition is 0.01 to 1.5 wt%, based on the total weight of the composition, and can even more preferably be between 0.1 and 1.0 wt%, based on the total weight of the composition.

Concentrations of the 4-hydroxyacetophenone present in the composition according to the present invention between 0.1 and 1.0 wt% are preferred at most: the composition is clear and is not turbid. This means, the avenanthramides are completely dissolved in the composition.

As it is obvious from the following examples, the 4-hydroxyacetophenone can effective improve solubility of an anventhramide or avenanthramides: In a concentration of 0.01 wt%, 4-hydroxyacetophenone already improves the solubility of an avenanthrahmide or avenanthramides, in particular avenanthramide A, and in a concentration of 0.50 wt% 4-hydroxyacetophenone completely solves an avenanthramide or avenanthramides even in low concentrations. The above concentrations corresponds to such concentrations, in which hydroxyacetophenone is usually used as antioxidant or antimicrobial agent in cosmetic or pharmaceutical preparations.

Particularly preferred mixtures according to the present invention are those in which the composition comprises or consists of:
- 0.0001 to 5.0 wt% of the at least one avenanthramide or an analogue thereof, in particular 0.0005 to 2.0 wt%, still more preferred 0.001 to 1.0 wt%,; and
- 0.005 to 2.0 wt% 4-hydroxyacetophenone, in particular 0.01 to 1.5 wt%, still more preferred 0.1 to 1.0 wt%,
based on the total weight of the composition.

A good solubility is in particular achieved, if the at least one avenanthramide to 4-hydroxyacetophenone is present in the composition according to the present invention in a ratio of 1 : 1 to 1 : 50; more prefered in a ratio of 1 : 1.5 to 1 : 40 or if the ratio of the at least one avenanthramide analogue, particularly Dihydroavenanthramide D, is 1 : 0.2 to 1 : 30, in particular 1 : 0.5 to 1 : 20, as it is demonstrated by Table8. The appearance of the composition is good and the avenanthramide or the avenanthramide analogue compound does not flocculate or precipitate.

The composition according to the invention, in particular those characterised as preferred compositions, possess a good solubility of avenanthramides or avenanthramide analogoue compound, in particular of avenanthramide A, aventhramide L or Dihydroavenanthramide D. The solubility of the composition according to the present invention is surprisingly superior to that of compositions comprising one or more avenanthramide(s) or avenanthramide analogue compound(s) only without 4-hydroxyacetophenone. The cosmetically or pharmaceutically active substances, i.e. avenanthramides or avenanthramide analogue, particularly Dihydroavenanthramide D, which exhibit biological benefits of great interest, such as anti-inflammatory, antioxidant, anti-itching, anti-irritant and anti-atherogenic activities, do not flocculate or precipitate and, thus, are longer available in a therapeutic effective amount and thus better reach their intended target.

The composition according to the present invention is also particularly effective and free of any toxicologically or dermatologically critical secondary components; the composition can therefore be used without further concerns in cosmetic or pharmaceutical preparations.

The composition according to the present invention can be prepared either in form of a liquid composition with improved solubility before adding it to the end formulation or the individual composition constituents can be added separately to the end formulation and improving the solubility of the at least one avenanthramide or of an avenanthramide analogue compound therein.

It should be borne in mind that 4-hydroxyacetophenone that is used in the composition and in the end preparation is
- toxicologically acceptable,
- well tolerated by the skin,
- stable (in particular in the customary formulations),
- preferably odourless and
- able to be produced inexpensively (i.e. using standard processes and/or starting from standard precursors)
in the concentration range relevant to activity and administration.

Due to the anti-inflammatory, anti-oxidant, anti-itch, anti-irritant and anti-atherogenic activities of the avenanthramides or the avenanthramide analogoue compounds in combination with the above described properties of the 4-hydroxyacetophenone, resulting in a desired concentration or bioavailability of the avenanthramides or the avenanthramide analogues for biological or pharmacological response, the composition according to the present invention is thus beneficial for skin protection and in the prevention and/or treatment of dermatoses.

Another aspect of the present invention therefore relates to the use of the composition according to the first aspect of the present invention as a cosmetic, in particular for use in skin care, scalp care, hair care, nail care or in the prevention and/or treatment of skin conditions, intolerant and sensitive skin, skin irritation, skin reddening, wheals, pruritus (itching), skin aging, wrinkle formation, loss of skin volume, loss of skin elasticity, pigment spots, pigment abnormalities, or dry skin, i.e. for moisturising the skin.

Another aspect of the present invention relates to the composition according to the first aspect of the present invention for use as a medicament.

Due to its aforementioned superior properties, the composition according to the first aspect of the present invention is particularly useful in the prevention and/or treatment of dermatological or keratological diseases, in particular of dermatological or keratological diseases having a barrier related, inflammatory, immunoallergic, atherogenic, xerotic or hyperproliferative component or in the prevention and/or treatment of cardiovascular diseases, allergic reactions, coronary heart disease, for decreasing the level of LDL cholesterol and lipids in blood serum, for reducing blood pressure, for improving sensitivity to insuling anf for enabling the control of blood glucose levels.

Due to the particular antioxidative effect of the avenanthramide(s), the present invention also relates to the composition according to the first aspect of the present invention for use in the prevention and/or treatment of dermatological diseases associated with increased ROS production.

Examples of such dermatological or keratological disorders include eczema, psoriasis, seborrhoea, dermatitis, erythema, pruritus (itching), otitis, inflammation, irritation, fibrosis, *lichen planus, pityriasis* rosea, *pityriasis versicolor,* autoimmune bullous diseases, urticarial, angiodermal and allergic skin reactions, and wound healing, and examples of skin diseases associated with increased ROS production are selected from the group consisting of atopic dermatitis, neurodermitis, psoriasis, rosacea, acneiform eruptions, sebostasis and xerosis.

In a particularly preferred variant of the present invention, the composition comprising or consisting of at least one avenanthramide or an analogue thereof according to the present invention is beneficially useful in the prevention and/or treatment of pruritis (itching).

Chronic pruritis is a common symptom associated with various dermatological conditions and systemic diseases, with no known underlying condition in some cases. Chronic pruritis is classified by clinical presentation (for example, association with diseased/inflamed or normal/non-inflamed skin and/or presence of secondary scratch lesions) and underlying causes (of for example dermatological, systemic, neurological, psychosomatic, mixed or undetermined origin).

The use of avenanthramide(s) or an analogue thereof for these respective purposes corresponds to a method for imparting the respective therapeutic activity of the active substance by adding a therapeutically effective amount of the active substance or composition.

Within the context of the present invention, an effective amount of a composition is the amount of each active component, i.e. an avenanthramide, that is sufficient to show a benefit, such as a reduction in a symptom associated with the disorder, disease or condition to be treated. When applied to a combination or a composition, as in the present case, the term refers to the amount of the combined active ingredients resulting in the benefit.

Accordingly, the present invention relates to a method for preventing and/or treating dermatological or keratological diseases, in particular of dermatological or keratological diseases having a barrier related, inflammatory, immunoallergic, atherogenic, xerotic or hyperproliferative component or in the prevention and/or treatment of cardiovascular diseases, allergic reactions, coronary heart disease, for decreasing the level of LDL cholesterol and lipids in blood serum, for reducing blood pressure, for improving sensitivity to insuling anf for enabling the control of blood glucose levels, in a subject in need thereof, wherein the method comprises administering the subject with a therapeutically effective amount of a composition comprising or consisting of at least one avenanthramide or an analogoue thereof and 4-hydroxyacetophenone in an amount which is sufficient for the prevention and/or treatment of dermatological or keratological diseases, in particular of dermatological or keratological diseases having a barrier related, inflammatory, immunoallergic, atherogenic, xerotic or hyperproliferative component or in the prevention and/or treatment of cardiovascular diseases, allergic reactions, coronary heart disease, for decreasing the level of LDL cholesterol and lipids in blood serum, for reducing blood pressure, for improving sensitivity to insuling anf for enabling the control of blood glucose levels.

Due to its marked effect as described above, the composition according to the first aspect of the present invention is beneficially suitable for the preparation of foods, food supplements, cosmetic, pharmaceutical or veterinary preparations.

The composition according to the present invention can be easily incorporated into conventional foods, food supplements, cosmetic, pharmaceutical or veterinary preparations.

A further aspect of the present invention therefore relates to foods, food supplements, cosmetic, pharmaceutical or veterinary preparations which comprise the composition according to the present invention. In a preferred variant of the present invention, a functional food which includes the composition is provided as an effective ingredient for skin care and/or preventing or ameliorating the above dermatological or keratological disorders.

In a preferred variant, the foods, food supplements, cosmetic, pharmaceutical or veterinary preparations comprise the composition according to the present invention in an amount of 0.0001 to 5.0 %, more preferred 0.0005 to 2.0 %, most preferred 0.001 to 1.0 % by weight of thepreparation.

Within this context, it is also possible - and in some cases advantageous - to combine the composition according to the present invention with other active compounds, for example other synergistically intensifying substances, such as anti-inflammatories, antibacterial or antimycotic substances, substances having a reddening-alleviating or itch-alleviating action, lenitive substances, moisturisers and/or cooling agents and/or antioxidants, preservatives, (metal) chelating agents, penetration enhancers, and/or cosmetically or pharmaceutically acceptable excipients, as in detail described and exemplified below.

An active substance means a substance or compound that imparts a primary utility to a composition or preparation/customary formulation. Examples of such active substances include antioxidants, preservatives, (metal) chelating agents, penetration enhancers, etc. An excipient refers to an inactive substance used to formulate cosmetics or pharmaceuticals as a result of processing or manufacture.

Since dermatological conditions or diseases are often associated with dry skin, scratched skin, skin lesions or even inflammation, the composition or cosmetic and/or pharmaceutical preparations according to the present invention particularly advantageously contains a skin-moisturising and/or moisture-retaining substance, a cooling agent, an osmolyte, a keratolytic substance, a nurturing substance, an anti-inflammatory, antibacterial or antimycotic substance and/or a substance having a reddening-alleviating or itch-alleviating action and/or a lenitive substance.

Itching occurs with particular intensity when the skin is dry. The use of skin-moisturising and/or moisture-retaining substances or regulators in cosmetic and pharmaceutical preparations can significantly alleviate itching. The cosmetic or pharmaceutical preparations according to the present invention can therefore also be particularly advantageously combined with one or more skin-moisturising and/or moisture-retaining substances or regulators. Cosmetic or pharmaceutical preparations according to the present invention can therefore advantageously also contain the following moisturising and/or moisture-retaining substances or regulators: sodium lactate, urea, urea derivatives, alcohols, glycerol, diols such as propylene glycol, hexylene glycol, 1,2-pentanediol, 1,2-hexanediol, 1,2-heptanediol, 1,2-octanediol, 1,2-nonanediol, 1,2-decanediol or mixtures of said diols, in particular mixtures of 1,2-hexanediol and 1,2-octanediol, collagen, elastin or hyaluronic acid, diacyl adipates, petrolatum, urocanic acid, lecithin, panthenol, phytantriol, lycopene, (pseudo-)ceramides, glycosphingolipids, cholesterol, phytosterols, chitosan, chondroitin sulphate, lanolin, lanolin esters, amino acids, alpha-hydroxy acids (such as citric acid, lactic acid, malic acid) and their derivatives, mono-, di- and oligosaccharides such as glucose, galactose, fructose, mannose, fructose and lactose, polysugars such as R-glucans, in particular 1,3-1,4-β-glucan from oats, alpha-hydroxy fatty acids, triterpene acids such as betulinic acid or ursolic acid, and algae extracts.

Depending on the substance, the concentration of the moisture retention regulators used is between 0.1 and 10% (m/m) and preferably between 0.5 and 5 % (m/m), based on the total weight of a ready-to-use cosmetic or pharmaceutical end product. These data apply in particular to such diols as are advantageously to be used, such as hexylene glycol, 1,2-pentanediol, 1,2-hexanediol, 1,2-octanediol and 1,2-decanediol, as well as mixtures of 1,2-hexanediol and 1,2-octanediol.

The use of cooling agents in the composition or cosmetic and pharmaceutical preparations that contain the composition according to the present invention can alleviate itching. The preparations according to the present invention can therefore also be particularly advantageously combined with one or more cooling agent(s). Preferred individual cooling agents for use within the framework of the present invention are listed below. The person skilled in the art can add many other cooling agents to this list; the cooling agents listed can also be used in combination with one another: l-menthol, d-menthol, racemic menthol, menthone glycerol acetal (trade name: Frescolat^{®} MGA), menthyl lactate (trade name: Frescolat^{®} ML; menthyl lactate is preferably l-menthyl lactate, in particular l-menthyl l-lactate), substituted menthyl-3-carboxamides (such as menthyl-3-carboxylic acid N-ethyl amide), 2-isopropyl-N-2,3-trimethyl butanamide, substituted cyclohexane carboxamides, 3-menthoxypropane-1,2-diol, 2-hydroxyethyl menthyl carbonate, 2-hydroxypropyl menthyl carbonate, N-acetylglycine menthyl ester, isopulegol, menthyl ethylamido oxalate (trade name: Frescolat^{®} X-cool), hydroxycarboxylic acid menthyl esters (such as menthyl 3-hydroxybutyrate), monomenthyl succinate, 2-mercaptocyclodecanone, menthyl 2-pyrrolidin-5-one carboxylate, 2,3-dihydroxy-p-menthane, 3,3,5-trimethyl cyclohexanone glycerol ketal, 3-menthyl-3,6-di- and trioxaalkanoates, 3-menthyl methoxyacetate and icilin.

Cooling agents which are preferred due to their particular synergistic effect are l-menthol, d-menthol, racemic menthol, menthone glycerol acetal (trade name: Frescolat^{®} MGA), menthyl lactate (preferably l-menthyl lactate, in particular l-menthyl l-lactate (trade name: Frescolat^{®} ML), substituted menthyl-3-carboxamides (such as menthyl-3-carboxylic acid N-ethyl amide), 2-isopropyl-N-2,3-trimethyl butanamide, substituted cyclohexane carboxamides, 3-menthoxypropane-1,2-diol, 2-hydroxyethyl menthyl carbonate, 2-hydroxypropyl menthyl carbonate, menthyl ethylamido oxalate (trade name: Frescolat^{®} X-cool), and isopulegol.

Particularly preferred cooling agents are l-menthol, racemic menthol, menthone glycerol acetal (trade name: Frescolat^{®} MGA), menthyl lactate (preferably l-menthyl lactate, in particular l-menthyl l-lactate (trade name: Frescolat^{®} ML), menthyl ethylamido oxalate (trade name: Frescolat^{®} X-cool), 3-menthoxypropane-1,2-diol, 2-hydroxyethyl menthyl carbonate and 2-hydroxypropyl menthyl carbonate.

Very particularly preferred cooling agents are l-menthol, menthone glycerol acetal (trade name: Frescolat^{®} MGA) and menthyl lactate (preferably l-menthyl lactate, in particular l-menthyl l-lactate (trade name: Frescolat^{®} ML).

Depending on the substance, the concentration of the cooling agents used is preferably between 0.01 and 20 wt% and particularly preferably between 0.1 and 5 wt%, based on the total weight of a ready-to-use cosmetic or pharmaceutical end product.

The composition or cosmetic or pharmaceutical preparations that contain the composition according to the present invention can also be used together with one or more osmolyte(s). Examples of osmolytes which may be mentioned here include substances from the group comprising sugar alcohols (myoinositol, mannitol, sorbitol), quaternary amines such as taurine, choline, betaine, betaine glycine, ectoin, diglycerol phosphate, phosphorylcholine or glycerophosphorylcholines, amino acids such as glutamine, glycine, alanine, glutamate, aspartate or proline, phosphatidylcholine, phosphatidylinositol, inorganic phosphates, and polymers of said compounds, such as proteins, peptides, polyamino acids and polyols. All osmolytes simultaneously have a skin-moisturising action.

Preferably, keratolytic substances can also be combined with the composition according to the present invention. Keratolytic compounds include the large group of alpha-hydroxy acids. Salicylic acid is for example preferably used.

In compositions or cosmetic or pharmaceutical preparations that contain the composition according to the present invention for the topical cosmetic or pharmaceutical treatment of for example dry and/or itchy skin, a high proportion of in particular nurturing substances is also particularly advantageous because of the reduced trans-epidermal water loss due to lipophilic components. In one preferred embodiment, the cosmetic or pharmaceutical preparations contain one or more nurturing animal and/or vegetable fats and oils such as olive oil, sunflower oil, refined soybean oil, palm oil, sesame oil, rapeseed oil, almond oil, borage oil, evening primrose oil, coconut oil, shea butter, jojoba oil, sperm oil, tallow, neatsfoot oil and lard, and optionally other nurturing components such as fatty alcohols having 8 to 30 C atoms. The fatty alcohols used here can be either saturated or unsaturated and either linear or branched. Nurturing substances which can be particularly preferably combined with the mixtures according to the present invention also include in particular ceramides, understood here to mean N-acylsphingosines (fatty acid amides of sphingosine) or synthetic analogues of such lipids (so-called pseudo-ceramides) which markedly improve the water retention capacity of the *stratum corneum;* phospholipids, such as soy lecithin, egg lecithin and cephalins; and petrolatum, paraffin oils and silicone oils, the latter including *inter alia* dialkyl- and alkylarylsiloxanes such as dimethylpolysiloxane and methylphenylpolysiloxane and their alkoxylated and quaternised derivatives.

The composition or cosmetic or pharmaceutical preparations that contain the composition according to the present invention can also contain other anti-inflammatory active compounds or active compounds exhibiting anti-reddening and anti-itching activity. Within this context, any anti-inflammatory active compounds and active compounds that alleviate reddening and itching and are suitable or customary in cosmetic and/or dermatological applications can be used. Advantageously, the anti-inflammatory active compounds and active compounds which alleviate reddening and/or itching that are used are steroidal anti-inflammatory substances of the corticosteroid type, such as for example hydrocortisone, dexamethasone, dexamethasone phosphate, methylprednisolone or cortisone, wherein this list may be expanded by adding other steroidal anti-inflammatory agents. Non-steroidal anti-inflammatory agents can also be used, for example: oxicams, such as piroxicam or tenoxicam; salicylates, such as aspirin, Disalcid^{®}, Solprin^{®} or fendosal; acetic acid derivatives, such as diclofenac, fenclofenac, indomethacin, sulindac, tolmetin or clindanac; fenamates, such as mefenamic, meclofenamic, flufenamic or niflumic acid; propionic acid derivatives, such as ibuprofen, naproxen, benoxaprofen; or pyrazoles, such as phenylbutazone, oxyphenylbutazone, febrazone or azapropazone. Alternatively, natural anti-inflammatory substances and substances that alleviate reddening and/or itching can be used. Plant extracts, special highly active plant extract fractions and also highly pure active substances isolated from plant extracts can be used. Extracts, fractions and active substances from camomile, *aloe vera, Commiphora* species, *Rubia* species, willows, willow-herb, ginger, marigold, arnica, Glycyrrhiza species, Echinacea species, Rubus species and pure substances such as *inter alia* bisabolol, apigenin, apigenin-7-glucoside, gingerols such as [6]-gingerol, paradols such as [6]-paradol, boswellic acid, phytosterols, glycyrrhizine, glabridin or licochalcone A are partsincicularly preferred. The preparations containing histamine-release inhibitors can also contain mixtures of two or more anti-inflammatory active compounds.

The composition or cosmetic or pharmaceutical preparations that contain the composition according to the present invention can also contain active compounds for preservative purposes, wherein any preservatives may be used which are suitable or customary in cosmetic and/or dermatological applications and which are advantageously selected from the group consisting of preservatives such as *inter alia* benzoic acid, its esters and salts; propionic acid and its salts; salicylic acid and its salts; 2,4-hexanoic acid (sorbic acid) and its salts; formaldehyde and paraformaldehyde; 2-hydroxybiphenyl ether and its salts; 2-zincsulphidopyridine N-oxide; inorganic sulphites and bisulphites; sodium iodate; chlorobutanol; 4-hydroxybenzoic acid and its salts and esters; dehydroacetic acid; formic acid; 1,6-bis(4-amidino-2-bromophenoxy)-n-hexane and its salts; the sodium salt of ethylmercury-(II)-thiosalicylic acid; phenylmercury and its salts; 10-undecylenic acid and its salts; 5-amino-1,3-bis(2-ethylhexyl)-5-methylhexahydropyrimidine; 5-bromo-5-nitro-1,3-dioxane; 2-bromo-2-nitro-1,3-propanediol; 2,4-dichlorobenzyl alcohol; N-(4-chlorophenyl)-N'-(3,4-dichlorophenyl)urea; 4-chloro-m-cresol; 2,4,4'-trichloro-2'-hydroxy-diphenyl ether; 4-chloro-3,5-dimethylphenol; 1,1'-methylene-bis(3-(1-hydroxymethyl-2,4-dioximidazolidin-5-yl)urea); poly(hexamethylene biguanide) hydrochloride; 2-phenoxyethanol; hexamethylenetetramine; 1-(3-chloroallyl)-3,5,7-triaza-1-azoniaadamantane chloride; 1-(4-chloro-phenoxy)-1(1H-imidazol-1-yl)-3,3-dimethyl-2-butanone; 1,3-bis(hydroxymethyl)-5,5-dimethyl-2,4-imidazolidinedione; benzyl alcohol; Octopirox^{®}; 1,2-dibromo-2,4-dicyanobutane; 2,2'-methylene-bis(6-bromo-4-chloro-phenol); bromochlorophene; mixture of 5-chloro-2-methyl-3(2H)-isothiazolinone and 2-methyl-3(2H)isothiazolinone with magnesium chloride and magnesium nitrate; 2-benzyl-4-chlorophenol; 2-chloroacetamide; chlorhexidine; chlorhexidine acetate; chlorhexidine gluconate; chlorhexidine hydrochloride; 1-phenoxy-propan-2-ol; N-alkyl(C12-C22)trimethylammonium bromide and chloride; 4,4-dimethyl-1,3-oxazolidine; N-hydroxymethyl-N-(1,3-di(hydroxymethyl)-2,5-dioxoimidazolidin-4-yl)-N'-hydroxymethylurea; 1,6-bis(4-amidinophenoxy)-n-hexane and its salts; glutaraldehyde 5-ethyl-1-aza-3,7-dioxabicyclo(3.3.0)octane; 3-(4-chlorophenoxy)-1,2-propanediol; hyamine; alkyl(C8-C18)dimethylbenzylammonium chloride; alkyl(C8-C18)dimethylbenzylammonium bromide; alkyl(C8-C18)dimethylbenzylammonium saccharinate; benzylhemiformal; 3-iodo-2-propynyl butylcarbamate; or sodium ((hydroxymethyl)amino)acetate.

Other antibacterial or antimycotic active substances can also particularly advantageously be used in the compositin or cosmetic or pharmaceutical preparations that contain the composition according to the present invention, wherein any antibacterial or antimycotic active substances can be used which are suitable or customary in cosmetic and/or dermatological applications. In addition to the large group of conventional antibiotics, other products which are advantageous here include those relevant to cosmetics such as in particular triclosan, climbazole, octoxyglycerin, Octopirox^{®} (1-hydroxy-4-methyl-6-(2,4,4-trimethylpentyl)-2(1H)-pyridone 2-aminoethanol salt), chitosan, farnesol, glycerol monolaurate or combinations of said substances, which are used *inter alia* against underarm odour, foot odour or dandruff.

The composition or cosmetic and/or pharmaceutical preparations that contain the composition according to the present invention can also contain one or more lenitive substances, wherein any lenitive substances can be used which are suitable or customary in cosmetic and/or pharmaceutical applications such as alpha-bisabolol, azulene, guaiazulene, 18-beta-glycyrrhetinic acid, allantoin, Aloe vera juice or gel, extracts of Hamamelis virginiana (witch hazel), Echinacea species, Centella asiatica, chamomile, Arnica monatana, Glycyrrhiza species, algae, seaweed and Calendula officinalis, and vegetable oils such as sweet almond oil, baobab oil, olive oil, panthenol, Laureth-9, Trideceth-9 and 4-t-Butylcyclohexanol.

In addition, the composition or cosmetic or pharmaceutical preparations according to the present invention can also particularly advantageously be used in combination with perspiration-inhibiting active compounds (antiperspirants) for controlling body odour. Perspiration-inhibiting active compounds used include in particular aluminium salts, such as aluminium chloride, chlorohydrate, nitrate, sulphate, acetate, etc. The use of zinc, magnesium or zirconium compounds can however also be advantageous. Aluminium salts and, to a somewhat lesser extent, aluminium/zirconium salt combinations have proven useful in cosmetic and dermatological antiperspirants. Partially neutralised aluminium hydroxychlorides, which are therefore more tolerable to the skin but are not quite as effective, are also noteworthy. Substances other than aluminium salts can also be used, such as for example: (a) protein-precipitating substances such as *inter alia* formaldehyde, glutaraldehyde, natural and synthetic tanning agents and trichloroacetic acid, which cause surface closure of the sweat glands; (b) local anaesthetics, including dilute solutions of for example lidocaine, prilocaine or mixtures of the same, which switch off the sympathetic supply to the sweat glands by blocking the peripheral nerve paths; (c) zeolites of the X, A or Y type, which reduce sweat secretion and also act as adsorbents for bad odours; and (d) botulinus toxin (the toxin of the bacterium *Chlostridium botulinum*)*,* which is also used in hyperhidrosis (pathological increase in sweat secretion), and the action of which is based on irreversibly blocking the release of the transmitter substance acetylcholine which is relevant to sweat secretion.

A combination with (metal) chelating agents can also be advantageous in the composition or cosmetic or pharmaceutical preparations that contain the composition according to the present invention, wherein any metal chelating agents can be used which are suitable or customary in cosmetic and/or dermatological applications. Preferred (metal) chelating agents include α-hydroxy fatty acids, phytic acid, lactoferrin, α-hydroxy acids, such as *inter alia* gluconic acid, glyceric acid, glycolic acid, isocitric acid, citric acid, lactic acid, malic acid, mandelic acid, tartaric acid, as well as humic acids, bile acids, bile extracts, bilirubin, biliverdin or EDTA, EGTA and their derivatives.

In order to be used, the composition or cosmetic or pharmaceutical preparations containing the composition according to the present invention are applied to the skin, scalp, hair and/or nail in an adequate amount in such manner as is customary with cosmetics and dermatological products. Within this context, cosmetic and dermatological preparations that contain a composition according to the present invention and which additionally act as a sunscreen offer particular advantages. Advantageously, these preparations contain at least one UVA filter and/or at least one UVB filter and/or at least one inorganic pigment. Within this context, the preparations can take various forms such as are for example customarily employed for this type of preparation, such as for example solutions, water-in-oil (W/O) emulsions, oil-in-water (O/W) emulsions or multiple emulsions such as water-in-oil-in-water (W/O/W) emulsions, gels, hydrodispersions, solid sticks or aerosols.

The compositin or cosmetic or pharmaceutical oreparations that contain the composition according to the present invention can advantageously be combined with substances that absorb UV radiation in the UVB range, the total amount of filter substances being for example 0.01 to 40 % (m/m), preferably 0.1 to 10 % (m/m), in particular 1.0 to 5.0 % (m/m), based on the dry weight of the preparations, in order to provide cosmetic preparations that protect the hair and/or skin against the entire range of ultraviolet radiation. They can also serve as sunscreens for hair. If the preparations according to the present invention contain UVB filter substances, these can be oil-soluble or water-soluble. Advantageous oil-soluble UVB filters include: 3-benzylidene camphor derivatives, preferably 3-(4-methylbenzylidene)camphor, 3-benzylidenecamphor; 4-aminobenzoic acid derivatives, preferably 2-ethylhexyl 4-(dimethylamino)benzoate, amyl 4-(dimethylamino)benzoate; esters of cinnamic acid, preferably 2-ethylhexyl 4-methoxycinnamate, isopentyl 4-methoxycinnamate; esters of salicylic acid, preferably 2-ethylhexyl salicylate, 4-isopropylbenzyl salicylate, homomenthyl salicylate; derivatives of benzophenone, preferably 2-hydroxy-4-methoxybenzophenone, 2-hydroxy-4-methoxy-4'-methylbenzophenone, 2,2'-dihydroxy-4-methoxybenzophenone; esters of benzalmalonic acid, preferably di(2-ethylhexyl) 4-methoxybenzalmalonate, 2,4,6-trianilino-(p-carbo-2'-ethyl-1'-hexyloxy)-1,3,5-triazine. Advantageous water-soluble UVB filters include salts of 2-phenylbenzimidazole-5-sulphonic acid, such as its sodium, potassium or triethanolammonium salts, as well as the sulphonic acid itself; sulphonic acid derivatives of benzophenones, preferably 2-hydroxy-4-methoxybenzophenone-5-sulphonic acid and its salts; sulphonic acid derivatives of 3-benzylidene camphor, such as for example 4-(2-oxo-3-bornylidenemethyl)benzenesulphonic acid, 2-methyl-5-(2-oxo-3-bornylidene-methyl)sulphonic acid and their salts and also 1,4-di(2-oxo-10-sulpho-3-bornylidenemethyl)-benzene and its salts (the corresponding 10-sulphato compounds, such as the corresponding sodium, potassium and triethanolammonium salts), and benzene-1,4-di(2-oxo-3-bornylidenemethyl-10-sulphonic acid.

It can also be advantageous to employ UVA filters, such as are customarily contained in cosmetic preparations. These substances are preferably derivatives of dibenzoylmethane, in particular 1-(4'-tert-butylphenyl)-3-(4'-methoxyphenyl)propane-1,3-dione and 1-phenyl-3-(4'-isopropylphenyl)propane-1,3-dione. The amounts used for the UVB combination can be used analogously.

The composition or cosmetic or pharmaceutical preparations that contain the composition according to the present invention can advantageously also be combined with other auxiliaries or excipients such as are customarily used in such preparations, such as for example antioxidants, perfume oils, anti-foaming agents, colorants, pigments having a colouring action, thickeners, surface-active substances, emulsifiers, plasticising substances, moistening and/or moisture-retaining substances, fats, oils, waxes or other conventional constituents of a cosmetic preparation, such as alcohols, polyols, polymers, foam stabilisers, electrolytes, organic solvents or silicone derivatives. Any conceivable antioxidants, perfume oils, anti-foaming agents, colorants, pigments having a colouring action, thickeners, surface-active substances, emulsifiers, plasticising substances, moistening and/or moisture-retaining substances, fats, oils, waxes, alcohols, polyols, polymers, foam stabilisers, electrolytes, organic solvents or silicone derivatives that are suitable or customary in cosmetic and/or dermatological applications can be used here in accordance with the invention.

A high content of treatment substances is usually advantageous in preparations containing the composition according to the present invention for the topical prophylactic or cosmetic treatment of the skin. In accordance with a preferred embodiment, the compositions contain one or more animal and/or vegetable treatment fats and oils, such as olive oil, sunflower oil, purified soybean oil, palm oil, sesame oil, rapeseed oil, almond oil, borage oil, evening primrose oil, coconut oil, shea butter, jojoba oil, sperm oil, beef tallow, neatsfoot oil and lard, and optionally other treatment constituents such as for example C8-C30 fatty alcohols. The fatty alcohols used here can be saturated or unsaturated and straight-chain or branched, wherein examples include decanol, decenol, octanol, octenol, dodecanol, dodecenol, octadienol, decadienol, dodecadienol, oleyl alcohol, ricinoleyl alcohol, erucic alcohol, stearyl alcohol, isostearyl alcohol, cetyl alcohol, lauryl alcohol, myristyl alcohol, arachidyl alcohol, capryl alcohol, capric alcohol, linoleyl alcohol, linolenyl alcohol and behenyl alcohol, as well their guerbet alcohols; this list may be extended as desired to include other alcohols which structurally are chemically related. The fatty alcohols preferably originate from natural fatty acids and are usually prepared from the corresponding esters of the fatty acids by reduction. Fatty alcohol fractions formed by reduction from naturally occurring fats and fat oils can also be used, such as for example beef tallow, peanut oil, colza oil, cottonseed oil, soybean oil, sunflower oil, palm kernel oil, linseed oil, maize oil, castor oil, rapeseed oil, sesame oil, cocoa butter and cocoa fat.

The treatment substances that can preferably be combined with the composition according to the present invention can also include: ceramides, being understood to be N-acylsphingosines (fatty acid amides of sphingosine) or synthetic analogues of such lipids (so-called pseudo-ceramides) which clearly improve the water retention capacity of the *stratum corneum;* phospholipids, for example soy lecithin, egg lecithin and cephalins; vaseline, paraffin and silicone oils, the latter including *inter alia* dialkyl- and alkylarylsiloxanes such as dimethylpolysiloxane and methylphenylpolysiloxane, as well as their alkoxylated and quaternised derivatives.

Hydrolysed animal and/or vegetable proteins can also advantageously be added to the preparations containing the composition according to the present invention. Advantageous examples in this regard include in particular elastin, collagen, keratin, lactoprotein, soy protein, oat protein, pea protein, almond protein and wheat protein fractions or corresponding hydrolysed proteins, as well as their condensation products with fatty acids, and also quaternised hydrolysed proteins, wherein the use of hydrolysed vegetable proteins is preferred.

If a cosmetic or dermatological preparation containing the composition according to the present invention is a solution or lotion, then solvents which can be used include: water or aqueous solutions; fatty oils, fats, waxes and other natural and synthetic fatty bodies, preferably esters of fatty acids with alcohols having a low C number, such as isopropanol, propylene glycol or glycerol, or esters of fatty alcohols with alkanoic acids having a low C number or with fatty acids; alcohols, diols or polyols having a low C number, and their ethers, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products. Mixtures of the abovementioned solvents are in particular used. In the case of alcoholic solvents, water can be an additional constituent.

The composition or cosmetic or pharmaceutical preparations that contain the composition according to the present invention can also contain antioxidants, wherein any antioxidants suitable or customary in cosmetic and/or dermatological applications can be used. Advantageously, the antioxidants are selected from the group consisting of amino acids (for example glycine, histidine, tyrosine, tryptophan) and their derivatives, imidazoles (for example urocanic acid) and their derivatives, peptides such as D,L-carnosine, D-carnosine, L-carnosine and their derivatives (for example anserine), carotenoids, carotenes (for example α-carotene, β-carotene, lycopene) and their derivatives, lipoic acid and its derivatives (for example dihydrolipoic acid), aurothioglucose, propylthiouracil and other thiols (for example thioredoxin, glutathione, cysteine, cystine, cystamine and their glycosyl, N-acetyl, methyl, ethyl, propyl, amyl, butyl and lauryl, palmitoyl, oleyl, γ-linoleyl, cholesteryl and glyceryl esters) and their salts, dilauryl thiodipropionate, distearyl thiodipropionate, thiodipropionic acid and their derivatives (esters, ethers, peptides, lipids, nucleotides, nucleosides and salts) as well as sulphoximine compounds (for example buthionine sulphoximines, homocysteine sulphoximines, buthionine sulphones, penta-, hexa-, hepta-thionine sulphoximine) in very low tolerated doses, and also (metal) chelating agents, for example α-hydroxy fatty acids, palmitic acid, phytic acid, lactoferrin, α-hydroxy acids (for example citric acid, lactic acid, malic acid), humic acid, bile acid, bile extracts, bilirubin, biliverdin, EDTA, EGTA and their derivatives, unsaturated fatty acids and their derivatives (for example γ-linolenic acid, linoleic acid, oleic acid), folic acid and its derivatives, ubiquinone and ubiquinol and their derivatives, Vitamin C and its derivatives (for example ascorbyl palmitate, magnesium ascorbyl phosphate, ascorbyl acetate), tocopherols and their derivatives (for example Vitamin E acetate), Vitamin A and its derivatives (for example Vitamin A palmitate) and also coniferyl benzoate of benzoin resin, rutinic acid and its derivatives, ferrulic acid and its derivatives, butylhydroxytoluene, butylhydroxyanisole, nordihydroguaiacic acid, nordihydroguaiaretic acid, trihydroxybutyrophenone, uric acid and its derivatives, mannose and its derivatives, zinc and its derivatives (for example ZnO, ZnSO₄), selenium and its derivatives (such as selenium methionine), stilbenes and their derivatives (such as stilbene oxide, trans-stilbene oxide), as well as the derivatives (such as salts, esters, ethers, sugars, nucleotides, nucleosides, peptides and lipids) of said active compounds such as are suitable in accordance with the invention.

The composition or cosmetic or pharmaceutical preparations that contain the composition according to the present invention can also contain vitamins and vitamin precursors, wherein any vitamins and vitamin precursors which are suitable or customary in cosmetic and/or dermatological applications can be used. Particular mention may be made here of vitamins and vitamin precursors such as tocopherols, Vitamin A, nicotinic acid and nicotinamide, other B-complex vitamins, in particular biotin, and Vitamin C. Other examples within this group which are preferably used include pantothenyl alcohol and its derivatives, in particular its esters and ethers, as well as derivatives of pantothenyl alcohols obtained cationically, such as for example pantothenyl alcohol triacetate, pantothenyl alcohol monoethyl ether and its monoacetate and also cationic pantothenyl alcohol derivatives.

The composition or cosmetic or pharmaceutical preparations that contain the composition according to the present invention can also contain active compounds having a skin-lightening action, wherein any skin-lightening active compounds that are suitable or customary in cosmetic and/or dermatological applications can be used in accordance with the invention. Advantageous skin-lightening active compounds in this regard include kojic acid, hydroquinone, arbutin, ascorbic acid, magnesium ascorbyl phosphate, resorcinols, liquorice root extracts and their constituents glabridin or licochalcone A, or extracts from *Rumex* and *Ramulus* species, extracts from pine species (*Pinus*) or extracts from *Vitis* species which contain *inter alia* skin-lightening stilbene derivatives.

The composition or cosmetic preparations that contain the composition according to the present invention can also contain active compounds having a skin-tanning action, wherein any skin-tanning active compounds that are suitable or customary in cosmetic and/or dermatological applications can be used. Dihydroxyacetone (DHA; 1,3-dihydroxy-2-propanone) may be mentioned here by way of example. DHA can be provided in either monomer or dimer form, the proportion of dimers being predominant in the crystalline form.

The composition or cosmetic or pharmaceutical preparations that contain the composition according to the present invention can also contain mono-, di- and oligosaccharides such as for example glucose, galactose, fructose, mannose and lactose.

The composition or cosmetic or pharmaceutical preparations that contain the composition according to the present invention can also contain plant extracts, which are usually prepared by extraction of the complete plant, but which in individual cases are also prepared exclusively from the blossom and/or leaves, wood, bark or roots of the plant. With regard to the plant extracts which can be used in accordance with the present invention, reference is made in particular to the extracts listed in the table starting on page 44 of the third edition of Leitfaden zur Inhaltsstoffdeklaration kosmetischer Mittel (Guide to the Declaration of Constituents of Cosmetic Agents), published by Industrieverband Körperpflegemittel und Waschmittel e.V. (IKW) (Industrial Association for Toiletries and Detergents), Frankfurt. Particularly advantageous extracts include aloe, Hamamelis, algae, oak bark, willow-herb, stinging nettles, dead nettles, hops, camomile, milfoil, arnica, calendula, burdock root, horse-tail, hawthorn, linden blossom, cucumber, almonds, pine needles, horse chestnut, sandalwood, juniper, coconut, mango, apricot, orange, lemon, lime, grapefruit, apple, green tea, grapefruit seed, wheat, oats, barley, sage, thyme, basil, rosemary, birch, mallow, bitter-crass, willow bark, restharrow, coltsfoot, althaea, ginseng and ginger root. Of these, particularly preferred extracts include aloe vera, camomile, algae, rosemary, calendula, ginseng, cucumber, sage, stinging nettles, linden blossom, arnica and Hamamelis. Mixtures of two or more plant extracts can also be employed. Extraction agents that can be used for preparing said plant extracts include water, alcohols and mixtures thereof. Preferred alcohols in this context are the lower alcohols such as ethanol and isopropanol, but also polyhydric alcohols such as ethylene glycol, propylene glycol and butylene glycol, specifically both as a sole extracting agent and in mixtures with water. The plant extracts can be used in pure form or dilute form in accordance with the invention.

The composition or cosmetic or pharmaceutical preparations that contain the composition according to the present invention can also contain anionic, cationic, non-ionic and/or amphoteric surfactants, especially if crystalline or microcrystalline solids, for example inorganic micropigments, are to be incorporated into the preparations according to the present invention. Surfactants are amphiphilic substances that are able to dissolve organic, non-polar substances in water. Surfactants are generally classified according to the nature and charge of the hydrophilic part of the molecule. Four groups can be differentiated here: anionic surfactants, cationic surfactants, amphoteric surfactants and non-ionic surfactants.

Anionic surfactants usually contain carboxylate, sulphate or sulphonate groups as functional groups. In aqueous solution, they form negatively charged organic ions in the acid or neutral medium. Cationic surfactants are characterised almost exclusively by the presence of a quaternary ammonium group. In aqueous solution, they form positively charged organic ions in the acid or neutral medium. Amphoteric surfactants contain both anionic and cationic groups and accordingly behave like anionic or cationic surfactants in aqueous solution, depending on the pH value. They have a positive charge in a strongly acid medium and a negative charge in an alkaline medium. In the neutral pH range, by contrast, they are zwitterionic. Polyether chains are typical of non-ionic surfactants. Non-ionic surfactants do not form ions in an aqueous medium.

Anionic surfactants that can advantageously be used include: acyl amino acids (and their salts), such as: acyl glutamates, for example sodium acyl glutamate, di-TEA-palmitoyl aspartate and sodium caprylic/capric glutamate; acyl peptides, for example palmitoyl-hydrolysed lactoprotein, sodium cocoyl-hydrolysed soy protein and sodium/potassium cocoyl-hydrolysed collagen; sarcosinates, for example myristoyl sarcosinate, TEA-lauroyl sarcosinate, sodium lauroyl sarcosinate and sodium cocoyl sarcosinate; taurates, for example sodium lauroyl taurate and sodium methyl cocoyl taurate; acyl lactylates, for example lauroyl lactylate and caproyl lactylate; alaninates; carboxylic acids and derivatives, such as for example lauric acid, aluminium stearate, magnesium alkanolate and zinc undecylenate; ester carboxylic acids, for example calcium stearoyl lactylate, laureth-6 citrate and sodium PEG-4 lauramide carboxylate; ether carboxylic acids, for example sodium laureth-13 carboxylate and sodium PEG-6 cocamide carboxylate; phosphoric acid esters and salts, such as for example DEA-oleth-10 phosphate and dilaureth-4 phosphate; sulphonic acids and salts, such as acyl isethionates, for example sodium/ammonium cocoyl isethionate; alkyl aryl sulphonates; alkyl sulphonates, for example sodium cocomonoglyceride sulphonate, sodium C12-14 olefin sulphonate, sodium lauryl sulphoacetate and magnesium PEG-3 cocamide sulphate; sulphosuccinates, for example dioctyl sodium sulphosuccinate, disodium laureth sulphosuccinate, disodium lauryl sulphosuccinate and disodium undecylenamido MEA-sulphosuccinate; and sulphuric acid esters, such as alkyl ether sulphate, for example sodium, ammonium, magnesium, MIPA, TIPA laureth sulphate, sodium myreth sulphate and sodium C12-13 pareth sulphate, and alkyl sulphates, for example sodium, ammonium and TEA lauryl sulphate.

Cationic surfactants that can advantageously be used include: alkyl amines, alkyl imidazoles, ethoxylated amines and quaternary surfactants.

Quaternary surfactants contain at least one N atom that is covalently bonded to four alkyl or aryl groups. This leads to a positive charge, irrespective of the pH value. Alkyl betaine, alkyl amidopropyl betaine and alkyl amidopropyl hydroxysulphaine are advantageous. The cationic surfactants used can also preferably be chosen from the group of quaternary ammonium compounds, in particular benzyl trialkyl ammonium chlorides or bromides, such as for example benzyl dimethylstearyl ammonium chloride, as well as alkyl trialkyl ammonium salts, for example cetyl trimethyl ammonium chloride or bromide, alkyl dimethyl hydroxyethyl ammonium chlorides or bromides, dialkyl dimethyl ammonium chlorides or bromides, alkyl amide ethyl trimethyl ammonium ether sulphates, alkyl pyridinium salts, for example lauryl or cetyl pyridinium chloride, imidazoline derivatives and compounds of a cationic nature, such as amine oxides, for example alkyl dimethyl amine oxides or alkyl aminoethyl dimethyl amine oxides. Cetyl trimethyl ammonium salts can particularly advantageously be used.

Amphoteric surfactants that can advantageously be used include: acyl/dialkyl ethylene diamine, for example sodium acyl amphoacetate, disodium acyl amphodipropionate, disodium alkyl amphodiacetate, sodium acyl amphohydroxypropyl sulphonate, disodium acyl amphodiacetate and sodium acyl amphopropionate; N-alkyl amino acids, for example aminopropyl alkyl glutamide, alkyl aminopropionic acid, sodium alkyl imidodipropionate and lauroamphocarboxyglycinate.

Non-ionic surfactants that can advantageously be used include: alcohols; alkanolamides, such as cocamides MEA/DEA/MIPA, amine oxides, such as cocoamidopropylamine oxide; esters formed by esterification of carboxylic acids with ethylene oxide, glycerol, sorbitan or other alcohols; ethers, for example ethoxylated/propoxylated alcohols, ethoxylated/propoxylated esters, ethoxylated/propoxylated glycerol esters, ethoxylated/propoxylated cholesterols, ethoxylated/propoxylated triglyceride esters, ethoxylated/propoxylated lanolin, ethoxylated/propoxylated polysiloxanes, propoxylated POE ethers and alkyl polyglycosides, such as lauryl glucoside, decyl glycoside and cocoglycoside; sucrose esters and ethers; polyglycerol esters, diglycerol esters, monoglycerol esters; methyl glucose esters, ester of hydroxy acids.

The use of a combination of anionic and/or amphoteric surfactants with one or more non-ionic surfactants is also advantageous. The surface-active substance can be present in a concentration of between 1 and 98 % (m/m) in the preparations containing histamine-release inhibitors in accordance with the invention, based on the dry weight of the preparations.

The composition or cosmetic or pharmaceutical preparations that contain the composition according to the present invention can also be formulated in a form suitable for topical application, for example as lotions, aqueous or aqueous-alcoholic gels, vesicle dispersions or as simple or complex emulsions (O/W, W/O, O/W/O or W/O/W), liquids, semi-liquids or solids, such as milks, creams, gels, cream-gels, pastes or sticks, and can optionally be packaged as an aerosol and take the form of mousses or sprays. These compositions are prepared according to usual methods.

For preparing emulsions, the oil phase can advantageously be chosen from the following group of substances: mineral oils, mineral waxes; fatty oils, fats, waxes and other natural and synthetic fatty bodies, preferably esters of fatty acids with alcohols having a low C number, for example with isopropanol, propylene glycol or glycerol, or esters of fatty alcohols with alkanoic acids having a low C number or with fatty acids; alkyl benzoates; silicone oils such as dimethyl polysiloxanes, diethyl polysiloxanes, diphenyl polysiloxanes and mixed forms thereof. Advantageously, esters of saturated and/or unsaturated, branched and/or straight-chain alkane carboxylic acids having a chain length of 3 to 30 C atoms and saturated and/or unsaturated, branched and/or straight-chain alcohols having a chain length of 3 to 30 C atoms, from the group of esters of aromatic carboxylic acids and saturated and/or unsaturated, branched and/or straight-chain alcohols having a chain length of 3 to 30 C atoms can be used. Preferred ester oils include isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl palmitate, 2-ethylhexyl laurate, 2-hexyldecyl stearate, 2-octyldodecyl palmitate, oleyl oleate, oleyl erucate, erucyl oleate, erucyl erucate and synthetic, semi-synthetic and natural mixtures of such esters, for example jojoba oil.

The oil phase can also advantageously be chosen from the group comprising branched and straight-chain hydrocarbons and waxes, silicone oils, dialkyl ethers, the group comprising saturated or unsaturated, branched or straight-chain alcohols, and fatty acid triglycerides, specifically triglycerol esters of saturated and/or unsaturated, branched and/or straight-chain alkane carboxylic acids having a chain length of 8 to 24, in particular 12 to 18 C atoms. The fatty acid triglycerides can for example advantageously be chosen from the group comprising synthetic, semi-synthetic and natural oils, for example olive oil, sunflower oil, soybean oil, peanut oil, rapeseed oil, almond oil, palm oil, coconut oil, palm kernel oil and the like. Arbitrary admixtures of such oil and wax components can also advantageously be used. In some cases, it is also advantageous to use waxes, for example cetyl palmitate, as the sole lipid component of the oil phase; the oil phase is advantageously chosen from the group consisting of 2-ethylhexyl isostearate, octyldodecanol, isotridecyl isononanoate, isoeicosane, 2-ethylhexyl cocoate, C12-15 alkyl benzoate, caprylic-capric acid triglyceride and dicaprylyl ether. Mixtures of C12-15 alkyl benzoate and 2-ethylhexyl isostearate, mixtures of C12-15 alkyl benzoate and isotridecyl isononanoate and mixtures of C12-15 alkyl benzoate, 2-ethylhexyl isostearate and isotridecyl isononanoate are particularly advantageous. The hydrocarbons paraffin oil, squalane and squalene can also advantageously be used. The oil phase can advantageously also contain or consist entirely of cyclic or linear silicone oils, although an additional content of other oil phase components in addition to the silicone oil or oils is preferably used. Cyclomethicone (for example, decamethylcyclopentasiloxane) can advantageously be used as the silicone oil. However, other silicone oils can also advantageously be used, such as for example undecamethylcyclotrisiloxane, polydimethylsiloxane and poly(methylphenylsiloxane). Mixtures of cyclomethicone and isotridecyl isononanoate and of cyclomethicone and 2-ethylhexyl isostearate are also particularly advantageous.

The aqueous phase of preparations that contain the composition according to the present invention and are provided in the form of an emulsion can include: alcohols, diols or polyols having a low C number, and their ethers, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products, as well as alcohols having a low C number, such as ethanol, isopropanol, 1,2-propanediol, glycerol and in particular one or more thickeners, which can advantageously be chosen from the group comprising silicon dioxide, aluminium silicates, polysaccharides and their derivatives, such as hyaluronic acid, xanthan gum, hydroxypropyl methyl cellulose, and particularly advantageously from the group comprising polyacrylates, preferably a polyacrylate from the group comprising so-called carbopols, such as type 980, 981, 1382, 2984, 5984 carbopols, each on their own or in combinations.

The composition or cosmetic or pharmaceutical preparations that contain the composition according to the present invention and provided in the form of an emulsion advantageously contain one or more emulsifiers commonly used in the art for preparing cosmetic or pharmaceutical preparations.

The composition or cosmetic or pharmaceutical preparations containing the composition according to the present invention may also include a cosmetically or pharmaceutically acceptable carrier, such as (without being limited to) one of the following which are commonly used in the art: lactose, dextrose, sucrose, sorbitol, mannitol, starch, acacia rubber, calcium phosphate, alginate, gelatine, calcium silicate, microcrystalline cellulose, polyvinyl pyrrolidone, cellulose, water, syrup, methyl cellulose, methyl hydroxybenzoate, propyl hydroxybenzoate, talc, magnesium stearate, mineral oil and the like. The cosmetic or pharmaceutical preparations may also include lubricants, wetting agents, sweeteners, flavouring agents, emulsifiers, suspensions, preserving agents and the like, in addition to the above components. Suitable pharmaceutically acceptable carriers and preparations are described in detail in Remington's Pharmaceutical Sciences (19th edition, 1995).

The pharmaceutical preparation may be administered topically, orally or parenterally.

A suitable dosage of the pharmaceutical preparation according to the present invention may be variously prescribed depending on factors such as age, body weight, gender or morbid condition, food, administration time, administration route, excretion rate and reaction sensitivity of the patient.

The pharmaceutical composition according to the present invention may be manufactured in a unit dosage form, by being formulated using the pharmaceutically acceptable carrier and/or excipient according to a method that can be easily executed by an average person skilled in the art to which the present invention pertains.

In a further aspect, the present invention relates to the use of 4-hydroxyacetophenone for improving the solubility of an avenanthramide or an analogue thereof or a composition comprising an avenanthramide or an analogue thereof, in particular avenanthramide A or avenanthramide L or Dihydroavenanthramide D.

Finally, the present invention relates to a method for improving the solubility of at least one avenanthramide or an avenanthramide analogue compound, preferably avenanthramide A or avenanthramide L or Dihydroavenanthramide D, in a composition, whereby 4-hydroxyacetophenone is added to the composition.

While the invention has been specifically shown and described with reference to preferred variants, it will be understood by those skilled in the art that various changes in form and detail may be made to it without departing from the spirit and scope of the invention. Moreover, the invention encompasses any combination of the elements described above, in all possible variations, unless specifically indicated otherwise.

In addition, where features or aspects of the invention are described in terms of Markush groups, those skilled in the art will recognise that the invention is also thereby described in terms of any individual member or sub-group of members of the Markush group.

The present invention shall now be described in detail with reference to the following examples, which are merely illustrative of the present invention, such that the content of the present invention is not limited by or to the following examples.

### Examples

### Example 1: Influence of 4-hydroxyacetophenone on the solubility of avenanthramide A, B, C, D or L and Dihydroavenanthramide D

The solubility of avenanthramides A, B, C, D or L and Dihydroavenanthramide D in solution in combination with 4-hydroxyacetophenone in different concentrations was evaluated against a solution of avenanthramides A, B, C, D and L without 4-hydroxyacetophenone.

The corresponding avenanthramides were presolved in ethanol or glycol. Water and 4-hydroxyacetophenone were added under stirring and heating up the solution to 60 °C. The appearance of the solutions was evaluated at 22 °C according to the following Table 1:

**Table 2: Appearance coding**

| **Precipitation** | **Turbidity** |
|---|---|
| clear (C) | clear ( C ) |
| precipitation (P) | turbid (T) |
| strong precipitation (P+) | strong turbididity (T+) |
| very strong precipitation (P++) | strong turbididity (T++) |

**Table 3: Solutions with avenanthramide A**

| | w/w% | | | |
|---|---|---|---|---|
| Solutions | placebo | B | C | D |
| Ethanol 99 % | 30.00 | 30.00 | 30.00 | 30.00 |
| Avenanthramide A | 0.03 | 0.03 | 0.03 | 0.03 |
| 4-Hydroxyacetophenone | - | 0.05 | 0.10 | 0.50 |
| Water, demin. | 69.97 | 69.92 | 69.87 | 69.47 |
| Σ | 100.00 | 100.00 | 100.00 | 100.00 |
| Appearance | P++ | P | C | C |
| | T+ | | | |

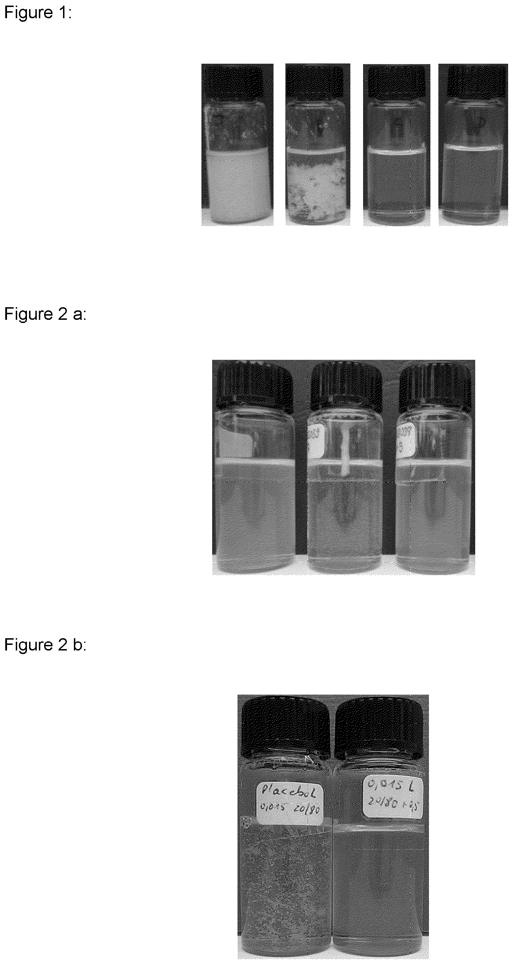

The appearance of the different solutions is shown in Figure 1.

Table 4: Solutions with aventhramide L

**Table 4 a:**

| | | w/w% | |
|---|---|---|---|
| Solutions | placebo | A | B |
| Ethanol 99 % | 30.00 | 30.00 | 30.00 |
| Avenanthramide L | 0.01 | 0.01 | 0.01 |
| 4-Hydroxyacetophenone | - | 0.05 | 0.10 |
| Water demin. | 69.99 | 69.94 | 69.89 |
| Σ | 100.00 | 100.00 | 100.00 |
| Appearance | P+ | P | T |
| | T+ | | |

It could be clearly observed that with increasing concentration of 4-hydroxyacetophenone the solubility of Avn L was improved. By further increasing the concentration to 0.5% as in Table 4 b, a clear solution was obtained.

**Table 4 b:**

| | | w/w% |
|---|---|---|
| Solutions | placebo | C |
| Ethanol 99 % | 20.00 | 20.00 |
| Avenanthramide L | 0.015 | 0.015 |
| 4-Hydroxyacetophenone | - | 0.50 |
| Water demin. | 79.85 | 79.35 |
| Σ | 100.00 | 100.00 |
| Appearance | P | C |
| | T | |

The appearance of the different solutions is shown in Figures 2 a and 2 b.

**Table 5: Solutions with a blend of avenanthramides A and B**

| | | w/w% | |
|---|---|---|---|
| Solutions | placebo | B | C |
| Ethanol 99 % | 30.0 | 30.0 | 30.0 |
| Avenanthramide A | 0.0175 | 0.0175 | 0.0175 |
| Avenanthramide B | 0.0175 | 0.0175 | 0.0175 |
| 4-Hydroxyacetophenone | | 0.200 | 0.500 |
| Water, demin. | 69.965 | 69.765 | 69.465 |
| Σ | 100.0 | 100.0 | 100.0 |
| Appearance | T+ | T+ | T |
| | P+ | | |

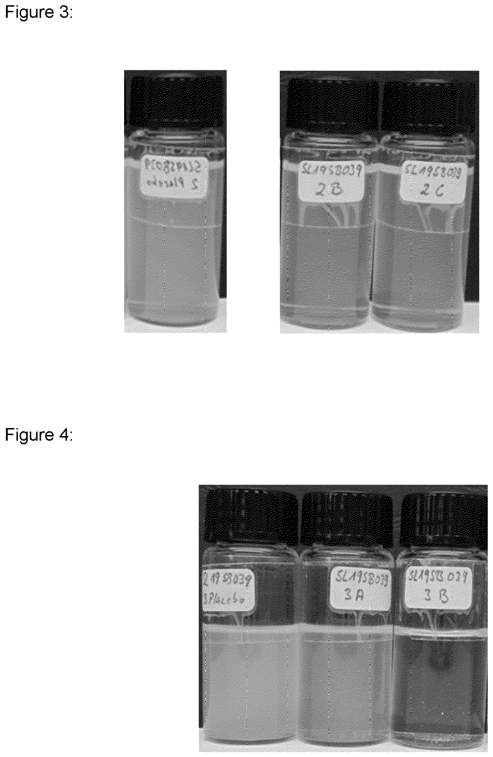

The appearance of the different solutions is shown in Figure 3.

**Table 6: Solutions with a blend of avenanthramides B, C and D**

| | w/w% | | |
|---|---|---|---|
| Solutions | placebo | A | B |
| Ethanol 99 % | 30.00 | 30.00 | 30.00 |
| Avenanthramide B | 0.03 | 0.03 | 0.03 |
| Avenanthramide C | 0.03 | 0.03 | 0.03 |
| Avenanthramide D | 0.01 | 0.01 | 0.01 |
| 4-Hydroxyacetophenone | - | 0.10 | 0.50 |
| Water demin. | 69.93 | 69.83 | 69.43 |
| Σ | 100.0 | 100.0 | 100.0 |
| Appearance | P | T | C |
| | T | | |

The appearance of the different solutions is shown in Figure 4.

Table 7: Solutions with Dihydroavenanthramide D

**Table 7 a:**

| | w/w% | | | |
|---|---|---|---|---|
| Solutions | 1A | 2A | 3A | 4A |
| Dihydroavenanthramide D (INCI Hydroxyphenyl Propamidobenzoic Acid) | 0.5 | 0.5 | 0. 5 | 0.5 |
| Butylene glycol | 20.0 | 20.0 | 20.0 | 20.0 |
| Hydrolite 5 (Pentylene Glycol) | 20.0 | 20.0 | 20.0 | 20. 0 |
| Water | 59.5 | 59.2 | 59. 0 | 58.,5 |
| SymSave H (4-Hydroxyacetophenone) | - | 0.3 | 0.5 | 1.0 |
| Appearance | P+ | P+ | P | C |

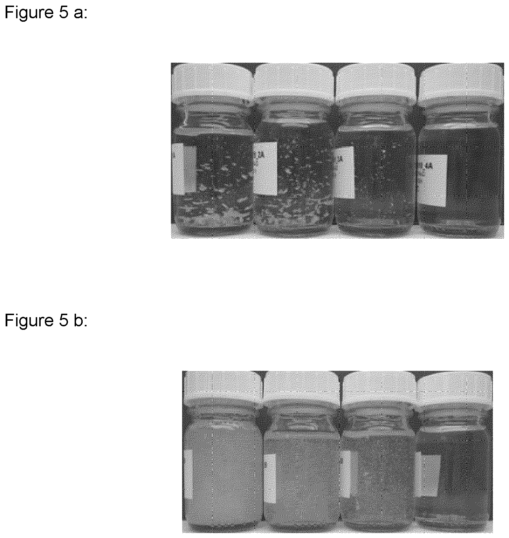

The appearance of the different solutions is shown in Figure 5 a.

**Table 7 b:**

| | w/w% | | | |
|---|---|---|---|---|
| Solutions | 1B | 2B | 3B | 4B |
| Dihydroavenanthramide D (INCI Hydroxyphenyl Propamidobenzoic Acid) | 1.0 | 1.0 | 1.0 | 1.0 |
| Butylene glycol | 20.0 | 20.0 | 20.0 | 20.0 |
| Hydrolite 5 | 20.0 | 20.0 | 20.0 | 20.0 |
| Water | 59.0 | 58.7 | 58.5 | 58.0 |
| SymSave H (4-Hydroxyacetophenone) | - | 0.3 | 0.5 | 1.0 |
| Appearance | P++ | P++ | P | C |

The appearance of the different solutions is shown in Figure 5 b.

**Table 8:**

| **Examples/ Solutions** | **Avenanthramide(s)** | **Concentration** | **EtOH (conc)** | **4-Hydroxyacetophenone (concentration)** | **Ratio Avenanthramide/Dihydroavenanthramide D and 4-hydroxyacetophenone** |
|---|---|---|---|---|---|
| Table 2 | A | 0.03 % | 30 % | 0.05 / 0.1 / 0.5% | 1 : 1. 7 / 1 : 3. 3 / 1 : 16. 7 |
| Table 3 a | L | 0.01 % | 30 % | 0,05 / 0,1 % | 1 : 5 / 1:10 |
| Table 3 b | L | 0.015 % | 20 % | 0,5 % | 1 : 33.3 |
| Table 4 | A + B | 0.0175 % each | 30 % | 0.2 / 0.5 % | 1 : 11.4 / 1 : 28.6 |
| Table 5 | B + C + D | 0.03 + 0.03 + 0.01 % | 30 % | 0.1 / 0.5 % | 1 : 3.3 / 1 : 16.7 |
| Table 6 a | Dihydroavenanthramide D | 0.5 % | - | 0.3 / 0.5 / 1.0 % | 1 : 0.6 / 1 : 1 /1 : 2 |
| Table 6 b | Dihydroavenanthramide D | 1.0 % | - | 0.3 / 0.5 / 1.0 % | 1 : 0.3 / 1 : 0.5 / 1 : 2 |

The results in Table 3 to Table 7 clearly show that the addition of 4-hydroxyacetophenone to the avenanthramide or Dihydroavenanthramide D solutions leads to a more transparent solution with less precipitation.

In particular, the addition of 4-hydroxyacetophenone to the avenanthramide A (Table 3) and avenanthramide L (Table 4 b) solutions in concentrations of 0.1 or 0.5 wt% leads to clear solutions without turbidity or precipitation.

Furthermore, in particular the addition of 4-hydroxyacetophenone to the dihydroavenanthramide D solutions (Tables 7 a and 7 b) in concentrations of 0.5 to 1.0% leads to clear solutions withous turbidity or precipitation.

The results in Table 8 also show that the preferred ratio of avenanthramide to 4-hydroxyacetophenone is 1 : 1 to 1 : 50, more preferred a ratio of 1 : 1.5 to 1 : 40 and the preferred ratio of Dihydroavenanthramide D to 4-hydroxyacetophenone is 1 : 0.2 to 1 : 2, more preferred a ratio of 1 : 0.5.

### Example 2: Formulation examples

**Table 9: Perfume oil 1 (PO1) (amounts in %o byweight.)**

| **Ingredients** | **Amount** |
|---|---|
| ALDEHYDE C14 SO-CALLED | 2 |
| ALLYL AMYL GLYCOLATE 10% DPG | 5 |
| ANISIC ALDEHYDE PURE | 5 |
| APPLE OLIFFAC TYPE | 10 |
| Benzylacetat | 50 |
| BERGAMOT IDENTOIL^{®} COLOURLESS | 15 |
| CANTHOXAL | 5 |
| CETALOX 10% IPM | 3 |
| CITRONELLOL 950 | 40 |
| DAMASCENONE TOTAL 1% DPG | 5 |
| DAMASCONE ALPHA 10% DPG | 5 |
| DAMASCONE DELTA 10% DPG | 2 |
| DIMETHYL BENZYL CARBINYL BUTYRATE | 2 |
| DIPROPYLENE GLYCOL | 178 |
| EBANOL | 2 |
| ETHYL DECADIENOATE TRANS CIS-2.4 10% IPM | 2 |
| FLOROSA | 5 |
| FRAMBINON^{®} 10% DPG | 7 |
| GALAXOLIDE 50% IN IPM | 100 |
| GALBEX TYPE BASE | 1 |
| GERANYL ACETATE PURE | 2 |
| HEDIONE | 30 |
| HELIOTROPIN | 10 |
| HEXENYL ACETATE CIS-3 10% DPG | 1 |
| HEXENYL SALICYLATE CIS-3 | 5 |
| HEXYL CINNAMIC ALDEHYDE ALPHA | 70 |
| HEXYL SALICYLATE | 50 |
| HYDROXY CITRONELLAL | 10 |
| ISO E SUPER | 15 |
| ISORALDEINE 70 | 17 |
| Jasmol (2 benzylheptanol) | 3 |
| LEAFOVERT^{®} | 1 |
| LILIAL | 60 |
| LINALOOL | 60 |
| LINALYL ACETATE | 20 |
| LYRAL | 7 |
| MANZANATE | 2 |
| PHENOXANOL | 7 |
| PHENYLETHYL ALCOHOL | 120 |
| SANDAL MYSORE CORE | 2 |
| SANDRANOLO | 7 |
| STYRALYL ACETATE | 3 |
| TAGETES RCO 10% TEC | 2 |
| TERPINEOL PURE | 20 |
| TETRAHYDROGERANIOL 10% DPG | 5 |
| TONALIDE | 7 |
| VERTOCITRAL 10% DPG | 5 |
| VERTOFIX | 15 |
| **Total** | **1000** |

**Table10: Perfume oil 2 (PO2) (amounts in %o by weight)**

| **Ingredients** | **Amount** |
|---|---|
| Acetophenone, 10% in DPG | 10 |
| n-Undecanal | 5 |
| Aldehyde C14. so-called (peach aldehyde) | 15 |
| Allylamyl glycolate, 10% in DPG | 20 |
| Amyl salicylate | 25 |
| Benzyl acetate | 60 |
| Citronellol | 80 |
| d-Limonene | 50 |
| Decenol trans-9 | 15 |
| Dihydromyrcenol | 50 |
| Dimethylbenzylcarbinyl acetate | 30 |
| Diphenyloxide | 5 |
| Eucalyptol | 10 |
| Geraniol | 40 |
| Nerol | 20 |
| Geranium oil | 15 |
| Hexenol cis-3, 10% in DPG | 5 |
| Hexenyl salicylate cis-3 | 20 |
| Indole, 10% in DPG | 10 |
| Alpha-ionone | 15 |
| Beta-ionone | 5 |
| Lilial^{®} (2-methyl-3-(4-tert-butylphenyl)propanal) | 60 |
| Linalool | 40 |
| Methylphenyl acetate | 10 |
| Phenylethyl alcohol | 275 |
| Styrolyl acetate | 20 |
| Terpineol | 30 |
| Tetrahydrolinalool | 50 |
| Cinnamyl alcohol | 10 |
| **Total:** | **1000** |

**Table11: Perfume oil 3 (PO3) (amounts in %o by weight)**

| **Ingredients** | **Amount** |
|---|---|
| Benzyl acetate | 60 |
| Citronellyl acetate | 60 |
| Cyclamenaldehyde (2-methyl-3-(4-isopropylphenyl)propanal | 20 |
| Dipropylene glycol (DPG) | 60 |
| Ethyllinalool | 40 |
| Florol (2-isobutyl-4-methyltetrahydro-2H-pyran-4-ol) | 30 |
| Globanone^{®} [(E/Z)-8-cyclohexadecen-1-one] | 180 |
| Hedione^{®} (methyldihydrojasmonate) | 140 |
| Hexenyl salicylate, cis-3 | 10 |
| Vertocitral (2.4-dimethyl-3-cyclohexenecarboxaldehyde) | 5 |
| Hydratropaldehyde, 10% in DPG | 5 |
| Isodamascone (1-(2.4.4-trimethyl-2-cyclohexen-1-yl)-2-buten-1-one, 10% in DPG | 5 |
| Isomuscone (cyclohexadecanone) | 40 |
| Jacinthaflor (2-methyl-4-phenyl-1.3-dioxolane) | 10 |
| Cis-jasmone, 10% in DPG | 20 |
| Linalool | 50 |
| Linalyl acetate | 30 |
| Methyl benzoate, 10% in DPG | 25 |
| para-Methyl cresol, 10% in DPG | 10 |
| Nerol | 20 |
| Phenylpropylaldehyde | 5 |
| 2-Phenylethyl alcohol | 82 |
| Tetrahydrogeraniol | 13 |
| 2.2-Dimethyl-3-cyclohexyl-1-propanol | 80 |
| **Total:** | **1000** |

**Table 12: Perfume oil 4 (PO4) (amounts in %o by weight)**

| **Ingredients** | **Amount** |
|---|---|
| AMBRETTOLIDE (MACRO) | 10 |
| AMBROXIDE 10% in IPM | 10 |
| BENZYL ACETATE | 20 |
| BENZYL SALICYLATE | 15 |
| BERGAMOT OIL. bergapten-free | 60 |
| CALONE^{®} 1951 10% in DPG | 15 |
| COUMARIN | 5 |
| CYCLOGALBANATE^{®} 10% in DPG | 10 |
| ALPHA -DAMASCONE 1% in DPG | 20 |
| DIHYDROMYRCENOL | 10 |
| ETHYL LINALOOL | 75 |
| ETHYL LINALYLACETATE | 50 |
| ETHYL MALTOL 1% in DEP | 10 |
| ETHYLENE BRASSYLATE (MACRO) | 80 |
| FLOROSA | 40 |
| GERANYLACETATE | 10 |
| HEDIONE^{®} HC/30 | 35 |
| HEDIONE^{®} | 210 |
| HELIONAL^{®} | 15 |
| HELVETOLIDE^{®} (ALICYC) | 30 |
| HEXENYLSALICYLATE CIS-3 | 20 |
| ISO E SUPER^{®} | 40 |
| LEAFOVERT^{®} , 10% in DEP | 10 |
| LILIAL^{®} | 80 |
| LYRAL^{®} | 20 |
| MANDARIN OIL | 10 |
| STYRALYL ACETATE | 5 |
| SYMROSE^{®} | 15 |
| VANILLIN, 10% in DEP | 20 |
| DIPROPYLENE GLYCOL (DPG) | 50 |
| TOTAL | 1000 |

**Table 13: Perfume oil 5 (PO5) (amounts in %o by weight)**

| **Ingredients** | **Amount** |
|---|---|
| AMAROCITE^{®} | 10 |
| AMBROCENIDE^{®} 10% in DPG | 5 |
| AMBROXIDE | 15 |
| AURELIONE^{®} (7/8-Cyclohexadecenone) (MACRO) | 70 |
| BERGAMOT OIL. bergapten-free | 90 |
| CALONE^{®} 1951 10% in DPG | 20 |
| CARAWAY OIL | 10 |
| CITRAL | 20 |
| COUMARIN | 10 |
| ALPHA-DAMASCONE, 1% in DPG | 15 |
| DIHYDROMYRCENOL | 70 |
| ESTRAGON OIL | 10 |
| ETHYL LINALOOL | 100 |
| ETHYL LINALYLACETATE | 90 |
| EUGENOL | 10 |
| EVERNYL^{®} | 5 |
| FRUCTATE^{®} | 5 |
| GERANIUM OIL | 5 |
| HEDIONE^{®} HC/30 | 100 |
| HELIONAL^{®} | 10 |
| INDOLE, 10% in DPG | 5 |
| ISO E SUPER^{®} | 100 |
| KEPHALIS^{®} | 5 |
| LAVENDER OIL | 40 |
| CITRUS OIL | 80 |
| LILIAL^{®} | 30 |
| MANDARIN OIL | 20 |
| MUSCENONE (MACRO) | 5 |
| SANDRANOL^{®} | 10 |
| VANILLIN 10% in DPG | 5 |
| DIPROPYLENE GLYCOL | 30 |
| **TOTAL** | **1000** |

The above perfume oils PO1, PO2, PO3, PO4, or PO5 were worked separately in each case into the preparations/formulations presented below.

Cosmetic preparations/formulations (amounts in % by weight for all preparations/formulations)

**Table 14: Cream, o/w**

| **Ingredients** | **INCI** | **Amount** |
|---|---|---|
| Dracorin^{®} CE | Glyceryl Stearate Citrate | 1.0 |
| Lanette^{®} O | Cetearyl Alcohol | 2.0 |
| Cutina^{®} GMS-V | Glyceryl Stearate | 1.0 |
| Tegosoft^{®} MM | Myristyl Myristate | 1.0 |
| Xiameter^{®} PMX-0246 | Cyclohexasiloxane, Cyclopentasiloxane | 0.5 |
| Dragoxat^{®} 89 | Ethylhexyl Isononanoate | 2.0 |
| PCL-Liquid 100 | Cetearyl Ethylhexanoate | 4.0 |
| Neutral Oil | Caprylic/Capric Triglyceride | 4.0 |
| Carbopol^{®} Ultrez 21 | Acrylates/C10-30 Alkyl Acrylate Crosspolymer | 0.2 |
| Keltrol^{®} CG-T | Xanthan Gum | 0.1 |
| Water | Water (Aqua) | ad 100 |
| Glycerol 99.5 P. | Glycerol | 3.0 |
| Hydrolite CG | Caprylyl Glycol | 0.2 |
| 1.2-Propylene Glycol 99 P GC | Propylene Glycol | 2.0 |
| Sodium Benzoate | Sodium Benzoate | 0.1 |
| Sodium Hydroxide (10% solution) | Sodium Hydroxide | 0.5 |
| Perfume oil PO1, PO2, PO3, PO4, or PO5 | Perfume | 0.3 |
| Euxyl^{®} K702 | Dehydroacetic Acid, Benzoic Acid, Phenoxyethanol, Polyaminoprop yl Biguanide, Ethylhexylglycerin | 0.3 |
| Avenanthramide A, B and C ≥ | | 1.0 |
| 100 ppm in sum in glycerine /water | | |
| Avenanthramide A | Avenanthramide A | 0.1 |

**Table 15: Hand and body cream**

| **Ingredients** | **INCI** | **Amount** |
|---|---|---|
| Dracorin^{®} GOC | Glyceryl Oleate Citrate, Caprylic/Capric Triglyceride | 2.0 |
| PCL-Solid | Stearyl Heptanoate, Stearyl Caprylate | 2.5 |
| Lanette^{®} O | Cetearyl Alcohol | 1.5 |
| Cutina^{®} GMS-V | Glyceryl Stearate | 1.0 |
| Dragoxat^{®} 89 | Ethylhexyl Isononanoate | 3.0 |
| PCL-Liquid 100 | Cetearyl Ethylhexanoate | 7.0 |
| Isodragol^{®} | Triisononanoin | 4.0 |
| Xiameter^{®} PMX-0345 Cyclosiloxane | Cyclopentasiloxane (and) Cyclohexasiloxane | 0.5 |
| Water | Water (Aqua) | ad 100 |
| Carbopol^{®} Ultrez 21 | Acrylates/C10-30 Alkyl Acrylate Crosspolymer | 0.2 |
| Keltrol^{®} CG-RD | Xanthan Gum | 0,1 |
| Glycerol 85 P, | Glycerol | 3,0 |
| DragoBetaGlucan | Water (Aqua), Butylene Glycol, Glycerol, Avena Sativa (Oat) Kernel Extract | 1,5 |
| Potassium Sorbate | Potassium Sorbate | 0,1 |
| Hydrolite-6 | 1,2 Hexanediol | 1,0 |
| Sodium Hydroxide (10% solution) | Sodium Hydroxide | 0,5 |
| Perfume oil PO1, PO2, | Fragrance | 0,2 |
| PO3, PO4, or PO5 | | |
| Avenanthramide L | Avenanthramide L | 0,1 |
| SymSave H | Hydroxyacetophenone | 0,5 |

**Table 16: Daily face cream SPF 20**

| **Ingredients** | **Amount** |
|---|---|
| **SymOcide PH** | 1 |
| Phenoxyethanol, Hydroxyacetophenone, Caprylyl Glycol, Water (Aqua) | |
| **Ascorbyl Palmitate** | 0,1 |
| Ascorbyl Palmitate | |
| **Biotive L-Arginine** | 0,2 |
| Arginine | |
| **Buriti** oil | 1 |
| Mauritia Flexuosa Fruit Oil | |
| **Cocoa butter** | 2 |
| Theobroma Cacao (Cocoa) Seed Butter | |
| **Dimethicone** | 0,5 |
| Dimethicone | |
| **Disodium EDTA** | 0,1 |
| Disodium EDTA | |
| **Dragosantol 100** | 0,1 |
| Bisabolol | |
| **Dragoxat 89** | 5 |
| Ethylhexyl Isononanoate | |
| **Emulsiphos** | 2 |
| Potassium Cetyl Phosphate, Hydrogenated Palm Glycerides | |
| **Extrapone Corail** | 1 |
| Glycerin, Aqua, Hydrolyzed Corallina Officinalis | |
| **Glycerin** | 3 |
| Glycerin | |
| **Isoadipate** | 5 |
| Diisopropyl Adipate | |
| **Jojoba Wax Flakes** | 1 |
| Hydrogenated Jojoba Oil | |
| **Keltrol CG-T** | 0.1 |
| Xanthan Gum | |
| **Lanette O** | 5 |
| Cetearyl Alcohol | |
| **Lanette 16** | 1 |
| Cetyl Alcohol | |
| **Lanette 22** | 1 |
| Behenyl Alcohol | |
| **Neo Heliopan 357** | 3 |
| Butyl Methoxydibenzoylmethane | |
| **Neo Heliopan HMS** | 10 |
| Homosalate | |
| **Neo Heliopan Hydro** used as a 25% aq, Solution | 8 |
| neutralized by arginine Phenylbenzimidazole Sulfonic Acid | |
| **Neo Heliopan OS** | 5 |
| Ethylhexyl Salicylate | |
| **Orgasol Caresse** | 1 |
| Polyamide-5 | |
| **Perfume oil PO1, PO2, PO3, PO4, or PO5** | 0.1 |
| **Shea butter** | 3 |
| Butyrospermum Parkii (Shea) Butter | |
| **Simugel EG** | 1 |
| Sodium Acrylate / Sodium Acryloyldimethyl Taurate Copolymer. Isohexadecane. Polysorbate 80 | |
| **SymFinity 1298** | 0.1 |
| Echinacea Purpurea Extract | |
| **SymDiol 68** | 0.5 |
| 1, 2 Hexanediol, Caprylyl Glycol | |
| **SymMatrix** | 0.1 |
| Maltodextrin, Rubus Fructicosus (Blackberry) Leaf Extract | |
| **SymSitive 1609** | 1 |
| Pentylene Glycol, 4-t-Butylcyclohexanol | |
| **Tegosoft TN** | 4 |
| C12-15 Alkyl Benzoate | |
| Avenanthramide L | 0.001 |
| SymSave H (Hydroxyacetophenone) | 0.1 |
| **Water** | ad 100 |
| Aqua | |

**Table 17: night cream, w/o**

| **Ingredients** | **INCI** | **Amount** |
|---|---|---|
| Avenanthramide A | Avenanthramide L | 0.005 |
| SymSave H | Hydroxyacetophenone | 0.3 |
| Avenanthramide B | Avenanthramide B | 0.005 |
| Aloe Vera Gel Concentrate 10/1 * | Water (Aqua), Aloe Barbadensis Leaf Juice | 3.0 |
| Alugel 34 TH | Aluminium Stearate | 1.0 |
| Dragosan W/O P* | Sorbitan Isostearate, Hydrogenated Castor Oil, Ceresin, Beeswax (Cera Alba) | 6.0 |
| Dragosantol^{®} 100* | Bisabolol | 0.2 |
| Extrapone^{®} Witch Hazel Distillate colourless | Propylene Glycol, Hamamelis Virginiana (Witch Hazel) Water, Water (Aqua), Hamamelis Virginiana (Witch Hazel) Extract | 1.0 |
| Perfume oil PO1, PO2, PO3, PO4, or PO5 | Fragrance | 0.4 |
| Glycerol 85 % | Glycerin | 2.0 |
| Hydrolite-5 | Pentylene Glycol | 0.5 |
| Karion F | Sorbitol | 2.0 |
| Magnesium Chloride | Magnesium Chloride | 0.7 |
| PCL Liquid 100 | Cetearyl Ethylhexoate | 12.0 |
| Retinyl Palmitate in Oil | Retinyl Palmitate | 0.2 |
| Sun Flower Oil | Helianthus Annuus (Sunflower) Seed Oil | 5.0 |
| Sweet Almond Oil | Prunus dulcis | 5.0 |
| SymMatrix^{®} | Maltodextrin, Rubus Fruticosus (Blackberry) Leaf Extract | 1.0 |
| SymOcide PS | Phenoxyethanol, Decylene glycol, 1,2-Hexanediol | 0.5 |
| SymVital^{®} AgeRepair | Zingiber Officinale (Ginger) Root Extract | 0.1 |
| Tocopherol Acetate | Tocopheryl Acetate | 3.0 |
| Water (demineralized) | Water (Aqua) | ad 100 |

**Table 18: Body lotion**

| **Ingredients** | **Amount** |
|---|---|
| Cetearyl Alcohol | 2.0 |
| Ethylhexyl Isononanoate | 5.0 |
| Cetearyl Ethylhexanoate, Isopropyl Myristate | 3.0 |
| Glyceryl Oleate Citrate, Caprylic/Capric Triglyceride | 4.0 |
| Water (Aqua) | ad 100 |
| Pentylene Glycol | 3.0 |
| Carbomer | 0.3 |
| Sodium Benzoate | 0.1 |
| Propylene Glycol | 5.0 |
| Sodium Hydroxide (30% solution) | 0.3 |
| Perfume oil PO1, PO2, PO3, PO4, or PO5 | 0.3 |
| 4-Hydroxyacetophenone | 0.3 |
| Avenanthramide L | 0.02 |
| Avenanthramide B | 0.02 |

**Table 19: Antibacterial body lotion, sprayable**

| **Ingredients** | **INCI** | **Amount** |
|---|---|---|
| Avenanthramide A, B and C ≥ 100 ppm in sum in glycerine /water | | 1.5 |
| SymSave H | Hydroxyacetophenone | 0.5 |
| Avenanthramide A | Avenanthramide A | 0.0005 |
| Triethyl Citrate | Triethyl Citrate | 0.2 |
| 2,4-Hexadienoic acid, potassium salt | Sorbic acid, potassium salt | 0.2 |
| Dow Corning 345 Fluid | Cyclomethicone | 0.5 |
| Dracorin^{®} GOC | Glyceryl Oleate Citrate, Caprylic/Capric Triglyceride | 2.0 |
| Drago-Calm | Water, Glycerin, Avena Sativa (Oat) Kernel Extract | 1.0 |
| Dragosantol^{®} 100* | Bisabolol | 0.1 |
| Perfume oil PO1, PO2, PO3, PO4, or PO5 | Fragrance | 0.3 |
| Hydrolite^{®}-5 | Pentylene Glycol | 5.0 |
| Neutral Oil | Caprylic/Capric Triglyceride | 4.0 |
| Paraffin Oil | Mineral Oil | 4.0 |
| PCL Liquid 100 | Cetearyl Ethylhexoate | 7.0 |
| Pemulen TR-2 | Acrylates/C10-30 Alkyl Acrylate Crosspolymer | 0.2 |
| Sodium Hydroxide (10% solution) | Sodium Hydroxide | 0.4 |
| SymDeo^{®} MPP | Dimethyl Phenylbutanol | 0.5 |
| SymRelief^{®} 100 | Bisabolol, Zingiber Officinale (Ginger) Root Extract | 0.1 |
| Water (demineralized) | Water (Aqua) | ad 100 |

**Table 20: Aseptic wound cream**

| **Ingredients** | **Amount** |
|---|---|
| Sorbitan Isostearate, Hydrogenated Castor Oil, Ceresin, Beeswax (Cera Alba) | 6.0 |
| Petrolatum | 21.0 |
| Cera Alba | 5.0 |
| Cetearyl Alcohol | 7.0 |
| Prunus Dulcis | 7.0 |
| Lanolin | 5.0 |
| Paraffinum Liquidum | 12.0 |
| Perfume oil PO1, PO2, PO3, PO4, or PO5 | 0.3 |
| Water (Aqua) | ad 100 |
| Panthenol | 7.0 |
| Magnesium Sulfate | 0.7 |
| Pentylene Glycol (Hydrolite-5 Green) | 1.0 |
| Tocopheryl Acetate | 1.0 |
| Octenidine dihydrochloride | 0.1 |
| Phenoxyethanol | 0.5 |
| Avenanthramide L | 0.001 |
| Hydroxyacetophenone (SymSave H) | 0.5 |

**Table 21: Anti acne balm**

| **Ingredients** | **INCI** | **Amount** |
|---|---|---|
| Avenanthramide L | Avenanthramide L | 0.0005 |
| Avenanthramide A | Avenanthramide A | 0.005 |
| SymSave H | Hydroxyacetophenone | 0.5 |
| Abil 350 | Dimethicone | 1.0 |
| Allantoin | Allantoin | 0.1 |
| Aloe Vera Gel Concentrate 10/1 * | Water (Aqua), Aloe Barbadensis Leaf Juice | 3.0 |
| Azelaic Acid | Azelaic Acid | 5.0 |
| Cetiol OE | Dicaprylyl Ether | 4.0 |
| Cetiol SB 45 | Butyrospermum Parkii (Shea Butter) | 1.0 |
| D-Panthenol | Panthenol | 1.0 |
| SymClariol | Decylene Glycol | 0.1 |
| Emulsiphos^{®} | Potassium Cetyl Phosphate, Hydrogenated Palm Glycerides | 2.0 |
| Perfume oil PO1, PO2, PO3, PO4, or PO5 | Fragrance | 0.2 |
| Frescolat^{®}ML cryst, | Menthyl Lactate | 0.8 |
| Glycerol 85 % | Glycerin | 4.0 |
| Hydroviton^{®} PLUS | Water, Pentylene Glycol, Glycerin, Fructose, Urea, Citric Acid, Sodium Hydroxide, Maltose, Sodium PCA, Sodium Chloride, Sodium Lactate, Trehalose, Allantoin, Sodium hyaluronate, Glucose | 1.0 |
| Lara Care A-200 | Galactoarabinan | 0.3 |
| Pemulen TR-2 | Acrylates/C10-30 Alkyl Acrylate Crosspolymer | 0.2 |
| Sodium Hydroxide (10% solution) | Sodium Hydroxide | 0.4 |
| SymTriol | Caprylyl glycol, 1,2-Hexanediol, Methylbenzyl alcohol | 1.0 |
| Tegosoft TN | C12-15 Alkyl Benzoate | 5.0 |
| Tocopherol Acetate | Tocopheryl Acetate | 0.5 |
| Water (demineralized) | Water (Aqua) | ad 100 |

**Table 22: Barrier repair cream**

| **Ingredients** | **INCI** | **Amount** |
|---|---|---|
| Avenanthramide L | Avenanthramide L | 0.001 |
| Avenanthramide B | Avenanthramide B | 0.003 |
| SymSave H | Hydroxyacetophenone | 0.6 |
| Abil 350 | Dimethicone | 0.5 |
| Allantoin | Allantoin | 0.25 |
| Ceramide BIO* | Cetylhydroxyproline Palmitamide | 0.5 |
| Dracorin^{®} CE | Glyceryl Stearate Citrate | 1.5 |
| Dragoxat^{®} 89 | Ethylhexyl Ethylisononan-oate | 2.0 |
| Emulsiphos^{®} | Potassium Cetyl Phosphate, Hydrogenated Palm Glycerides | 2.0 |
| Extrapone^{®} Rosemary GW | Glycerin, Water (Aqua), Rosmarinus officinalis (Rosemary) Leaf Extract | 0.5 |
| Perfume oil PO1, PO2, PO3, PO4, or PO5 | Fragrance | 0.1 |
| Glycerol 85 % | Glycerin | 3.0 |
| Glyceryl Stearate | Glyceryl Stearate | 2.0 |
| Hydroviton^{®} 24 | Water, Glycerin, Sodium Lactate, TEA Lactate, Serine, Lactic Acid, Urea, Sorbitol, Sodium Chloride, Lauryl Diethylenediaminoglycine, Lauryl Aminopropyl-glycine, Allantoin | 1.0 |
| Hydrolite- 5 Green | Pentylene Glycol | 1.0 |
| Isodragol^{®} | Triisononanoin | 3.0 |
| Lanette O | Cetearyl Alcohol | 2.0 |
| NaOH (10% solution) | Sodium Hydroxide | 0.3 |
| Neutral Oil | Caprylic/Capric Triglyceride | 10.0 |
| SymCalmin^{®} | Pentylene Glycol, Butylene Glycol, Hydroxyphenyl Propamidobenzoic Acid | 1.0 |
| SymRepair^{®} 100 | Hexyldecanol, Bisabolol, Cetylhydroxyproline Palmitamide, Stearic Acid, Brassica Campestris (Rapeseed) Sterols | 2.0 |
| SymTriol | Caprylyl glycol, 1,2-Hexanediol, Methylbenzyl alcohol | 1.0 |
| Tegosoft PC 31 | Polyglyceryl 3- Caprate | 0.3 |
| Tocopherol Acetate | Tocopheryl Acetate | 0.3 |
| Water (demineralized) | Water (Aqua) | ad 100 |

**Table 23: Skin soothing lotion**

| **Ingredients** | **INCI** | **Amount** |
|---|---|---|
| Avenanthramide L | Avenanthramide L | 0.001 |
| SymSave H | Hydroxyacetophenone | 0.3 |
| Avenanthramide A | Avenanthramide A | 0.01 |
| Abil 350 | Dimethicone | 2.0 |
| Allantoin | Allantoin | 0.2 |
| Carbopol Ultrez-10 | Carbomer | 0.1 |
| Ceramide BIO* | Cetylhydroxyproline Palmitamide | 0.1 |
| Citric Acid (10% solution) | Citric Acid | 0.4 |
| Emulsiphos^{®} | Potassium Cetyl Phosphate, Hydrogenated Palm Glycerides | 2.0 |
| Extrapone^{®} Green Tea GW | Glycerin, Water (Aqua), Camellia Sinensis Leaf Extract | 0.2 |
| Extrapone^{®} Rosemary GW | Glycerin, Water (Aqua), Rosmarinus officinalis (Rosemary) Leaf Extract | 0.3 |
| Perfume oil PO1, PO2, PO3, PO4, or PO5 | Fragrance | 0.3 |
| Glycerol 85 % | Glycerin | 2.0 |
| Glyceryl Stearate | Glyceryl Stearate | 2.0 |
| Isodragol^{®} | Triisononanoin | 2.0 |
| Keltrol RD | Xanthan Gum | 0.1 |
| Lanette O | Cetearyl Alcohol | 3.0 |
| Neo PCL wssl, N | Trideceth-9, PEG-5 Ethylhexanoate, Water | 1.0 |
| PCL Liquid 100 | Cetearyl Ethylhexanoate | 5.0 |
| PCL Solid | Stearyl Heptanoate, Stearyl Caprylate | 2.0 |
| Propylene Glycol | Propylene Glycol | 5.0 |
| Sodium Hydroxide (10% solution.) | Sodium Hydroxide | 0.3 |
| SymCalmin^{®} | Pentylene Glycol, Butylene Glycol, Hydroxyphenyl Propamidobenzoic Acid | 2.0 |
| SymMatrix^{®} | Maltodextrin, Rubus Fruticosus (Blackberry) Leaf Extract | 0.1 |
| 2-Phenoxyethyl Alcohol | Phenoxyethanol | 0.4 |
| SymSitive^{®}1609 | Pentylene Glycol, 4-t-Butylcyclohexanol | 1.5 |
| Water (demineralized) | Water (Aqua) | ad 100 |

**Table 24: Baby nappy rash cream, w/o**

| **Ingredients** | **Amount** |
|---|---|
| **SymOcide PH** | 1 |
| Phenoxyethanol, Hydroxyacetophenone, Caprylyl Glycol, Water (Aqua) | |
| **Cupuaçu butter** | 1 |
| Theobroma Grandiflorum Seed Butter | |
| **Cutina HR Powder** | 1.5 |
| Hydrogenated Castor Oil | |
| **Dehymuls PGPH** | 5 |
| Polyglyceryl-2 Dipolyhydroxystearate | |
| **Glycerin** | 5 |
| Glycerin | |
| **Jojoba oil** | 5 |
| Simmondsia Chinensis (Jojoba) Seed Oil | |
| **Magnesium Sulfate Hepta Hydrate** | 0.5 |
| Magnesium Sulfate | |
| **Monomuls 90-018** | 1 |
| Glyceryl Oleate | |
| **Neutral oil** | 8 |
| Caprylic/capric triglyceride | |
| **PCL Liquid 100** | 5 |
| Cetearyl Ethylhexanoate | |
| **SymCalmin** | 1 |
| Butylene Glycol, Pentylene Glycol, Hydroxyphenyl Propamidobenzoic Acid | |
| **Tamanu oil** | 0.2 |
| Calophyllum Inophyllum Seed Oil | |
| **Tetrasodium EDTA** | 0.1 |
| Tetrasodium EDTA | |
| **Titan dioxide** | 4 |
| Titan dioxide | |
| **Water** | ad 100 |
| Aqua | |
| **Wheat germ oil** | 2 |
| Triticum Vulgare (Wheat) Germ Oil | |
| **Zinc oxide** | 10 |
| Zinc oxide | |
| SymSave H (Hydroxyacetophenone) | 0.5 |
| Avenanthramide L | 0.0003 |
| Avenanthramide A | 0.002 |

**Table 25: Skin lightening day cream, o/w**

| **Ingredients** | **INCI** | **Amount** |
|---|---|---|
| Avenanthramide L | Avenanthramide L | 0.001 |
| Avenanthramide B | Avenanthramide B | 0.002 |
| Avenanthramide A | Avenanthramide A | 0.002 |
| SymSave H | Hydroxyacetophenone | 0.5 |
| Abil 350 | Dimethicone | 0.5 |
| Dracorin^{®} CE | Glyceryl Stearate Citrate | 2.5 |
| Dracorin^{®} GOC | Glyceryl Oleate Citrate, Caprylic/Capric Triglyceride | 0.5 |
| Drago-Beta-Glucan | Water (Aqua), Butylene Glycol, Glycerin, Avena Sativa (Oat), Kernel Extract | 0.3 |
| Dragosantol^{®} 100* | Bisabolol | 0.2 |
| Perfume oil PO1, PO2, PO3, PO4, or PO5 | Fragrance | 0.1 |
| Frescolat^{®}MGA | Menthone Glycerol Acetal | 0.5 |
| Glycerol 85 % | Glycerin | 3.0 |
| Isopropyl Palmitate | Isopropyl Palmitate | 4.0 |
| Keltrol RD | Xanthan Gum | 0.2 |
| Lanette 16 | Cetyl Alcohol | 1.0 |
| Neo Heliopan^{®} AV | Ethylhexyl Methoxy-cinnamate | 5.0 |
| Neutral Oil | Caprylic/Capric Triglyceride | 6.0 |
| PCL Liquid 100 | Cetearyl Ethylhexoate | 3.0 |
| Sodium Benzoate | Sodium Benzoate | 0.1 |
| Symdiol^{®}68T | 1,2-Hexanediol, Caprylylglycol, Tropolone | 0.5 |
| SymVital^{®} AgeRepair | Zingiber Officinale (Ginger) Root Extract | 0.1 |
| SymWhite^{®}377 | Phenylethyl Resorcinol | 0.5 |
| Water (demineralized) | Water (Aqua) | ad 100 |

**Table 26: Shampoo**

| **Ingredients** | **Amount** |
|---|---|
| **Antil 127** | 0.5 |
| PEG-120 Methyl Glucose Dioleate | |
| **Brazilian nut oil** | 0.5 |
| Bertholletia Excelsa Seed Oil | |
| **Cocamidopropyl Betaine 38%** | 5 |
| Cocamidopropyl Betaine | |
| **Octopirox** | 0.3 |
| Piroctone olamine | |
| **Dragoderm** | 0.5 |
| Glycerin. Triticum Vulgare Gluten. Aqua | |
| **Fragrance** | 0.5 |
| Perfum | |
| **Glycerin** | 0.5 |
| Glycerin | |
| **Jojoba oil** | 0.5 |
| Simmondsia Chinensis (Jojoba) Seed Oil | |
| **Marlinat 242/90 M** | 15 |
| MIPA Laureth Sulfate. Propylene Glycol | |
| **Marlowet CG** | 2 |
| PEG-18 Castor Oil Dioleate | |
| **Plantacare 1200 UP** | 0.5 |
| Lauryl Glucoside | |
| **Polyquaternium-10** | 0.3 |
| Polyquaternium-10 | |
| **Sodium Chloride** | 1.5 |
| Sodium Chloride | |
| **SymCalmin** | 1 |
| Butylene Glycol. Pentylene Glycol. Hydroxyphenyl Propamidobenzoic Acid | |
| **SymOcide PS** | 0.8 |
| Phenoxyethanol. Decylene Glycol.1.2-Hexanediol | |
| Avenanthramide L | 0.0001 |
| Avenanthramide A | 0.002 |
| **SymSave H (Hydroxyacetophenone)** | 0.5 |
| **Water** | ad 100 |
| Aqua | |

**Table 27: Anti dandruff shampoo**

| **Ingredients** | **INCI** | **Amount** |
|---|---|---|
| Avenanthramide L | Avenanthramide L | 0.001 |
| Avenanthramide A | Avenanthramide A | 0.15 |
| Avenanthramide B | Avenanthramide B | 0.05 |
| SymSave H | Hdroxyacetophenone | 1.0 |
| Aloe Vera Gel Concentrate 10/1 * | Water (Aqua), Aloe Barbadensis Leaf Juice | 0.5 |
| Abrasive/Exfoliant | Perlite | 0.3 |
| Cellulose fibre | Microcrystalline Cellulose | 0.1 |
| Avocado oil | Persea Gratissima (Avocado) Oil | 0.5 |
| Citric Acid 10% sol. | Citric Acid | 0.3 |
| Comperlan 100 | Cocamide MEA | 0.5 |
| Crinipan AD | Climbazole | 0.2 |
| Dragoderm^{®} | Glycerin, Triticum Vulgare (Wheat) Gluten, Water (Aqua) | 2.0 |
| Perfume oil PO1, PO2, PO3, PO4, or PO5 | Fragrance | 0.5 |
| Genapol LRO liquid | Sodium Laureth Sulfate | 37.0 |
| Merquat 550 | Polyquaternium-7 | 0.5 |
| Xylityl Caprylate | Xylityl Caprylate | 0.5 |
| Sodium Chloride | Sodium Chloride | 1.0 |
| Hydrolite-5 Green | Pentylene Glycol | 0.5 |
| Tego Betain L7 | Cocamidopropyl Betaine | 6.0 |
| Water (demineralized) | Water (Aqua) | ad 100 |

**Table 28: 2-in-1 Shampoo**

| **Ingredients** | **INCI Name** | **Amount** |
|---|---|---|
| Deionized water | Water | ad 100 |
| Shea butter | Butyrospermum Parkii (Shea) Butter | 0.1 |
| SymSave H | Hydroxyacetophenone | 0.5 |
| SymDiol 68 | 1.2 Hexanediol, Caprylyl Glycol | 0.5 |
| Plantacare PS 10 | Sodium Laureth Sulfate, Lauryl Glucoside | 20.0 |
| Euperlan PK 771 | Glycol Distearate, Sodium Lauryl Sulfate, Cocamide MEA, Laureth-10 | 6.0 |
| Sodium chloride | Sodium Chloride | 1.4 |
| Citric acid monohydrate crystalline | Citric acid | 0.1 |
| Perfume oil PO1, PO2, PO3, PO4, or PO5 | Fragrance | 0.5 |
| Zinc Omadine | Zinc pyrithione | 0.10 |
| Avenanthramide L | Avenanthramide L | 0.001 |
| Avenanthramide B | Avenanthramide B | 0.002 |

**Table 29: Body wash**

| **Ingredients** | **INCI** | **Amount** |
|---|---|---|
| Lumerol K 28 | Disodium Laureth Sulfosuccinate, Cocamidopropyl Betaine, Magnesium Lauryl Sulfate | 33.0 |
| Amphotensid B 4 | Cocamidopropyl Betaine | 10.0 |
| Pearly Gloss | MIPA-Pareth-25 Sulfate, Glycol Stearate | 4.0 |
| Sodium Chloride | Sodium Chloride | 2.0 |
| Avocado oil | Persea Gratissima (Avocado) Oil | 3.0 |
| SymSave H | Hydroxyacetophenone | 0.8 |
| Hydrolite-5 Green | Pentylene Glycol | 1.0 |
| Water | Water | ad 100 |
| Perfume oil PO1, PO2, PO3, PO4, or PO5 | Fragrance | 0.5 |
| Glyceryl monocaprylate | Glyceryl caprylate | 0.15 |
| Avenanthramide A | Avenanthramide A | 0.1 |
| Avenanthramide L | Avenanthramide L | 0.15 |

**Table 30: Shower gel**

| **Ingredients** | **INCI** | **Amount** |
|---|---|---|
| Deionized water | Water | ad 100 |
| Shea butter | Butyrospermum Parkii (Shea) Butter | 1.0 |
| Plantacare PS 10 | Sodium Laureth Sulfate, Lauryl Glucoside | 20.0 |
| Hydrolite-6 | 1.2 Hexanediol | 0.5 |
| Dehydroacetic acid | Dehydroacetic acid | 0.2 |
| SymSave H | Hydroxyacetophenone | 0.3 |
| Sodium chloride | Sodium Chloride | 1.4 |
| Citric acid monohydrate crystalline | Citric Acid | 1.3 |
| Perfume oil PO1, PO2, | Fragrance | 0.6 |
| PO3, PO4, or PO5 | | |
| Avenanthramide L | Avenanthramide L | 0.1 |
| Symlite G 8 | Glyceryl caprylate | 0.3 |
| Avenanthramide A | Avenanthramide A | 0.2 |

**Table 31: Intimate wash**

| **Ingredients** | **INCI** | **Amount** |
|---|---|---|
| Tegobetaine HS | Cocamidopropyl Betaine, Glyceryl Laurate | 15.0 |
| Tagat L 2 | PEG-20 Glyceryl Laurate | 2.0 |
| Arlacide G | Chlorhexidine Digluconate | 0.1 |
| Rewoquat B 50 | Benzalkonium Chloride | 0.1 |
| Lactic Acid. 80% | Lactic Acid | 0.1 |
| euxyl^{®} K700 | Potassium Sorbate, Benzyl Alcohol. Phenoxyethanol | 0.3 |
| Water | Water | ad 100 |
| Perfume oil PO1, PO2, PO3, PO4, or PO5 | Fragrance | 0.2 |
| Hydrolite-5 Green | Pentylene Glycol | 0.3 |
| SymSave H | Hydroxyacetophenone | 0.3 |
| Avenanthramide L | Avenanthramide L | 0.001 |
| Avenanthramide A | Avenanthramide A | 0.002 |

**Table 32: Liquid soap, transparent**

| **Ingredients** | **INCI** | **Amount** |
|---|---|---|
| Tagat O 2 | PEG-20 Glyceryl Oleate | 2.5 |
| Coconut oil diethanolamine | Cocamide DEA | 5.0 |
| condensate | | |
| Abil B 8842 | Cyclomethicone | 0.5 |
| Sodium laurylethersulfate. 28% | Sodium Laureth Sulfate | 35.0 |
| Tego-Betaine L7 | Cocamidopropyl Betaine | 5.0 |
| SymSave H | Hydroxyacetophenone | 0.5 |
| Soap. 25% | Coconut acid, Potassium salt, Potassium Oleate | 20.0 |
| Perfume oil PO1, PO2, PO3, PO4, or PO5 | Fragrance | 0.4 |
| SymSave H | Hydroxyacetophenone | 0.5 |
| Avenanthramide L | Hydroxypropyl caprylate | 0.15 |
| Water | Water | ad 100 |

**Table 33: Syndet soap, liquid**

| **Ingredients** | **INCI** | **Amount** |
|---|---|---|
| Elfan OS 46 | Sodium Olefin C14-C16 Sulfonate | 35.5 |
| Armoteric LB | Lauryl Betaine | 8.0 |
| Euperlan PK 3000 OK | Glycol Distearate, Glycerin, Laureth-4, Cocamidopropyl Betaine | 10.0 |
| Elfacos GT 282 L | Talloweth-60 Myristyl Glycol | 3.0 |
| PCL-Liquid 100 | Cetearyl Ethylhexanoate | 4.0 |
| Perfume oil PO1, PO2, PO3, PO4, or PO5 | Fragrance | 0.4 |
| SymSave H | 4-Hydroxyacetophenone | 0.6 |
| Avenanthramide L | Avenanthramide L | 0.05 |
| Avenanthramide A | Avenanthramide A | 0.5 |
| Water | Water | ad 100 |

**Table 34: Anti-acne wash**

| **Ingredients** | **Amount** |
|---|---|
| Water (Aqua) | ad 100 |
| Polyquaternium-7 | 0.5 |
| Cocamidopropyl Betaine | 9.0 |
| Coco Glucoside | 2.0 |
| Polysorbate 80, Glycerol. Gossypium Herbaceum (Cotton) Seed Oil, Water (Aqua) | 1.0 |
| Trideceth-9, PEG-5 Ethylhexanoate, Water (Aqua) | 1.0 |
| Glycereth-90 Isostearate, Laureth-2 | 0.5 |
| Sodium Laureth Sulfate | 37.0 |
| Glycerol. Triticum Vulgare (Wheat) Gluten. Water (Aqua) | 1.0 |
| Sodium Chloride | 0.3 |
| Perfume oil PO1, PO2, PO3, PO4, or PO5 | 1.0 |
| SymOcide BHO (Hydroxyacetophenone, Benzyl alcohol, Caprylyl glycol, Water) | 0.3 |
| SymSave H (Hydroxyacetophenone) | 0.8 |
| Avenanthramide L | 0.25 |
| Avenanthramide A | 0.15 |

**Table 35: Mineral wash and cleaning gel**

| **Ingredients** | **INCI** | **Amount** |
|---|---|---|
| Water | Water (Aqua) | ad 100 |
| Pionier^{®} NP 37 G | Sodium Carbomer | 1.5 |
| SymSol^{®} PF-3 | Water (Aqua), Pentylene Glycol, Sodium Lauryl Sulfoacetate, Sodium Oleoyl Sarcosinate, Sodium Chloride, Disodium | 5.0 |
| | Sulfoacetate, Sodium Oleate, Sodium Sulfate | |
| Hydroviton^{®} 24 | Water (Aqua), Pentylene Glycol. Glycerol, Sodium Lactate, Lactic Acid, Serine, Urea, Sorbitol, Sodium Chloride, Allantoin | 1.0 |
| Extrapone^{®} Silk GW | Water (Aqua), Glycerol, Hydrolyzed Silk | 1.0 |
| Hydrolite^{®} 5 | Pentylene Glycol | 4.0 |
| Actipearls Red Star # DH10402/6 | Water (Aqua), Propylene Glycol, Algin, Gellan Gum, Xanthan Gum, Calcium Chloride, CI 12490 (Pigment Red 5), Mica (CI 77019), Titanium Dioxide (Cl 77891) | 1.0 |
| Perfume oil PO1, PO2, PO3, PO4, or PO5 | Fragrance | 0.5 |
| SymGuard CD | Phenylpropanol, o-cymen-5-ol, Decylene glycol | 0.3 |
| SymSave H | Hydroxyacetophenone | 0.5 |
| Avenanthramide L | Avenanthramide L | 0.001 |
| Avenanthramide A | Avenanthramide A | 0.03 |

**Table 36: After Shave Tonic**

| **Ingredients** | **INCI** | **Amount** |
|---|---|---|
| SymSol^{®} PF-3 | Water (Aqua). Pentylene Glycol. Sodium Lauryl Sulfoacetate, Sodium Oleoyl Sarcosinate, Sodium Chloride, Disodium Sulfoacetate, SodiumOleate, Sodium | 3.0 |
| | Sulfate | |
| SymSitive^{®} 1609 | Pentylene Glycol, 4-t-Butylcyclohexanol | 1.0 |
| Frescolat^{®} ML | Menthyl Lactate | 0.3 |
| Glycerol 99.5 P. | Glycerol | 5.0 |
| Water | Water (Aqua) | ad 100 |
| Extrapone^{®} Glacier Water GW | Glycerol, Water (Aqua) | 1.0 |
| SymCalmin^{®} | Butylene Glycol, Pentylene Glycol, Hydroxyphenyl Propamidobenzoic Acid | 0.5 |
| Dragosine^{®} | Carnosine | 0.1 |
| Hydrolite^{®} 5 | Pentylene Glycol | 5.0 |
| Ethanol 96 % | Alcohol Denat. | 5.0 |
| Colour Pigment | Colour Pigment | 0.05 |
| Perfume oil PO1, PO2, PO3, PO4, or PO5 | Fragrance | 0.15 |
| SymSave H | Hydroxyacetophenone | 0.8 |
| Avenanthramide L | Avenanthramide L | 0.001 |
| Avenanthramide A | Avenanthramide A | 0.002 |

**Table 37: Hair conditioner with Crinipan, rinse-off**

| **Ingredients** | **INCI** | **Amount** |
|---|---|---|
| Lanette^{®} O | Cetearyl Alcohol | 4.0 |
| Dragoxat 89 | Ethylhexyl Isononanoate | 2.0 |
| Genamin^{®} KDM-P | Behentrimonium Chloride | 1.0 |
| SymClariol | Decylene Glycol | 0.1 |
| SF 1550 | Phenyl Trimethicone | 0.1 |
| Neo Heliopan^{®} BB | Benzophenone-3 | 0.1 |
| Crinipan^{®} AD | Climbazole | 0.4 |
| Glycerol 99.5 P. | Glycerol | 6.0 |
| Water | Water (Aqua) | ad 100 |
| Actipone^{®} Alpha Pulp | Water (Aqua), Butylene Glycol, Malic Acid, Actinidia Chinensis (Kiwi) Fruit Juice, Citrus Aurantium Dulcis (Orange) Juice, Citrus Paradisi (Grapefruit) Juice, Pyrus Malus (Apple) Juice, Trideceth-9, Prunus Amygdalus Dulcis (Sweet Almond) Seed Extract | 0.5 |
| Extrapone^{®} Bamboo P | Propylene Glycol, Water (Aqua), Butylene Glycol, Bambusa Vulgaris Shoot Extract | 0.5 |
| Sodium Hydroxide (10% solution) | Sodium Hydroxide | 0.4 |
| Colour I | Colour | 0.6 |
| Colour II | Colour | 0.3 |
| Perfume oil PO1, PO2, PO3, PO4, or PO5 | Fragrance | 0.4 |
| Preservative | Methylparaben | 0.3 |
| Avenanthramide L | Avenanthramide L | 0.0005 |
| SymSave H | Hydroxyacetophenone | 0,3 |
| Avenanthramide A | Avenanthramide A | 0.001 |

**Table 38: Scalp soothing hair conditioner with UV-B/UV-A protection, rinse off**

| **Ingredients** | **INCI** | **Amount** |
|---|---|---|
| Avenanthramide L | Avenanthramide L | 0.0005 |
| SymSave H | Hydroxyacetophenone | 0.5 |
| Avenanthramide A | Avenanthramide A | 0.0025 |
| | | 0.1 |
| Abil 350 | Dimethicone | 0.1 |
| Dehyquart A CA | Cetrimonium Chloride | 0.5 |
| Dehyquart SP | Quaternium-52 | 4.0 |
| Dracorin^{®} CE | Glyceryl Stearate Citrate | 1.0 |
| EDETA BD | Disodium EDTA | 0.1 |
| Extrapone^{®} Green Tea GW | Glycerin, Water (Aqua), Camellia Sinensis Leaf Extract | 0.7 |
| Perfume oil PO1, PO2, PO3, PO4, or PO5 | Fragrance | 0.5 |
| Lara Care A-200 | Galactoarabinan | 0.5 |
| Neutral Oil | Caprylic/Capric Triglyceride | 1.0 |
| PCL Liquid 100 | Cetearyl Ethylhexoate | 0.3 |
| PCL Solid | Stearyl Heptanoate, Stearyl Caprylate | 3.0 |
| SymOcide^{®}PS | Phenoxyethanol, Decylene Glycol, 1,2-Hexanediol | 0.5 |
| Water (demineralized) | Water (Aqua) | ad 100 |

**Table 39: Hair conditioner with UV protection**

| **Ingredients** | **INCI** | **Amount** |
|---|---|---|
| Renex PEG 6000 | PEG-150 | 2.5 |
| Hair Conditioner Base | Cetyl alcohol, Behentrimonium chloride, Triticum Vulgare (Wheat) bran extract, Linoleic acid | 3.0 |
| PCL-Solid | Stearyl heptanoate, stearyl caprylate | 0.5 |
| Dow Corning 5200 | Laurylmethicone copolyol | 0.5 |
| Natrosol 250 HR | Hydroxyethylcellulose | 0.5 |
| Benzophenone-4 | Benzophenone-4 | 1.0 |
| Neo Heliopan AP | Disodiumphenyldibenzimidazole tetrasulphonate | 1.0 |
| Amino methyl propanol | Amino methyl propanol | 2.0 |
| Dow Corning 949 cationic emulsion | Amodimethicone, Cetrimonium chloride, Trideceth-12 | 2.0 |
| Perfume oil PO1, PO2, PO3, PO4, or PO5 | Fragrance | 0.8 |
| 1.2-Hexanediol | 1.2-Hexanediol | 0.5 |
| SymSave H | Hydroxyacetophenone | 0.5 |
| Avenanthramide L | Avenanthramide L | 0.002 |
| Avenanthramide A | Avenanthramide A | 0.01 |
| Water | Water (Aqua) | ad 100 |

**Table 40: Hair conditioner, leave on**

| **Ingredients** | **INCI** | **Amount** |
|---|---|---|
| Avenanthramide L | Avenanthramide L | 0.015 |
| SymSave H | Hydroxyacetophenone | 0.3 |
| Avenanthramide A | Avenanthramide A | 0.02 |
| Dehyquart A CA | Cetrimonium Chloride | 0.2 |
| Dehyquart SP | Quaternium-52 | 2.0 |
| Dracorin^{®} CE | Glyceryl Stearate Citrate | 1.0 |
| Drago-Calm | Water, Glycerin, Avena Sativa (Oat) Kernel Extract | 2.0 |
| Farnesol | Farnesol | |
| Perfume oil PO1, PO2, PO3, PO4, or PO5 | Fragrance | 0.5 |
| Lara Care A-200 | Galactoarabinan | 0.1 |
| Polymer JR 400 | Polyquaternium-10 | 0.1 |
| Propylene Glycol | Propylene Glycol | 0.8 |
| SymMollient^{®} WS | Trideceth-9. PEG-5 Isononanoate. Water | 1.0 |
| SymSol^{®}PF3 | Water, Pentylene Glycol, Sodium Lauryl Sulfoacetate, Sodium Oleoyl Sarcosinate, Sodium Chloride, Disodium Sulfoacetate, Sodium Oleate, Sodium Sulfate | 1.5 |
| SymTriol^{®} | Caprylyl Glycol, 1.2-Hexanediol, Methylbenzyl Alcohol | 0,6 |
| Water (demineralized) | Water (Aqua) | ad 100 |

**Table 41: Anti-itch hair conditioner, leave on**

| **Ingredients** | **INCI** | **Amount** |
|---|---|---|
| Avenanthramide L | Avenanthramide L | 0.5 |
| SymSave H | Hydroxyacetophenone | 1.0 |
| Avenanthramide A | Avenanthramide A | 0.1 |
| (-)-alpha Bisabolol | Bisabolol | 0.1 |
| Dehyquart A CA | Cetrimonium Chloride | 0.5 |
| Dehyquart SP | Quaternium-52 | 4.0 |
| Dracorin^{®} CE* | Glyceryl Stearate Citrate | 1.0 |
| Drago-Oat-Active* | Water (Aqua), Butylene Glycol, Avena Sativa (Oat) Kernel Extract | 2.0 |
| Perfume oil PO1, PO2, PO3, PO4, or PO5 | Fragrance | 0.1 |
| Lara Care A-200 | Galactoarabinan | 1.5 |
| Neutral Oil | Caprylic/Capric Triglyceride | 1.0 |
| PCL Liquid 100* | Cetearyl Ethylhexoate | 0.3 |
| Polymer JR 400 | Polyquaternium-10 | 0.1 |
| Propylene Glycol | Propylene Glycol | 0.8 |
| SymGlucan^{®} | Aqua, Glycerin, 1,2-Hexandiol, Caprylyl Glycol, Beta-Glucan | 5 |
| SymMollient^{®} W/S | Trideceth-9, PEG-5 Isononanoate, Water (Aqua) | 2.0 |
| SymSol^{®}PF3 * | Water, Pentylene Glycol, Sodium Lauryl Sulfoacetate, Sodium Oleoyl Sarcosinate, Sodium Chloride, Disodium Sulfoacetate, Sodium Oleate, Sodium Sulfate | 1.5 |
| Water, demineralized | Water (Aqua) | ad 100 |

**Table 42: Sprayable hair conditioner with zinc pyrithrione, leave-on**

| **Ingredients** | **INCI** | **Amount** |
|---|---|---|
| Monomuls 60-35 C | Hydrogenated Palm Glycerides | 1.7 |
| Cetiol OE | Dicaprylyl Ether | 7.2 |
| Abil 100 | Dimethicone | 3.6 |
| Dehyquart F 75 | Distearoylethyl Hydroxyethylmonium Methosulfate, Cetearyl Alcohol | 4.0 |
| Eumulgin B1 | Ceteareth-12 | 3.5 |
| Cetiol S | Diethylhexylcyclohe xane | 7.2 |
| D-Panthenol | Panthenol | 0.1 |
| Glycerol 99.5 P. | Glycerol | 1.5 |
| Water | Water (Aqua) | ad 100 |
| Actipone^{®} Rosemary | Water (Aqua), PropyleneGlycol, Rosmarinus Officinalis (Rosemary) Leaf Extract | 0.1 |
| Frescolat^{®} ML Cryst. | Menthyl Lactate | 0.5 |
| Dragosantol100 | Bisabolol | 0.1 |
| Zinc Omadine | Zinc pyrithione | 0.1 |
| Perfume oil PO1, PO2, PO3, PO4, or PO5 | Fragrance | 0.4 |
| 2-Phenoxyethyl alcohol | Phenoxyethanol | 0.4 |
| SymSave H | Hydroxyacetophenone | 0.3 |
| SymDiol 68 | 1,2-Hexanediol, Caprylyl glycol | 0.3 |
| Avenanthramide L | Avenanthramide L | 0.001 |
| Avenanthramide A | Avenanthramide A | 0.001 |

**Table 43: Hair styling gel**

| **Ingredients** | **Amount** |
|---|---|
| Water | ad 100 |
| PVM/MA Decadiene Crosspolymer | 0.6 |
| PVP | 3.0 |
| Isocetyl Stearate | 4.0 |
| Ethylhexyl Methoxycinnamate | 0.5 |
| Hydrolite-5 Green (Pentylene Glycol) | 0.5 |
| Aminomethyl Propanol | 0.4 |
| Perfume oil PO1, PO2, PO3, PO4, or PO5 | 0.6 |
| SymDiol^{®} 68T (1,2-Hexanediol, 1,2-Octanediol, Tropolone) | 0.4 |
| Phenoxyethanol | 0.3 |
| Avenanthramide L | 0.0005 |
| Avenanthramide A | 0.00025 |

**Table 44: Deodorant stick**

| **Ingredients** | **Amount** |
|---|---|
| Sodium stearate | 8.0 |
| PPG-3 Myristyl ether | 70.0 |
| 1.2-propylene glycol | 10.0 |
| 1.1-dimethyl-3-phenylpropanol | 0.2 |
| 2-butyloctanoic acid | 0.2 |
| Perfume oil PO1, PO2, PO3, PO4, or PO5 | 0.6 |
| Water | ad 100 |
| SymDeo Plus (Jasmol (2-benzlheptanol), 1-Dodecanol (Lauryl Alcohol), 1,2-Decanediol (Decylene Glycol), 2-Phenoxyethyl Alcohol (Phenoxyethanol)) | 0.5 |
| Avenanthramide L | 0.001 |
| SymSave H (Hydroxyacetophenone) | 0.4 |
| Avenanthramide A | 0.001 |

**Table 45: Zirconium suspensoid antiperspirant stick**

| **Ingredients** | **INCI** | **Amount** |
|---|---|---|
| PCL Liquid 100 | Cetearyl ethylhexanonate | ad 100 |
| Silicone Fluid 345 | Cyclomethicone | 10.0 |
| CRODACOL C90 | Cetyl Alcohol | 8.0 |
| SYNCROWAX HGLC | C18-36 Triglyceride | 8.0 |
| CRODAMOL PTC | Pentaerythritol T etracaprylate/Caprate | 5.0 |
| SymDeo MPP | Dimethyl Phenylbutanol | 0.3 |
| SYNCROWAX HRC | Tribehenin | 4.0 |
| VOLPO N5 | Oleth-5 | 1.0 |
| Titanium Dioxide | | 1.0 |
| Rezal 36GP | Aluminium Tetrachlorohydrex GLY | 20.0 |
| Dry Flo C | Aluminium Starch Octenyl Succinate | 22.5 |
| Symlite G8 | Glyceryl caprylate | 0.3 |
| Perfume oil PO1, PO2, PO3, PO4, or PO5 | Fragrance | 0.6 |
| Avenanthramide L | Avenanthramide L | 0.0005 |
| SymSave H | Hydroxyacetophenone | 0.5 |
| Avenanthramide A | Avenanthramide A | 0.002 |

**Table 46: Antiperspirant/deodorant roll-on**

| **Ingredients** | **INCI** | **Amount** |
|---|---|---|
| Avenanthramide L | Avenanthramide L | 0.001 |
| SymSave H | Hydroxyacetophenone | 0.5 |
| Avenanthramide B | Avenanthramide A | 0.004 |
| Dragosantol^{®} 100* | Bisabolol | 0.1 |
| Ethanol 96 % | Ethanol | 30.0 |
| Farnesol | Farnesol | 0.5 |
| Perfume oil PO1, PO2, PO3, PO4, or PO5 | Fragrance | 1.5 |
| Frescolat^{®}ML cryst, | Menthyl Lactate | 0.2 |
| Irgasan DP 300 | Triclosan | 0.3 |
| Natrosol 250 HHR | Hydroxyethyl-cellulose | 0.3 |
| Solubilizer 611674 | PEG-40 Hydrogenated Castor Oil, Trideceth-9, Water (Aqua) | 2.0 |
| SymDeo^{®} B125 | 2-Methyl 5-Cyclohexylpentanol | 0.5 |
| Water (demineralized) | Water (Aqua) | ad 100 |
| Zirkonal L 450 | Aluminium Zirconium Pentachloro-hydrate (40 % aqueous solution) | 37.0 |

**Table 47: Deodorant formulation in the form of a roll-on gel**

| **Ingredients** | **Amount** |
|---|---|
| 1.3-butylene glycol | 2.0 |
| 2-Methyl 5-cyclohexylpentanol | 0.1 |
| PEG-40-hydrogenated castor oil | 2.0 |
| Hydroxyethylcellulose | 0.5 |
| Pentylene Glycol | 1.0 |
| Perfume oil PO1, PO2, PO3, PO4, or PO5 | 0.3 |
| 1,3-propanediol | 0.5 |
| SymGuard CD (3-Phenylpropanol, o-cymen-3-ol, Decylene glycol) | 0.4 |
| Ethylhexyl glycerin | 0.1 |
| SymSave H (Hydroxyacetophenone) | 0.5 |
| Avenanthramide L | 0.001 |
| Avenanthramide B | 0.004 |
| Water | ad 100 |

**Table 48: Clear deo anti-perspirant roll-on**

| **Ingredients** | **INCI** | **Amount** |
|---|---|---|
| Methocel E4M Premium | Hydroxypropyl Methylcellulose | 0.5 |
| Water | Water (Aqua) | ad 100 |
| Neo-PCL Water Soluble N | Trideceth-9, PEG-5 Ethylhexanoate, Water (Aqua) | 1.0 |
| Solubilizer | PEG-40 Hydrogenated Castor | 3.0 |
| | Oil, Trideceth-9, Propylene Glycol, Water (Aqua) | |
| Deolite | Dimethyl Phenylpropanol, Pentylene Glycol | 0.5 |
| Locron LW | Aluminium Chlorohydrate | 25.0 |
| Aloe Vera Gel Concentrate 10/1 | Aloe Barbadensis Leaf Juice | 1.0 |
| 1.2-Propylene Glycol 99 P GC | Propylene Glycol | 4.0 |
| Ethanol 96 % | Alcohol Denat. | 30.0 |
| Perfume oil PO1, PO2, PO3, PO4, or PO5 | Fragrance | 1.0 |
| SymSave H | Hydroxyacetophenone | 0.5 |
| Avenanthramide A | Avenanthramide A | 0.005 |
| Avenanthramide L | Avenanthramide L | 0.001 |
| Avenanthramide C | Avenanthramide C | 0.005 |

**Table 49: Deodorant pump spray with SymClariol**

| **Ingredients** | **INCI** | **Amount** |
|---|---|---|
| SymClariol^{®} | Decylene Glycol | 0.2 |
| Solubilizer | PEG-40 Hydrogenated Castor Oil, Trideceth-9, Propylene Glycol, Water (Aqua) | 4.0 |
| Neo-PCL Water Soluble N | Trideceth-9, PEG-5 Ethylhexanoate, Aqua | 1.5 |
| SymRelief^{®} | Bisabolol, Zingiber Officinale (Ginger) Root Extract | 0.1 |
| Water | Water (Aqua) | ad 100 |
| 1,2-Propylene Glycol | Propylene Glycol | 6.0 |
| Perfume oil PO1, PO2, | Perfume | 0.4 |
| PO3, PO4, or PO5 | | |
| SymDiol^{®} 68 | 1,2-Hexanediol, Caprylyl Glycol | 0.2 |
| Avenanthramide A | Avenanthramide A | 0.15 |
| Avenanthramide L | Avenanthramide L | 0.2 |
| Avenanthramide D | Avenanthramide D | 0.1 |

**Table 50: Whitening deodorant spray**

| **Ingredients** | **Amount** |
|---|---|
| PEG-40-hydrogenated castor oil | 3.0 |
| Ethylhexylglycerol (Octoxyglycerol) | 0.2 |
| Symbright 2036 (Sclareolide) | 0.1 |
| Ethanol | 40.0 |
| Citrate buffer | 0.5 |
| 1.2-Hexanediol, 1.2-Octanediol (SymDiol 68) | 0.3 |
| SymOcide C (o-cymen-5-ol) | 0.05 |
| 2-Benzylheptan-1-ol (Jasmol) | 0.1 |
| Perfume oil PO1, PO2, PO3, PO4, or PO5 | 0.75 |
| Phenoxyethanol | 0.4 |
| Avenanthramide L | 0.001 |
| SymSave H (Hydroxyacetophenone) | 0.5 |
| Water | ad 100 |

**Table 51: Deodorant Aoerosol Spray**

| **Ingredients** | **Amount** |
|---|---|
| Perfume oil PO1, PO2, PO3, PO4, or PO5 | 0.75 |
| Avenanthramide A | 0.002 |
| SymSave H (Hydroxyacetophenone) | 0.2 |
| Disiloxane | Ad 100 |
| Isoadipate | 5.0 |
| C12-C15 Alkyl Benzoate | 10.0 |
| Tocopheryl Acetate | 0.5 |
| Farnesol | 0.3 |
| 40 % bulk, charged with 60 % Propane/Butane | |

**Table 52: Sunscreen lotion (o/w, broadband protection)**

| **Ingredients** | **INCI** | **Amount** |
|---|---|---|
| Avenanthramide A | Avenanthramide A | 0.005 |
| SymSave H | Hydroxyacetophenone | 0.5 |
| Avenanthramide B | Avenanthramide B | 0.005 |
| Carbopol Ultrez-10 | Carbomer | 0.2 |
| Dow Corning 246 Fluid | Cyclohexasiloxane and Cyclopentasiloxane | 2.0 |
| Dragosantol^{®} 100* | Bisabolol | 0.3 |
| EDETA BD | Disodium EDTA | 0.1 |
| Emulsiphos^{®} | Potassium Cetyl Phosphate, Hydrogenated Palm Glycerides | 1.5 |
| Perfume oil PO1, PO2, PO3, PO4, or PO5 | Fragrance | 0.4 |
| Frescolat^{®}MGA | Menthone Glycerol Acetal | 0.3 |
| Glycerol 85 % | Glycerin | 4.7 |
| Keltrol RD | Xanthan Gum | 0.2 |
| Lanette O | Cetearyl Alcohol | 1.0 |
| Neo Heliopan^{®} 357 | Butyl Methoxy-dibenzoyl-methane | 1.0 |
| Neo Heliopan^{®} AP (10 % as sodium salt) | Disodium Phenyl Dibenzimidazole Tetrasulfonate | 10.0 |
| Neo Heliopan^{®} AV | Ethylhexyl Methoxy-cinnamate | 3.0 |
| Neo Heliopan^{®} Hydro (15 % as sodium salt) | Phenylbenz-imidazole Sulfonic Acid | 6.7 |
| Neo Heliopan^{®} MBC | 4-Methylbenzyl-idene Camphor | 1.5 |
| Neo Heliopan^{®} OS | Ethylhexyl Salicylate | 5.0 |
| Neutral Oil | Caprylic/Capric Triglyceride | 2.0 |
| SymMatrix^{®} | Maltodextrin, Rubus Fruticosus (Blackberry) Leaf Extract | 0.3 |
| SymOcide^{®} BHO | Hydroxyacetophenone, Benzyl alcohol, Caprylyl glycol, Aqua | 1.5 |
| Tegosoft TN | C12-15 Alkyl Benzoate | 5.0 |
| Tocopherol Acetate | Tocopheryl Acetate | 0.5 |
| Triethanolamine, 99% | Triethanolamine | 0.5 |
| Water (demineralized) | Water (Aqua) | ad 100 |

**Table 53: Emulsion with UV-A/B-broadband protection**

| **Ingredients** | **INCI** | **Amount** |
|---|---|---|
| Avenanthramide A | Avenanthramide A | 0.005 |
| SymSave H | Hydroxyacetophenone | 0.5 |
| Avenanthramide B | Avenanthramide B | 0.005 |
| Abil 350 | Dimethicone | 0.3 |
| Butylene Glycol | Butylene Glycol | 3.0 |
| Carbopol Ultrez-10 | Carbomer | 0.2 |
| Citric Acid 10% sol. | Citric Acid | 0.3 |
| Dragosantol^{®} 100* | Bisabolol | 0.1 |
| EDETA BD | Disodium EDTA | 0.1 |
| Emulsiphos^{®} | Potassium Cetyl Phosphate, Hydrogenated Palm Glycerides | 1.5 |
| Perfume oil PO1, PO2, PO3, PO4, or PO5 | Fragrance | 0.1 |
| Frescolat^{®}X-COOL | Menthyl Ethylamido Oxalate | 1.0 |
| Glyceryl Stearate | Glyceryl Stearate | 2.0 |
| Keltrol RD | Xanthan Gum | 0.2 |
| Lanette 16 | Cetyl Alcohol | 1.2 |
| Lanette E | Sodium Cetearyl Sulfate | 0.7 |
| Neo Heliopan^{®} AP (10 % as sodium salt) | Disodium Phenyl Dibenzimidazole Tetrasulfonate | 22.0 |
| Neo Heliopan^{®} HMS | Homosalate | 5.0 |
| Neutral Oil | Caprylic/Capric Triglyceride | 2.0 |
| PCL Liquid 100 | Cetearyl Ethylhexoate | 3.0 |
| Sodium Hydroxide (10% solution) | Sodium Hydroxide | 2.8 |
| Symdiol^{®}68 | 1.2-Hexanediol, Caprylylglycol | 0.5 |
| SymMollient^{®}S | Cetearyl Nonanoate | 1.5 |
| SymSitive^{®} 1609 | Pentylene Glycol, 4-t-Butylcyclohexanol | 0.5 |
| SymWhite^{®}377 | Phenylethyl Resorcinol | 0.5 |
| Tocopherol Acetate | Tocopheryl Acetate | 0.5 |
| Water (demineralized) | Water (Aqua) | ad 100 |

**Table 54: Sun protection soft cream (w/o; SPF 40)**

| **Ingredients** | **INCI** | **Amount** |
|---|---|---|
| Dehymuls PGPH | Polyglyceryl-2 dipolyhydroxystearate | 5.0 |
| Copherol 1250 | Tocopheryl acetate | 0.5 |
| Permulgin 3220 | Ozocerite | 0.5 |
| Zinc stearate | Zinc stearate | 0.5 |
| Tegosoft TN | C12-15 Alkyl benzoate | 10.0 |
| Neo Heliopan^{®} E1000 | Isoamyl-p-methoxycinnamate | 2.0 |
| Neo Heliopan^{®} 303 | Octocrylene | 5.0 |
| Neo Heliopan^{®} MBC | 4-Methylbenzylidene camphor | 3.0 |
| Zinc oxide. neutral | Zinc oxide | 5.0 |
| Water, distilled | Water (aqua) | ad 100 |
| EDETA BD | Disodium EDTA | 0.1 |
| Glycerol | Glycerol | 4.0 |
| Magnesium sulfate | Magnesium sulfate | 0.5 |
| Perfume oil PO1, PO2, PO3, PO4, or PO5 | Fragrance | 0.3 |
| Symdiol^{®} 68 | 1,2-Hexanediol, Caprylylglycol | 0.3 |
| Avenanthramide L | Avenanthramide L | 0.001 |
| Avenanthramide A | Avenanthramide A | 0.005 |
| SymSave H | Hydroxyacetophenone | 0.5 |

**Table 55: Sun protection milk (w/o)**

| **Ingredients** | **INCI** | **Amount** |
|---|---|---|
| Dehymuls PGPH | Polyglyceryl-2 dipolyhydroxystearate | 3.0 |
| Beeswax 8100 | Beeswax | 1.0 |
| Monomuls 90-0-18 | Glyceryl oleate | 1.0 |
| Zinc stearate | Zinc stearate | 1.0 |
| Hydrolite-8 | Caprylyl Glycol | 0.3 |
| Cetiol SN | Cetearyl isononanoate | 5.0 |
| Cetiol OE | Dicaprylyl ether | 5.0 |
| Tegosoft TN | C12-15 alkyl benzoate | 4.0 |
| Vitamin E | Tocopherol | 0.5 |
| Neo Heliopan^{®} OS | Ethylhexyl salicylate | 5.0 |
| Neo Heliopan^{®} AV | Ethylhexyl methoxycinnamate | 7.5 |
| Uvinul^{®} T150 | Ethylhexyl triazone | 1.5 |
| Water. distilled | Water (Aqua) | ad 100 |
| Trilon BD | Disodium EDTA | 0.1 |
| Glycerol | Glycerol | 5.0 |
| Neo Heliopan^{®} AP 10% solution. neutralized with NaOH | Disodium phenyl dibenzimidazole tetrasulfonate | 15.0 |
| Perfume oil PO1, PO2, PO3, PO4, or PO5 | Fragrance | 0.25 |
| Alpha bisabolol | Bisabolol | 0.1 |
| SymOcide^{®} PT | Phenoxyethanol. Tropolone | 0.25 |
| SymSave H | Hydroxyacetophenone | 0.8 |
| Avenanthramide A | Avenanthramide A | 0.002 |
| Avenanthramide B | Avenanthramide B | 0.002 |

**Table 56: Sun spray with UV-A/B-broadband protection with low oil content**

| **Ingredients** | **INCI** | **Amount** |
|---|---|---|
| Avenanthramide L | Avenanthramide L | 0.001 |
| SymSave H | Hydroxyacetophenone | 0.5 |
| Avenanthramide C | Avenanthramide C | 0.002 |
| | | 0.05 |
| Ethanol 96 % | Ethanol | 13.0 |
| Perfume oil PO1, PO2, PO3, PO4, or PO5 | Fragrance | 0.5 |
| Glyceryl Stearate | Glyceryl Stearate | 4.0 |

| **Ingredients** | **INCl** | **Amount** |
|---|---|---|
| Hydroviton^{®} PLUS | Water, Pentylene Glycol, Glycerin, Fructose, Urea, Citric Acid, Sodium Hydroxide, Maltose, Sodium PCA, Sodium Chloride, Sodium Lactate, Trehalose, Allantoin, Sodium hyaluronate, Glucose | 1.0 |
| Isoadipate^{®} | Diisopropyl Adipate | 1.0 |
| Neo Heliopan^{®} AV | Ethylhexyl Methoxy-cinnamate | 25.0 |
| Neo Heliopan^{®} MBC | 4-Methylbenzyl-idene Camphor | 33.3 |
| Propylene Glycol | Propylene Glycol | 0.8 |
| Tego Betain L7 | Cocamidopropyl Betaine | 1.0 |
| Water (demineralized) | Water (Aqua) | ad 100 |

**Table 57: Sunscreen spray (o/w; SPF 15 - 20)**

| **Ingredients** | **INCI** | **Amount** |
|---|---|---|
| Dracorin^{®} GOC | Glyceryl Oleate Citrate, Caprylic/Capric Triglyceride | 2.0 |
| Corapan^{®} TQ | Diethylhexyl 2,6-Naphthalate | 3.0 |
| Neo Heliopan^{®} HMS | Homosalate | 7.0 |
| Neo Heliopan^{®} OS | Ethylhexyl Salicylate | 5.0 |
| Neo Heliopan^{®} 357 | Butyl Methoxydibenzoylmethane | 3.0 |
| Isoadipate | Diisopropyl Adipate | 6.0 |
| Baysilone^{®} Oil M10 | Dimethicone | 1.0 |
| Edeta^{®} BD | Disodium EDTA | 0.1 |
| Vitamin E Acetate | Tocopheryl Acetate | 0.5 |
| Dragosantol^{®} 100 | Bisabolol | 0.1 |
| Pemulen^{®} TR-2 | Acrylates/C10-30 Alkyl Acrylate Crosspolymer | 0.25 |
| Water | Water (Aqua) | ad 100 |
| Glycerol 99,5 P, | Glycerol | 4.0 |
| Butylene Glycol | Butylene Glycol | 5.0 |
| Neo Heliopan^{®} Hydro (103089), used as 25% aq, solution neutralized with Biotive^{®} L-Arginine | Phenylbenzimidazole Sulfonic Acid | 8.0 |
| Biotive^{®} L-Arginine | Arginine | 0.55 |
| Perfume oil PO1, PO2, PO3, PO4, or PO5 | Fragrance | 0.4 |
| SymOcide PS | Phenoxyethanol, 1,2-Hexanediol, Decylene glycol | 0.5 |
| SymSave H | Hydroxyacetophenone | 0.5 |
| Avenanthramide B | Avenanthramide B | 0.005 |
| Avenanthramide L | Avenanthramide L | 0.05 |
| Avenanthramide A | Avenanthramide A | 0.05 |

**Table 58: After sun gel**

| **Ingredients** | **INCI** | **Amount** |
|---|---|---|
| SymSol^{®} PF-3 | Water (Aqua), Pentylene Glycol, Sodium Lauryl Sulfoacetate, Sodium Oleoyl Sarcosinate, Sodium Chloride, Disodium Sulfoacetate, Sodium Oleate, Sodium Sulfate | 3.0 |
| Glycerol 99,5 P | Glycerol | 5.0 |
| SymHelios^{®} 1031 | Benzylidene Dimethoxydimethylin danone | 0.1 |
| Water | Water (Aqua) | ad 100 |
| Pemulen^{®} TR-2 | Acrylates/C10-30 Alkyl Acrylate Crosspolymer | 1.0 |
| D-Panthenol 75 W | Panthenol | 0.5 |
| SymFinity^{®} 1298 | Echinacea Purpurea Extract | 0.1 |
| Extrapone^{®} Pearl GW | Water (Aqua), Glycerol, Hydrolyzed Pearl, Xanthan Gum | 1.0 |
| Sodium Hydroxide (10% solution) | Sodium Hydroxide | 2.5 |
| Ethanol 96 % | Alcohol Denat, | 15.0 |
| Perfume oil PO1, PO2, PO3, PO4, or PO5 | Fragrance | 0.2 |
| SymOcide^{®} PS | Phenoxyethanol, 1,2-Hexanediol, Decyleneglycol | 0.8 |
| SymSave H | Hydroxyacetophenone | 0.5 |
| Avenanthramide A | Avenanthramide A | 0.05 |

**Table 59: After sun lotion**

| **Ingredients** | **Amount** |
|---|---|
| Acrylate/C10-30 alkylacrylate crosspolymer | 0.4 |
| Cetearylethyl hexanoate | 15.0 |
| Bisabolol | 0.2 |
| Tocopheryl acetate | 1.0 |
| Panthenol | 1.0 |
| Alcohol | 15.0 |
| Glycerol | 3.0 |
| Perfume oil PO1, PO2, PO3, PO4, or PO5 | 0.30 |
| 1.2-Hexanediol (Hydrolite-6) | 1.0 |
| Triethanolamine | 0.2 |
| Pentylene glycol (Hydrolite-5 Green) | 4.0 |
| Aqua dem. | ad 100 |
| 4-Hydroxyacetophenone (SymSave H) | 0.3 |
| Avenanthramide A | 0.005 |

**Table60: Syndet antimicrobial soap bar**

| **Ingredients** | **INCI** | **Amount** |
|---|---|---|
| Zetesap 813 A | Disodium Lauryl Sulfosuccinate, Sodium Lauryl Sulfate, Corn Starch, Cetearyl Alcohol, Paraffin, Titanium Dioxide | ad 100 |
| Amphotensid GB 2009 | Disodium Cocoamphodiacetate | 6.0 |
| Allantoin | Allantoin | 1.0 |
| Perfume oil PO1, PO2, PO3, PO4, or PO5 | Fragrance | 1.0 |
| SymOcide C | o-cymen-5-ol | 0.1 |
| SymSave H | Hydroxyacetophenone | 0,5 |
| Avenanthramide L | Avenanthramide L | 0.001 |
| Avenanthramide B | Avenanthramide B | 0.005 |
| Avenanthramide A | Avenanthramide A | 0.005 |

**Table61: Syndet soap bar**

| **Ingredients** | **INCI** | **Amount** |
|---|---|---|
| Fenopon AC-78 | Sodium Cocoyl Isethionate | 20.0 |
| Natrium laurylsulfoacetate | Sodium Lauryl Sulfoacetate | 16.0 |
| Paraffin | Paraffin | 19.0 |
| Wax. microcrystalline | Microcrystalline Wax | 1.0 |
| Corn Starch | Corn Starch | 8.0 |
| Coconut acid | Coconut acid | 2.0 |
| Lauric acid diethanol amide | Lauramide DEA | 2.0 |
| Dextrin | Dextrin | 21.0 |
| Lactic acid, 88% | Lactic Acid | 1.0 |
| SymGuard CD | 3-Phenylpropanol, o-cymen-5-ol, Decylene glycol | 0.3 |
| Thymol | Thymol | 0.05 |
| Symlite G8 | Glyceryl Caprylate | 0.2 |
| Water | Water | ad 100 |
| Perfume oil PO1, PO2, PO3, PO4, or PO5 | Fragrance | 1.0 |
| Avenanthramide L | Avenanthramide L | 0.001 |
| SymSave H | Hydroxyacetophenone | 0.5 |
| Avenanthramide A | Avenanthramide A | 0.003 |

**Table 62: Shaving foam**

| **Ingredients** | **Amount** |
|---|---|
| Dem. Water | ad 100 |
| Triethanolamine | 4.0 |
| Edenor L2 SM (Stearinic acid, Palmitinic acid) (Cognis) | 5.3 |
| Laureth-23 | 3.0 |
| Stearylalcohol | 0.5 |
| Avenanthramide L | 0.001 |
| SymSave H (Hydroxyacetophenone) | 0.3 |
| Avenanthramide B | 0.003 |
| Sodium lauryl sulfate | 3.0 |
| Extrapone Seaweed (Water, Propylene glycol, Potasium iodide Fucus Vesiculosus Extract) | 1.0 |
| Dragosantol (Bisabolol, Farnesol) | 0.1 |
| Perfume oil PO1, PO2, PO3, PO4, or PO5 | 1.0 |
| Propane, butane 4,2 Bar | 4.0 |

**Table 63: Sprayable disinfecting gel**

| **Ingredients** | **INCI** | **Amount** |
|---|---|---|
| Water | Water (Aqua) | ad 100 |
| Stabileze QM | PVM / Ma Decadiene Crosspolymer | 0.25 |
| Sodium Hydroxide (10% solution) | Sodium Hydroxide | 0.4 |
| Coffein pure | Caffeine | 0..5 |
| Extrapone^{®} Horse Chestnut | Propylene Glycol, Water (Aqua), Glucose, Aesculus Hippocastanum (Horse Chestnut) Seed Extract, Lactic Acid | 1.0 |
| Hydrolite^{®} 5 | Pentylene Glycol | 3.0 |
| 1,3 Butylene Glycol | Butylene Glycol | 5.0 |
| Biotive^{®} Esculin Sesquihydrate | Esculin | 0.3 |
| Ethanol 96 % | Alcohol Denat, | 10.0 |
| Solubilizer | PEG-40 Hydrogenated Castor Oil, Trideceth-9, Water (Aqua) | 0.5 |
| Perfume oil PO1, PO2, PO3, PO4, or PO5 | Fragrance | 0.2 |
| Octenidine dihydrochloride | Octenidine dihydrochloride | 0.1 |
| Phenoxyethanol | Phenoxyethanol | 0.5 |
| SymSave H | Hydroxyacetophenone | 0.4 |
| Avenanthramide L | Avenanthramide L | 0.0005 |
| Avenanthramide A | Avenanthramide A | 0.003 |
| Avenanthramide B | Avenanthramide B | 0.002 |

**Table 64: Solution for wet wipes**

| **Ingredients** | **INCI** | **Amount** |
|---|---|---|
| SymSol^{®} PF-3 | Water (Aqua), Pentylene Glycol, Sodium Lauryl Sulfoacetate, SodiumOleoyl Sarcosinate, Sodium Chloride, Disodium Sulfoacetate, SodiumOleate, Sodium Sulfate | 2.0 |
| Dragosantol^{®} 100 | Bisabolol | 0.1 |
| Glycerol 99,5 P, | Glycerol | 5.0 |
| Water | Water (Aqua) | ad 100 |
| Hydrolite^{®} 5 | Pentylene Glycol | 5.0 |
| D-Panthenol 75 W | Panthenol | 0.8 |
| DragoCalm^{®} | Water (Aqua), Glycerol, Avena Sativa (Oat) Kernel Extract | 1.0 |
| Witch Hazel-Distillate | Hamamelis Virginiana (Witch Hazel) Water, Water (Aqua), Alcohol | 1.0 |
| Allplant Essence^{®} Org, Rose Geranium P | Pelargonium Graveolens Flower/Leaf/Stem Water | 1.0 |
| Perfume oil PO1, PO2, PO3, PO4, or PO5 | Fragrance | 0.1 |
| Avenanthramide L | Avenanthramide L | 0.2 |
| SymSave H | Hydroxyacetophenone | 0.6 |
| Avenanthramide B | Avenanthramide A | 0.3 |

**Table 65: Further preferred cleansing formulations without sodium lauryl ether sulfate (SLES) (% (w/w)). 1: Mild hair and body ash 2: Shampoo 3: Anti acne face wash 4: Color care shampoo 5: Feminine wash 6: Micellar water 7: Liquid soap 8: Antidandruff shampoo 9: Baby shampoo 10: Solid shampoo**

| INCI | 1 | 2 | 3 | 4 | 5 | 6 | 7 | 8 | 9 | 10 |
|---|---|---|---|---|---|---|---|---|---|---|
| Avenanthramide L | - | 0.001 | - | - | 0.001 | - | - | 0.0005 | 0.2 | - |
| Avenanthramide B | - | - | - | 0.005- | 0.005 | - | - | 0.002 | 0.1 | - |
| Avenanthramide A | - | - | - | 0.005 | 0.005 | - | - | 0.002 | - | - |
| Avenanthramide A, B and C ≥ 100 ppm in sum in glycerine/water | 1.0 | - | 0.5 | - | - | 1.5 | 1.0 | - | - | 2.0 |
| Hydroxyacetophenone (SymSave H) | 0.5 | 0.6 | 0.3 | 0.8 | 0.2 | 0.5 | 0.5 | 0.5 | 0.3 | 0.1 |
| 1.2 Hexanediol, Caprylyl Glycol (Symdiol 68) | 0.5 | 0.5 | - | - | 1.0 | 0.5 | - | 0.5 | 0.5 | - |
| 1,2 Hexanediol, Caprylyl Glycol, Tropolone (Symdiol 68 T) | - | - | 0.7 | - | - | - | 0.7 | - | - | - |
| Ammonium Lauryl Sulfate | - | 5.0 | - | - | - | - | - | - | - | - |
| (Stepanol AM) | - | - | - | - | - | - | - | - | - | - |
| Aqua, Glycerin, Echinacea Purpurea Extract (Extrapone Echinacea) | - | - | - | - | - | 0.3 | - | - | - | - |
| Aqua, Pentylene Glycol, Sodium Lauryl Sulfoacetate, Sodium Oleoyl Sarcosinate, Sodium Chloride, Sodium Oleate (SymSol PF3) | - | - | - | - | - | 3.0 | - | - | - | - |
| Bisabolol (Dragosantol 100) | - | - | - | - | 0.1 | - | - | - | - | - |
| Butyrospermum Parkii Butter (Cetiol SB 45) | - | - | - | - | - | - | - | - | - | 13.0 |
| Caprylic/Capric Triglyceride Hydroxymethoxyphenyl Decanone (Symdecanox HA) | - | - | - | - | - | - | - | - | - | 2.0 |
| Caprylyl Glycol, 1,2 Hexanediol, Methylbenzyl Alcohol | - | - | - | - | 0.8 | - | - | - | 0.8 | - |
| Citric Acid 30% aqueous sol. | - | - | - | 3.0 | - | - | - | - | - | - |
| Climbazole (Crinipan AD) | - | - | | - | - | - | - | 0.5 | - | - |
| Cocamide MEA (Mackamide CMA) | - | - | - | - | - | - | - | 3.0 | - | - |

| INCl | 1 | 2 | 3 | 4 | 5 | 6 | 7 | 8 | 9 | 10 |
|---|---|---|---|---|---|---|---|---|---|---|
| Cocamide MIPA | 0.5 | - | - | - | - | - | - | - | - | - |
| Cocoam idopropyl Betaine (Tego Betain F50) | 15.0 | 5.0 | 3.0 | 6.0 | 14.0 | - | - | 15.0 | 17.0 | - |
| Coco Betaine (Dehyton AB 30) | - | - | - | - | - | - | 2.0 | - | - | - |
| Coco-Glucoside (Plantacare 818 UP) | - | 10.0 | - | - | - | - | - | - | - | - |
| Decyl Glycoside (Ecosense 3000) | - | - | - | - | 2.0 | - | - | - | - | - |
| Disodium Cocoyl Glutamate (Plantapon ACG LC) | - | 3.0 | - | - | - | - | - | - | - | - |
| Disodium EDTA (EDTA BD) | - | - | - | - | - | - | - | 0.1 | - | - |
| Disodium Lauryl Sulfosuccinate (Setacin F spezial) | - | - | 2.0 | 2.0 | - | - | - | - | - | - |
| Fragrance PO1, PO2, PO3, PO4, or PO5 | 1.0 | 0.5 | 0.1 | 0.3 | 0.1 | 0.3 | 0.5 | 0.3 | 0.05 | - |
| Glycerin 99% | - | - | 0.5 | - | 3.0 | - | - | - | - | - |
| Glycerin, Aqua, Hamamelis Virginia Bark/Leaf/Twig Extract (Extrapone Witch Hazel GW) | - | - | - | - | 1.0 | - | - | - | - | - |
| Glyceryl Caprylate (Symlite G8) | 0.1 | 0.5 | 0.2 | 0.3 | 0.5 | 0.1 | - | | - | - |
| Glycol Distearate, Laureth-4, Cocoam idopropyl | - | - | - | 2.0 | - | - | - | 3.0 | - | - |

| INCI | 1 | 2 | 3 | 4 | 5 | 6 | 7 | 8 | 9 | 10 |
|---|---|---|---|---|---|---|---|---|---|---|
| Betaine (Quickpearl PK3) | | | | | | | | | | |
| Isostearamide MIPA, Glyceryl Laurate (Antil SPA 80) | - | - | - | 1.0 | - | - | - | - | - | - |
| Kaolin (lmerCare^{®} 02K-S) | - | - | - | - | - | - | - | - | - | 18.8 |
| Lactic Acid 90% aqueous sol. | - | - | | - | 0.3 | - | - | - | - | - |
| Lauroyl /Myristoyl Methyl Glucamide (Glucotain Clean) | 12.0 | - | - | - | - | - | - | - | - | - |
| Lauryl Hydroxysultaine (45% AS) | - | - | - | - | - | - | 11.0 | - | - | - |
| Lauryl Lactate (Schercemol LL Ester) | - | - | - | - | - | - | 0.3 | - | - | - |
| Maltodextrin, Lactobacillus Ferment (Sym Reboot L19) | - | - | - | - | - | - | - | - | - | 0.5 |
| Menthyl Lactate (Frescolat ML) | - | - | - | - | - | - | - | 0.2 | - | - |
| PEG-200 Hydrogenated Glyceryl Palmate, PEG-7 Glyceryl Cocoate (Antil 200) | - | - | - | 4.5 | - | - | - | - | - | - |
| PEG-4 Rapeseedamide (92% AS) | - | - | - | - | - | - | 2.0 | - | 2.8 | - |
| PEG-40 Hydrogenated Castor Oil, Trideceth-9, | - | - | - | - | 1.5 | - | - | 0.9 | - | - |
| Propylene Glycol, Aqua (Solubilizer Symrise) | | | | | | | | | | |
| PEG-45 M (PolyoxWSR N 60K) | - | - | - | - | - | - | - | 0.15 | - | - |
| Pentylene Glycol (Hydrolite-5 Green) | - | - | - | - | - | 2.0 | - | - | - | 1.5 |
| Pentylene Glycol, 4-t-Butylcyclohexanol (Symsitive 1609) | - | - | - | - | - | 1.0 | - | - | - | - |
| Pentylene Glycol, Butylene Glycol, Hydroxyphenyl Propam idobenzoic Acid (SymCalmin) | - | - | - | - | 1.0 | - | - | 2.0 | - | - |
| Phenyl Propanol, O-Cymen-5-ol, Decylene Glycol (Symguard CD) | - | - | - | - | 0.5 | - | - | - | - | - |
| Piroctone Olamine (Octopirox) | 0.1 | | 0.5 | - | - | - | - | - | - | - |
| Polyacrylate 33 (Rheomer 33T) | - | - | - | - | - | - | - | 6.5 | - | - |
| Polyquaternium-10 (Polymer JR 400) | - | - | | 0.3 | - | - | - | 0.2 | - | - |
| Polyquaternium-7 (Dehyquart CC7) | 0.4 | - | - | - | - | - | - | - | - | - |
| Polysilicone -19 (Abil UV Quat 50) | - | - | - | 2.0 | - | - | - | - | - | - |
| Potassium Sorbate | - | - | 0.3 | 0.4 | - | 0.5 | - | - | - | 0.3 |
| Propylene Glycol | - | - | - | - | 3.0 | - | - | - | - | - |
| Rhamnose | - | - | - | - | - | 0.5 | - | - | - | - |
| Sodium C14-C16 Olefin Sulfonate (38% AS) | - | - | - | - | - | - | 27.0 | - | - | - |
| Sodium Chloride | 0.5 | - | - | - | - | - | - | - | - | - |
| Sodium Cocoamphoacetate (Rewoteric AMC) | - | - | - | 6.0 | - | - | - | - | - | - |
| Sodium Cocoyl Alaninate (Amilite ACS 12) | - | - | - | - | - | 2.0 | - | - | - | - |
| Sodium Cocoyl Glutamate (Hostapon CCG) | - | 5.0 | 3.0 | - | - | - | - | - | - | - |
| Sodium Cocoyl Glycinate (Hostapon SG) | 10.0 | - | - | - | - | - | - | - | - | - |
| Sodium Cocoyl Isethionate (ELFAN^{®} AT 84) | 4.5 | - | - | - | - | - | - | - | - | 15.0 |
| Sodium Hydroxide (50% solution) | - | - | - | - | - | - | - | 0.5 | - | - |
| Sodium Laureth-5 Carboxylate (Akypo Foam RL 40) | - | - | - | - | - | - | - | - | 8.0 | - |
| Sodium Laureth-6 Carboxylate (Akypo SOFT 45 HP) | - | - | - | - | - | 1.0 | - | - | - | - |
| Sodium Lauroyl Glutamate (Hostapon CLG) | 3.0 | - | - | - | - | - | - | - | - | - |
| Sodium Lauroyl | - | - | - | - | 2.0 | - | - | - | - | - |
| Lactylate (Dermosoft SLL) | | | | | | | | | | |
| Sodium Lauroyl Methyl Isethionate (Iselux LQ-CLR SB) | - | - | - | 22.0 | - | - | - | - | - | - |
| Sodium Lauroyl Sarcosinate (Protelan LS 9011) | - | 3.0 | - | 3.0 | - | - | - | - | - | - |
| Sodium Lauryl Glucose Carboxylate Lauryl Glucoside (Plantapon LGC Sorb) | - | - | - | - | - | - | - | 6.0 | - | - |
| Sodium Myristoyl Glutamate (Amisoft MS11) | - | - | - | - | - | - | - | - | - | 30.0 |
| Sodium Salicylate (Seboclear) | - | - | 0.3 | - | - | - | - | - | - | - |
| Sorbitol | 1.0 | - | - | - | - | - | - | | - | - |
| Trideceth-9, PEG-5 Isononanoate, Water (Aqua) | - | - | - | - | - | - | - | 2.0 | - | - |
| Water (Aqua), Glycerin, Tetraselmis Suecica Extract (SymControl Care) | - | - | 1.0 | - | - | - | - | - | - | - |
| Xanthan Gum (Keltrol RD) | - | 0.5 | - | - | 0.5 | - | - | - | - | - |
| Water (Aqua) | Ad 100 | | | | | | | | | |

**Table 66: Gel dental cream**

| **Ingredients** | **I (%)** | **II (%)** | **III (%)** |
|---|---|---|---|
| Sodium carboxymethylcellulose | 0.40 | 0.40 | 0.40 |
| Sorbitol 70 %, in water | 72.00 | 72.00 | 72.00 |
| Polyethylenglycol (PEG) 1500 | 3.00 | 3.00 | 3.00 |
| Sodium saccharinate | 0.07 | 0.07 | 0.07 |
| Sodium fluoride | 0.24 | 0.24 | 0.24 |
| p-Hydroxybenzoic acid (PHB) ethylester | 0.15 | 0.15 | |
| SymDiol 68 | | | 0.5 |
| SymSave H | 0.2 | 0.02 | 0.25 |
| Peppermint flavor | 1.00 | 1.00 | 1.00 |
| Avenanthramide A and B 1:1 (w/w) | 0.03 | | |
| Avenanthramide L | | | 0.01 |
| Dianthramide B | | 0.005 | |
| Abrasive Silica | 11.00 | 11.00 | 11.00 |
| Thickening Silica | 6.00 | 6.00 | 6.00 |
| Sodium dodecylsulfate (SDS) | 1.40 | 1.40 | 1.40 |
| Distilled water | ad 100.00 | ad 100.00 | ad 100.00 |

**Table 67: Ready-to-use mouthwash with fluoride**

| **Ingredients** | **I (%)** | **II (%)** | **III (%)** |
|---|---|---|---|
| Ethanol | 7.00 | 7.00 | |
| Glycerin | 12.00 | 12.00 | |
| Sodium fluoride | 0.05 | 0.05 | 0.18 |
| Pluronic F-127^{®} (BASF, surface active substance) | 1.40 | 1.40 | |
| Sodium phosphate buffer pH 7.0 | 1.10 | 1.10 | |
| Sorbic acid | 0.20 | 0.20 | |
| Sodium saccharinate | 0.10 | 0.10 | 0.10 |
| Cinnamon/menthol flavor | 0.15 | 0.15 | 0.15 |
| Avenanthramide A | | | 0.005 |
| Dianthramide B | | 0.002 | |
| Avenanthramide A, B and C ≥ 100 ppm in sum in glycerine/water | 5.0 | | |
| Colour | 0.01 | 0.01 | 0.01 |
| Sorbitol 70 % | | | 10 |
| Cremophor RH455 | | | 1.8 |
| SymDiol 68 | | | 0.5 |
| SymSave H | 0.1 | 0.05 | 0.15 |
| Distilled water | ad 100.00 | ad 100.00 | ad 100.00 |

## Claims

1. A composition comprising or consisting of:
- at least one avenanthramide or an analogue thereof; and
- 4-hydroxyacetophenone.

2. The composition according to Claim 1, wherein the at least one avenanthramide is selected from the group consisting of the avenanthramides A, B, C, G, H, K, L and R or mixtures of these avenanthramides, in particular wherein the at least one avenanthramide is selected from the group consisting of avenanthramide A and avenanthramide L.

3. The composition according to Claim 1, wherein the at least one avenanthramide analogue is Dihydroavenanthramide D.

4. The composition according to Claim 1 or Claim 2, wherein the at least one avenanthramide is obtained from oat source of the species *Avena sativa* or *Avena nuda,* in particular milled or non-milled grains or oat straw.

5. The composition according to any one of Claims 1 to 4, comprising
- 0.0001 to 5.0 wt% of the at least one avenanthramide or an analogue thereof, in particular 0.001 to 1.0 wt%; and
- 0.005 to 2.0 wt% 4-hydroxyacetophenone, in particular 0.1 to 1.0 wt%,
based on the total weight of the composition; and/or
wherein the ratio of the at least one avenanthramide, particularly avenanthramide A or avenanthramide L, to 4-hydroxyacetophenone is 1 : 1 to 1 : 50, in particular 1 : 1.5 to 1 : 40 or wherein the ratio of the at least one avenanthramide analogue, particularly Dihydroavenanthramide D, is 1 : 0.2 to 1 : 30, in particular 1 : 0.5 to 1 : 20.

6. The non-therapeutic or cosmetic use of the composition according to any one of Claims 1 to 5, for skin care, scalp care, hair care, nail care or in the prevention and/or treatment of skin conditions, intolerant and sensitive skin, skin irritation, skin reddening, wheals, pruritus (itching), skin aging, wrinkle formation, loss of skin volume, loss of skin elasticity, pigment spots, pigment abnormalities, or dry skin, i.e. for moisturising the skin.

7. Composition according to any one of Claims 1 to 5 for use as a medicament.

8. Composition according to Claim 7 for use in the prevention and/or treatment of dermatological or keratological diseases, in particular of dermatological or keratological diseases having a barrier related, inflammatory, immunoallergic, atherogenic, xerotic or hyperproliferative component or in the prevention and/or treatment of dermatological diseases associated with increased ROS production or in the prevention and/or treatment of cardiovascular diseases, allergic reactions, coronary heart disease, for decreasing the level of LDL cholesterol and lipids in blood serum, for reducing blood pressure, for improving sensitivity to insulin and for enabling the control of blood glucose levels.

9. Composition according to Claim 8, wherein the dermatological or keratological diseases are selected rom the group consisting of eczema, psoriasis, seborrhoea, dermatitis, erythema, pruritus (itching), otitis, inflammation, irritation, fibrosis, *lichen planus, pityriasis rosea*, *pityriasis versicolor,* autoimmune bullous diseases, urticarial, angiodermal and allergic skin reactions, and wound healing, and/or wherein the skin diseases associated with increased ROS production are selected from the group consisting of atopic dermatitis, neurodermitis, psoriasis, rosacea, acneiform eruptions, sebostasis and xerosis.

10. The use of the composition according to any one of Claims 1 to 5 for preparing foods, food supplements, cosmetic, pharmaceutical or veterinary preparations, in particular dermatological or keratological preparations.

11. Foods, food supplements, cosmetic, pharmaceutical or veterinary preparations comprising the composition according to any one of Claims 1 to 5, in particular in an amount of 0.0001 to 5.0 %, more preferred 0.0005 to 2.0 %, most preferred 0.001 to 1.0 % by weight of the composition.

12. Composition according to any one of Claims 1 to 5 or foods, food supplements, cosmetic, pharmaceutical or veterinary preparations according to Claim 11, further comprising one or more active substance(s) selected from the group consisting of skin-moisturising and/or moisture-retaining substances, cooling agents, osmolytes, keratological substances, nurturing substances, anti-inflammatory, antibacterial or antimycotic substances, substances having a reddening-alleviating or itch-alleviating action, lenitive substances, and any mixtures of these; and/or cosmetically or pharmaceutically acceptable excipients selected from the group consisting of antioxidants, preservatives, (metal) chelating agents, penetration enhancers, surface-active substances, emulsifiers, perfume oils, anti-foaming agents, colorants, pigments having a colouring action, thickeners, plasticisers, fats, oils, waxes or other conventional components of a cosmetic composition, such as alcohols, polyols, polymers, foam stabilisers, electrolytes, organic solvents or silicone derivatives, and any mixtures of these.

13. Cosmetic or pharmaceutical preparations according to Claim 11 or Claim 12, provided as a fluid, tincture, lotion, emulsion, gel, cream, ointment, spray or shampoo.

14. Use of 4-hydroxyacetophenone for improving solubility of an avenanthramide, particularly avenanthramide A or avenanthramdie L, or an analogue thereof, particularly Dihydroavenenthramide D.

15. Method for improving the solubility of at least one avenanthramide, particularly avenanthramide A or avenanthramide L, or at least one avenanthramide analogue, particularly Dihydroavenanthramide D, in a composition, whereby 4-hydroxyacetophenone is added to the composition or preparation.
